(19) Europäisches Patentamt
European Patent Office
Office européen des brevets

(11) Publication number: 0 077 938 A2

# (12) EUROPEAN PATENT APPLICATION

(21) Application number: 82109201.2

(22) Date of filing: 05.10.82

(51) Int. Cl.$^3$: C 07 D 209/48
A 01 N 43/38

(30) Priority: 23.10.81 JP 169846/81
11.12.81 JP 199847/81

(43) Date of publication of application:
04.05.83 Bulletin 83/18

(84) Designated Contracting States:
AT BE CH DE FR GB IT LI LU NL SE

(71) Applicant: MITSUBISHI CHEMICAL INDUSTRIES LIMITED
5-2, Marunouchi 2-chome Chiyoda-ku
Tokyo 100(JP)

(72) Inventor: Jukihara, Tetsuo
146-1, Suenaga, Takatsu-ku
Kawasaki-shi Kanagawa-ken(JP)

(72) Inventor: Ushinohama, Kazuyuki
10-4, Ogawa 2-chome
Machida-shi Tokyo(JP)

(72) Inventor: Natsume, Bunzi
5-1, Tsutsujigaoka, Midori-ku
Yokohama-shi Kanagawa-ken(JP)

(72) Inventor: Watanabe, Hisao
6-20, Satsukigaoka, Midori-ku
Yokohama-shi Kangawa-ken(JP)

(72) Inventor: Suzuki, Seiichi
8-2, Ogawa 2-chome
Machida-shi Tokyo(JP)

(74) Representative: Patentanwälte Grünecker, Dr. Kinkeldey, Dr. Stockmair, Dr. Schumann, Jakob, Dr. Bezold, Meister, Hilgers, Dr. Meyer-Plath
Maximilianstrasse 43
D-8000 München 22(DE)

## (54) N-(3-substituted aminophenyl) tetrahydrophthalimides and herbicidal composition.

(57) Novel tetrahydrophthalimides are represented by the general formula (I):

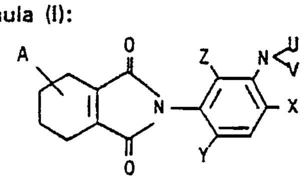

(I)

wherein A is a hydrogen atom or a methyl group,
X is a hydrogen atom, a halogen atom, a methyl group or a methoxy group,
each of Y and Z is a hydrogen atom or a halogen atom,
each of U and V is a hydrogen atom; a

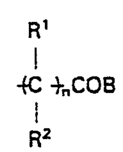

group; an alkyl group; an alkyl group having one or two substituents selected from the group consitsing of a halogen atom, an alkoxy group, a cyano group and an aryl group which may be substituted by a halogen atom; an alkenyl group; an alkenyl group having one or two substituents selected from the group consitsing of an alkoxycarbonyl group and a cyano group; an alkynyl group; a

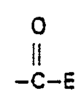

group; or an alkylsulfonyl group. The novel compounds are useful as herbicides.

EP 0 077 938 A2

BACKGROUND OF THE INVENTION:

The present invention relates to novel N-substituted tetrahydrophthalimides and herbicidal compositions thereof.

N-substituted aryl-$\Delta^1$-tetrahydrophthalimide derivatives having herbicidal activity have been reported.

For example, Japanese Examined Patent Publication No. 11940/1973 (US Patent 3,878,224 and US Patent 3,984,435 and West German Unexamined Patent Publication 2,165,651) discloses N-substituted-$\Delta^1$-tetrahydrophthalimide which is represented by the general formula

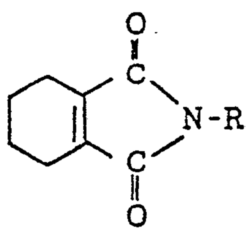

wherein R may be aryl, aralkyl or phenyl optionally substituted by 1 to 5 halogen atoms, hydroxy, alkoxy, nitro, cyano, thiocyanno, carboxy, halogenated alkyl, alkyl, phenyl, $-OCH_2A$ (wherein A is phenyl or naphthyl) or the like. N-(4-chloro-3-methoxyphenyl)-$\Delta^1$-tetrahydrophthalimide and N-(4-bromo-3-methoxyphenyl)-$\Delta^1$-tetrahydrophthalimide are exemplified as the compounds having the formula wherein R is a halogen- and alkoxy-substituted phenyl group.

U.S. Patent 4,001,272 and U.S. Patent 4,032,326 disclose herbicidal 2-substituted aryl-4,5,6,7-tetrahydro-2H-isoindole-1,3-diones of the following formula:

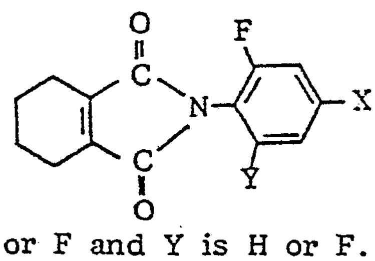

wherein X is Cl, Br or F and Y is H or F.

Published U.K. Patent Application No. 2046754 (U.S. Patent 4,292,070 and Canadian Patent 1,109,881) discloses tetrahydrophthalimides represented by the following formula and their herbicidal activities.

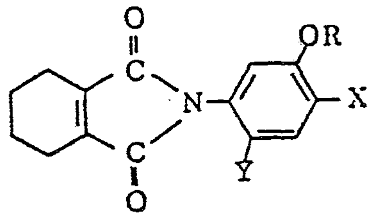



wherein X represents a chlorine or bromine atom; Y represents a chlorine or hydrogen atom; R represents alkenyl, alkynyl, aralkyl, aryloxyalkyl, akyl having two or more carbon atoms and cycloalkyl.

Even slight changes in the structure (such as changes in the kind, number or position of the substituents) of these compounds greatly affect their herbicidal activities (such as the presence or absence or the strength of their herbicidal effects or their herbicidal selectivities). Thus, it is difficult to predict herbicidal activities or selectivities of novel compounds on the basis of the similarity of chemical structures.

## SUMMARY OF THE INVENTION:

The present inventors have conducted extensive researches with an aim to provide tetrahydrophthalimides having superior herbicidal activities and safty to plants, and as a result, have found that novel N-substituted aryl-3,4,5,6-tetrahydrophthalimides in which the N-substituted aryl group is a phenyl group having a hydrogen atom or a halogen atom at 2- and 6-positions, a hydrogen atom, a halogen atom, a methyl group or a methoxy group at 4-position and a specific substituent at 3-position, exhibit extremely superior characteristics as herbicides.

The present invention provides novel N-(3-substituted amino) phenyl-3,4,5,6-tetrahydrophthalimides represented by the following general formula (I), a process for their production and herbicidal compositions containing them as an active ingredient.

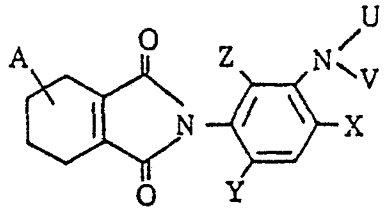

(I)

wherein A is a hydrogen atom or a methyl group, X is a hydrogen atom, a halogen atom, a methyl group or a methoxy group, each of Y and Z is a hydrogen atom or a halogen atom, each of U and V is a hydrogen atom; a $-(\overset{R^1}{\underset{R^2}{C}})_n-COB$ group; an alkyl group; an alkyl group having one or two substituents selected from the group consisting of a halogen atom, an alkoxy group, a cyano group and an aryl group which may be substituted by a halogen atom; an alkenyl group; an alkenyl group having one or two substituents selected from an alkoxycarbonyl group and a cyano group; an alkynyl group; a $-\overset{O}{\overset{\|}{C}}-E$ group; or an alkylsulfonyl group.

In the above substituents, $R^1$ is a hydrogen atom, an alkyl group or a phenyl group, $R^2$ is a hydrogen atom or a methyl group, n is an integer of from 1 to 6, B is $-OR^3$, $-SR^4$ or $-N{<}^{R^5}_{R^6}$ and E is $-R^7$, $-OR^8$, $-SR^9$, $-N{<}^{R^{10}}_{R^{11}}$ or a hydrogen atom.

In the above substituents, $R^3$ is a hydrogen atom, an alkyl group, an alkenyl group, an alkynyl group, a cycloalkyl group, an aryl group, an aralkyl group or $-\overset{R^{12}}{CH}-COD$, $R^4$ is an alkyl group, each of $R^5$ and $R^6$ is a hydrogen atom, an alkyl group, an alkoxy group, an alkoxyalkyl group, an alkenyl group, an alkynyl group, an aryl group, an aralkyl group, a cycloalkyl group, an alkylsulfonyl group or $-\overset{R^{12}}{CH}-COD$, or $-N{<}^{R^5}_{R^6}$ is a heterocyclic group which may include an oxygen atom, $R^7$ is an alkyl group which may be substituted by one or more halogen atoms; an alkenyl group or an aryl group which may be substituted by a halogen atom, $R^8$ is an alkyl group, a cycloalkyl group, an aralkyl group or an aryl group, $R^9$ is an alkyl group or an aryl group, and each of $R^{10}$ and

$R^{11}$ is an alkyl group, an alkoxy group or an aryl group which may be substituted by a halogen atom.

In the above substituents, $R^{12}$ is a hydrogen atom or an alkyl group, and D is a hydroxyl group, an alkoxy group or an amino group which may be substituted by one or two alkyl or alkenyl groups.

## DETAILED DESCRIPTION OF THE INVENTION:

In the compounds of the above general formula (I), A is a hydrogen atom, a 3-methyl group or a 4-methyl group. X is a hydrogen atom; a halogen atom such as a fluorine atom, a chlorine atom or a bromine atom; a methyl group or a methoxy group. Each of Y and Z is a hydrogen atom or a halogen atom such as a fluorine atom, a chlorine atom or a bromine atom. Each of U and V is a hydrogen atom; a $-(\overset{R^1}{\underset{R^2}{C}})_n COB$ group [where $R^1$ is a hydrogen atom; a $C_1$ - $C_{12}$ alkyl group, preferably a $C_1$ - $C_8$ alkyl group, more preferably a $C_1$ - $C_4$ alkyl group or a phenyl group, $R^2$ is a hydrogen atom or a methyl group, n is an integer of from 1 to 6 and B is $-OR^3$ (where $R^3$ is a hydrogen atom; a $C_1$-$C_{30}$ alkyl group, preferably a $C_1$ - $C_{20}$ alkyl group, more preferably a $C_1$ - $C_5$ alkyl group; a $C_2$ - $C_6$ alkenyl group, preferably a $C_3$ - $C_4$ alkenyl group; a $C_2$ - $C_6$ alkynyl group, preferably a $C_3$ - $C_4$ alkynyl group; a $C_3$ - $C_8$ cycloalkyl group, preferably a $C_5$ - $C_7$ cycloalkyl group; an aryl group such as a phenyl group; an aralkyl group such as a benzyl group or a $-\overset{R^{12}}{C}HCOD$ where $R^{12}$ is a hydrogen atom or a $C_1$ - $C_6$ alkyl group, preferably a $C_1$ - $C_4$ alkyl group, and D is a hydroxyl group, a $C_1$ - $C_4$ alkoxy group or an amino group which may be substituted by 1 or 2 $C_1$ - $C_8$ alkyl, preferably $C_1$ - $C_5$ alkyl or $C_3$ - $C_4$ alkenyl groups), $-SR^4$ (where $R^4$ is a $C_1$ - $C_{10}$ alkyl group, preferably a $C_2$ -$C_5$ alkyl group) or $-N{<}^{R^5}_{R^6}$

(where each of $R^5$ and $R^6$ is a hydrogen atom; a $C_1$ - $C_{30}$ alkyl group, preferably a $C_1$ - $C_{20}$ alkyl group, more preferably a $C_1$ - $C_5$ alkyl group; a $C_1$ - $C_{12}$ alkoxy group, preferably a $C_1$ - $C_8$ alkoxy group, more preferably a $C_1$ - $C_4$ alkoxy group; a $C_2$ - $C_8$ alkoxyalkyl group, preferably a $C_2$ - $C_6$ alkoxyalkyl group, more preferably a $C_2$ - $C_4$ alkoxyalkyl group; a $C_2$ - $C_8$ alkenyl group, preferably a $C_3$ - $C_5$ alkenyl group; a $C_2$ - $C_8$ alkynyl group, preferably a $C_3$ - $C_5$ alkynyl group; an aryl group such as a phenyl group; an aralkyl group such as a benzyl group; a $C_3$ - $C_8$ cycloalkyl group, preferably a $C_3$ - $C_6$ cycloalkyl group; a $C_1$ - $C_8$ alkylsulfonyl group, preferably a $C_1$ - $C_5$ alkylsulfonyl group or a $\underset{}{-\overset{R^{12}}{\overset{|}{C}}HCOD}$ group where $R^{12}$ and D are as defined above, or $-N{<}^{R^5}_{R^6}$ is a 5-7 membered heterocyclic group, preferably a 5-7 membered saturated heterocyclic group, which may contain an oxygen atom)]; a $C_1$ - $C_6$ alkyl, preferably $C_1$ - $C_4$ alkyl group which may be substituted by a halogen atom such as a fluorine atom or a chlorine atom, preferably a chlorine atom, a $C_1$ - $C_4$ alkoxy group, a cyano group or an aryl group, preferably phenyl group, which may be substituted by a halogen atom such as a chlorine atom; a $C_2$ - $C_6$ alkenyl, preferably $C_2$ - $C_4$ alkenyl group which may be substituted by 1 or 2 substituents selected from the group consisting of a $C_2$-$C_5$ alkoxycarbonyl group and a cyano group; a $C_2$ - $C_6$ alkyl, preferably $C_3$ - $C_4$ alkynyl group; a $-\overset{O}{\overset{\|}{C}}-E$ group [where E is $-R^7$ (where $R^7$ is a $C_1$ - $C_5$ alkyl, preferably $C_1$ - $C_3$ alkyl group which may be substituted by from 1 to 3 halogen atoms, preferably fluorine or chlorine atoms; a $C_2$ - $C_5$ alkenyl, preferably $C_3$ - $C_4$ alkenyl group or an aryl group, preferably a phenyl group, which may be substituted by a halogen atom such as a chlorine atom), $-OR^8$ (where $R^8$ is a $C_1$ - $C_{12}$, preferably $C_1$ - $C_8$ alkyl group, a $C_3$ - $C_8$ cycloalkyl, preferably $C_5$ - $C_7$ cycloalkyl group, a $C_7$ - $C_9$ aralkyl group, preferably a benzyl group, or an aryl group such as a phenyl group), $-SR^9$ (where $R^9$ is a $C_1$ - $C_6$ alkyl,

preferably $C_1$ - $C_4$ alkyl group or an aryl group such as a phenyl group) or $-N\begin{smallmatrix}R^{10}\\R^{11}\end{smallmatrix}$ (where each of $R^{10}$ and $R^{11}$ is a hydrogen atom, a $C_1$ - $C_4$ alkyl, preferably $C_1$ - $C_2$ alkyl group, a $C_1$ - $C_4$ alkoxy, preferably $C_1$ - $C_2$ alkoxy group, or an aryl group, preferably a phenyl group, which may be substituted by a halogen atom such as a chlorine atom)]; or a $C_1$ - $C_4$ alkylsulfonyl, preferably $C_1$ - $C_2$ alkylsulfonyl group.

Preferred compounds of the present invention are represented by the following general formulas:

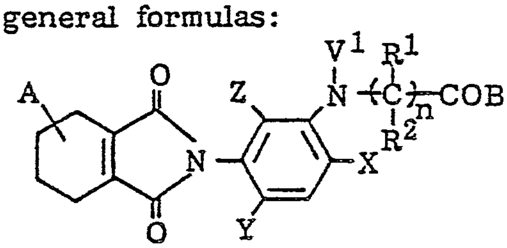

(II)

wherein A, X, Y, Z, $R^1$, $R^2$, n and B are as defined above, and $V^1$ is a hydrogen atom, an alkyl group, a $-\overset{O}{\overset{\|}{C}}$-alkyl group, or a $-\overset{O}{\overset{\|}{C}}$-O-alkyl group. Particularly preferred are compounds in which each of A, Z and $V^1$ is a hydrogen atom, X is a halogen atom and Y is a hydrogen atom or a fluorine atom.

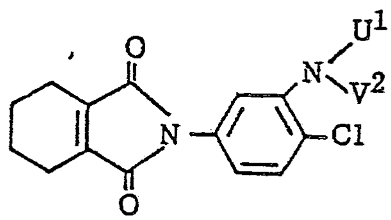

(III)

wherein $U^1$ is an alkyl group which may be substituted by a halogen atom, an alkoxy group, a cyano group or an aryl group which may be substituted by a halogen atom; an alkenyl group which may be substituted by an alkoxycarbonyl group or a cyano group; an alkynyl group; a $-\overset{O}{\overset{\|}{C}}$-E group which E is as defined above; or an alkylsulfonyl group, and $V^2$ is a hydrogen atom or an alkyl group.

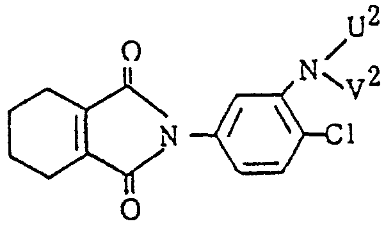

(IV)

wherein each of $U^2$ and $V^2$ is an alkyl group which may be substituted by a halogen atom, an alkoxy group or a cyano group; an alkenyl group or an alkynyl group, provided one of $U^2$ and $V^2$ may be a hydrogen atom.

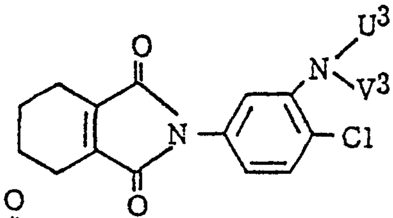

(V)

wherein $U^3$ is a $-\overset{O}{\overset{\|}{C}}-OR^8$ where $R^8$ is as defined above, or $-\overset{O}{\overset{\|}{C}}-SR^9$ where $R^9$ is as defined above, and $V^3$ is an alkyl group.

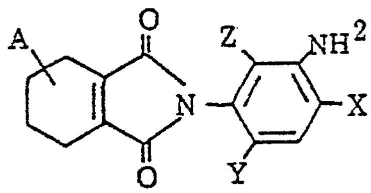

(VI)

wherein A, X, Y and Z are as defined above.

The compounds represented by the following formula (VII) and (VIII) are also particularly suitable for use as herbicides.

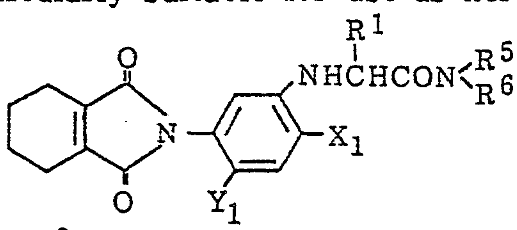

(VII)

wherein $R^1$, $R^5$ and $R^6$ are as defined above with respect to the general formula (I), $X_1$ is a chlorine atom or a bromine atom, and Y is a hydrogen atom or a fluorine atom. Particuarly preferred are the compounds of the formula (VII) wherein $R^1$ is a lower alkyl group having from 1 to 3 carbon atoms, $R^5$ is a hydrogen atom or a $C_1$ - $C_3$ alkyl or alkoxy group, and $R^6$ is a lower alkyl group having from 1 to 5 carbon atoms.

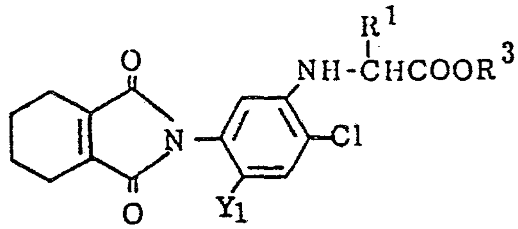

(VIII)

wherein $Y_1$ is a hydrogen atom or a fluorine atom, and $R^1$ and $R^3$ are as defined above with respect to the general formula (I). Particularly preferred are the compounds of the formula (VIII) wherein $R^1$ is an alkyl group having from 1 to 4 carbon atoms, and $R^3$ is an alkyl group having from 1 to 5 carbon atoms or an alkenyl group having 3 or 4 carbon atoms.

The compounds of the present invention may be prepared, for instance, by the reactions represented by the following reaction formulas.

(1)

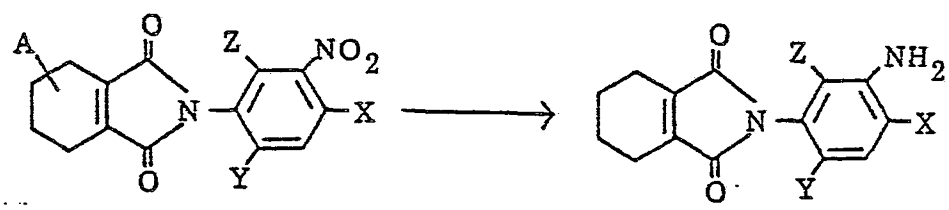

wherein A, X, Y and Z are as defined above.

The above reduction reaction is carried out in a solvent such as acetic acid, ethanol, ethyl acetate, benzene or toluene in the presence of a catalyst such as palladium or Raney nickel under atmospheric or elevated pressure at a temperature of from 0 to 150°C.

The starting material N-(3-nitrophenyl)-3,4,5,6-tetrahydrophthalimides may readily be obtained by reacting cyclohexene-1,2-dicarboxylic acid anhydride with nitroanilines in the absence of any solvent or in a solvent such as acetic acid, methanol, toluene, dioxane or water at a temperature of from 60 to 200°C, and they may be used directly for the above mentioned reduction reaction without isolating them.

(2)

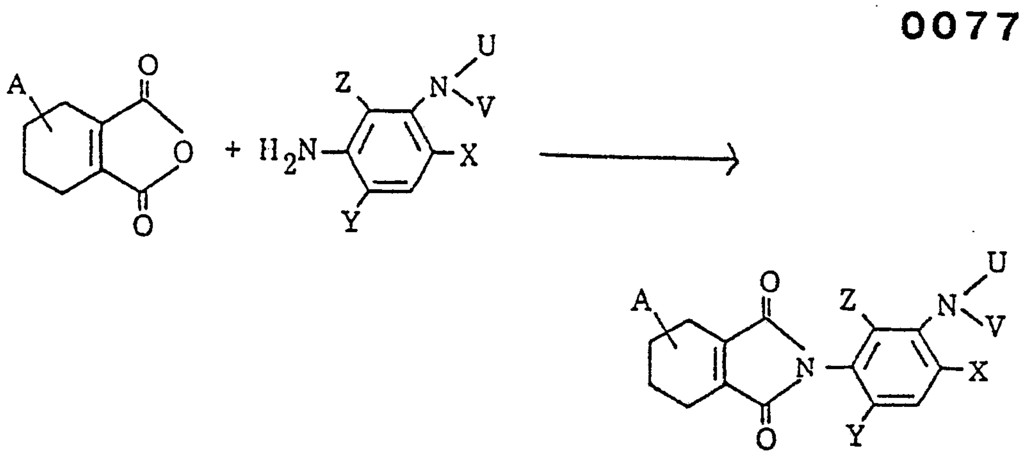

wherein A, X, Y, Z, U and V are as defined above.

The above reaction of cyclohexene-1,2-dicarboxylic acid anhydride with m-phenylenediamines is carried out in the absence of any solvent or in a solvent such as acetic acid, methanol, toluene, dioxane or water at a temperature of from 60 to 200°C.

The starting material m-phenylenediamines may be prepared for instance, by the following steps:

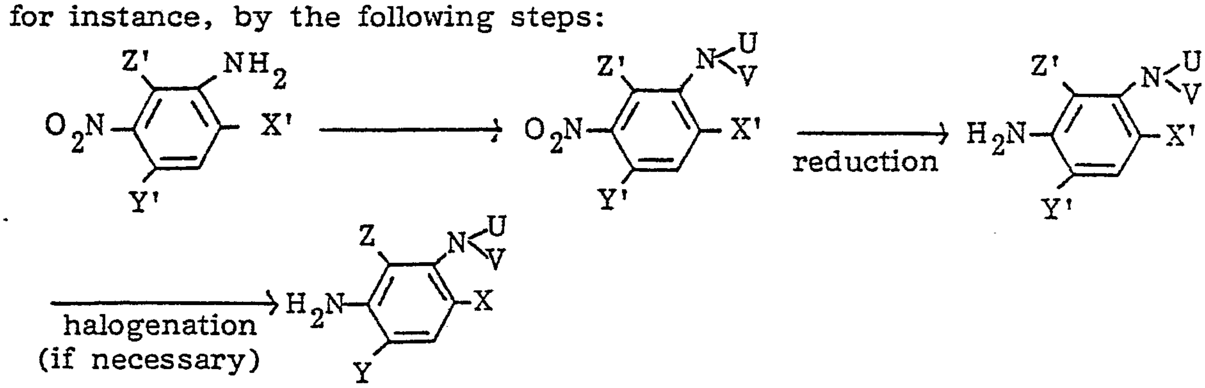


wherein at least one of X', Y' and Z' is a hydrogen atom, the rest being the same as the corresponding X, Y and Z, and U and V are as defined above with respect to the general formula (I). The above reduction and halogenation may be conducted by various conventional methods.

(3)

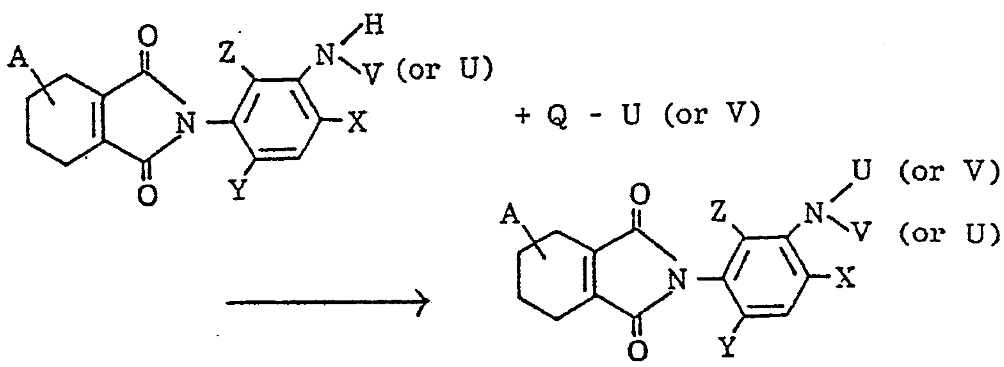

wherein A, X, Y, Z, U and V are as defined above, and Q is a halogen atom or, in some cases, represents mesyl, tosyl or an alkoxy group such as a methoxy group or an ethoxy group, or Q forms together with U an acid anhydride or a mixture of acid anhydrides.

The starting material tetrahydrophthalimides may be prepared, for instance, by the above mentioned method (1) or (2).

The reaction may be carried out without using any solvent or in a solvent such as tetrahydrofuran, dioxane, N,N-dimethylformamide, N,N-dimethylacetamide, dimethyl sulfoxide, sulfolane, benzene, toluene, xylene, cumene, chlorobenzene, dichlorobenzene, methylene chloride, chloroform, carbon tetrachloride, Tri-clene, ethyl acetate, acetone, ethyl methyl ketone, ether or diisopropyl ether in the absence or presence of sodium acetate, potassium bicarbonate, sodium carbonate, potassium carbonate, sodium hydroxide, potassium hydroxide, barium hydroxide, calcium hydroxide, metal lithium, metal sodium, metal potassium, sodium hydride, potassium hydride, sodium amide, lithium chloride, sodium chloride, potassium chloride, magnesium chloride, zinc chloride, iron (II) chloride, iron (III) chloride, copper (I) chloride, copper (II) chloride, lithium iodide, sodium iodide, potassium iodide, potassium fluoride, cesium fluoride, zinc oxide, calcium oxide, barium oxide, iron (II) oxide, iron (III) oxide, lead carbonate, pyridine, 4-dimethylaminopyridine, triethylamine, N,N-diethylaniline, benzyltrimethylammoniumchloride or bromide, benzyltriethylammoniumchloride or bromide, tetra-n-butylammoniumbromide or iodide, crown ethers, or polyoxyethylenes at a temperature of from -20 to 250°C, preferably from 0 to 200°C.

(4)

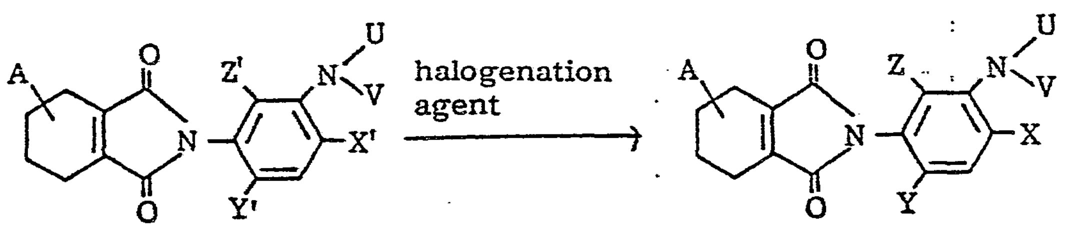

wherein A, U and V are as defined above, at least one of X, Y and Z is a halogen atom, the rest being as defined above, and at least one of X', Y' and Z' is a hydrogen atom, the rest being the same as the corresponding X, Y and Z.

The reaction is carried out in a solvent such as acetic acid, chloroform, carbon tetrachloride, benzene or chlorobenzene under the action of a halogenation agent such as chlorine, bromine, sulfuryl chloride, N-chlorosuccinimide or N-bromosuccinimide at a temperature of from -70 to 150°C, preferably from -20 to 120°C.

(5)

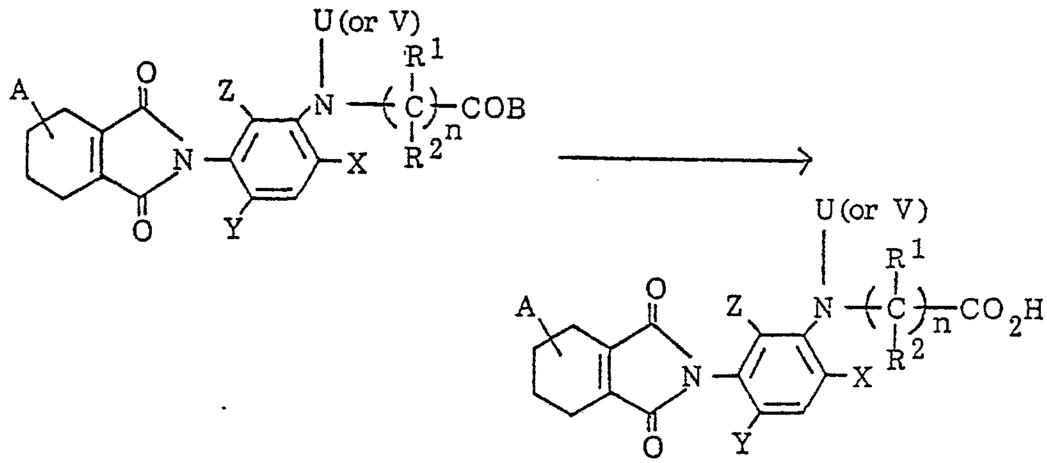

wherein A, X, Y, Z, $R^1$, $R^2$ and n are as defined above, each of U and V is a hydrogen atom, an alkyl group, an alkenyl group or an alkynyl group and B is $-OR^3$, $-SR^4$ or $-N{<}^{R^5}_{R^6}$, where $R^3$, $R^4$, $R^5$ and $R^6$ are as defined above.

The above hydrolysis reaction is carried out (i) in a solvent such as water, water-methanol, water-ethanol or water-dioxane in the presence of hydrochloric acid, sulfuric acid etc. at a temperature of from 0 to 120°C, or in a solvent such as formic acid, acetic acid, etc. in the presence of methane sulfonic acid, p-toluene sulfonic acid, etc. at a temperature of from 80 to 180°C; (ii) in a solvent such as N,N-dimethylformamide, N-methyl-2-pyrrolidone, 2,4,6-collidine, 2,6-lutidine or pyridine in the presence of lithium hydroxide, lithium bromide, lithium iodide, sodium iodide, etc. at a temperature of from 100 to 200°C; or (iii) in a solvent such as

water, water-methanol, water-ethanol, water-dioxane or water-acetone in the presence of sodium hydroxide, potassium hydroxide, barium hydroxide, etc. at a temperature of from -20 to 150°C and after removing the solvent by distillation upon completion of the reaction, the residue is reacted at a temperature of from 50 to 200°C in the presence of a solvent such as acetic acid, dilute hydrochloric acid or dilute hydrochloric acid-dioxane.

(6)

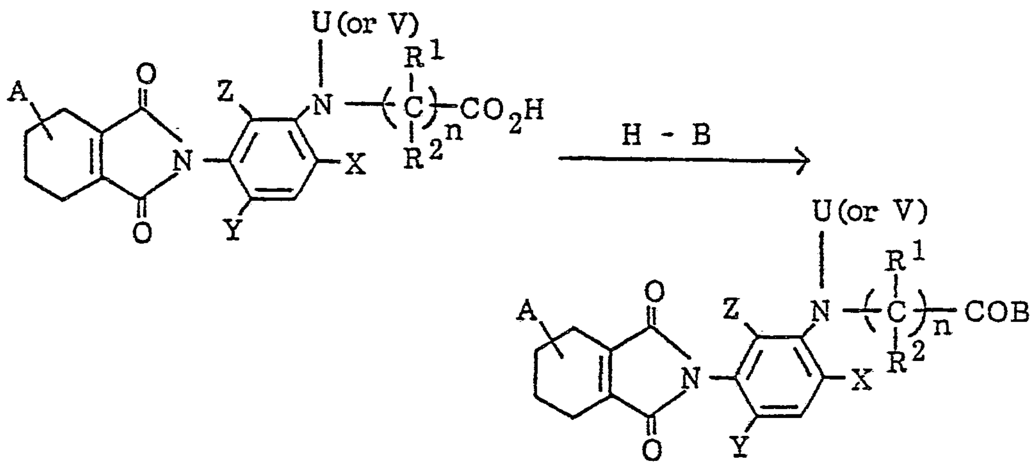


wherein A, X, Y, Z, $R^1$, $R^2$ and n are as defined above, each of U and V is a hydrogen atom, an alkyl group, an alkenyl group or an alkynyl group, and B is $-OR^3$, $-SR^4$ or $-N{<}^{R^5}_{R^6}$ , where $R^3$, $R^4$, $R^5$ and $R^6$ are as defined above.

The above reaction is carried out without using any solvent or in a solvent such as benzene, toluene, methylene chloride, chloroform, carbon tetrachloride, dioxane, ether, acetonitrile, pyridine or N,N-dimethylformamide in the presence of hydrochloric acid, sulfuric acid, phosphoric acid, methane sulfonic acid, p-toluene sulfonic acid, thionyl chloride, phosphorus oxychloride, methyl chlorocarbonate, ethyl chlorocarbonate, N,N'-dicyclohexylcarbodiimide, etc. and in the absence or presence of a base such as sodium bicarbonate, potassium bicarbonate, sodium carbonate, potassium carbonate, sodium hydroxide, potassium hydroxide, pyridine, triethylamine or N,N-diethylaniline at a temperature of from -20 to 150°C.

In this condensation reaction, the starting material carboxylic acids form, in some cases, various intermediates such as acid chlorides, acid anhydrides or mixed acid anhydrides, which in turn react with various alcohols, mercaptans or amines to form the desired compounds. In certain cases, it is possible to use such intermediates after isolating them.

(7)

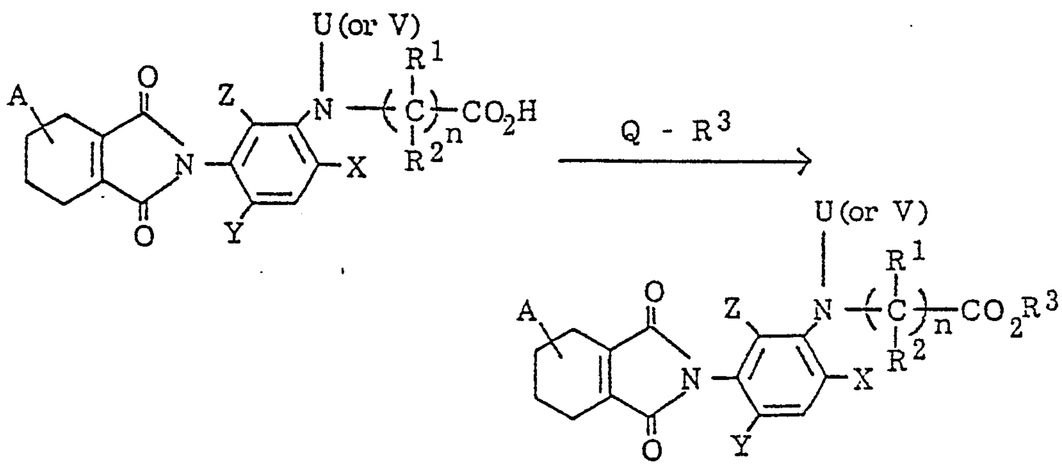


wherein A, X, Y, Z, $R^1$, $R^2$, n and $R^3$ are as defined above, each of U and V is a hydrogen atom or an alkyl group and Q is a halogen atom such as chlorine, bromine, or iodine, or mesyl or tosyl.

The above reaction is carried out in a solvent such as acetone, ethyl methyl ketone, acetonitrile, benzene, toluene, N,N-dimethylformamide, tetrahydrofuran, or dioxane in the presence of sodium carbonate, potassium carbonate, sodium hydroxide, potassium hydroxide, metal sodium, metal potassium, sodium hydride, potassium fluoride, cesium fluoride, etc. at a temperature of from 0 to 180°C.

The compounds having the formula (I) can be various isomers such as optical isomers and diastereoisomers. These isomers and mixtures are included in the definition of the present invention. These isomers are usually obtained in the form of a mixture thereof. In the examples, mixtures of the isomers are formed otherwise specifided.

These isomers can be separated by various processes such as asymmetric synthesis, optical resolution and various chromatography such as column chromatography, thin layer chromatography and high speed liquid chromatography. The separation of diasteroisomers is exemplified in Example 5.

Now, the processes for preparing the compounds of the present invention will be described in detail with reference to Examples. However, it should be understood that these Examples are presented for the purpose of illustration only and are not intended to be limiting the present invention.

## EXAMPLE 1:

Production of N-(5-amino-4-chloro-2-fluorophenyl)-3,4,5,6-tetrahydrophthalimide:

A mixture of 250 ml of benzene and 60.0 g of N-(4-chloro-2-fluoro-5-nitrophenyl)-3,4,5,6-tetrahydrophthalimide (m.p. 136-138°C) obtained by heating cyclohexene-1,2-dicarboxylic acid anhydride and 4-chloro-2-fluoro-5-nitroaniline in acetic acid, was subjected to catalytic reduction at 55°C in the presence of 5% palladium/carbon. After the reduction, the catalyst was filtered off and the solvent was distilled off, whereupon crystals were obtained. The crystals were recrystallized from benzene-n-hexane to obtain 50.8 g of Compound No. 277 as shown in Table 1.

## EXAMPLE 2:

### Production of N-(3-amino-4-chlorophenyl)-3,4,5,6-tetrahydrophthalimide:

A mixture of 16.0 g of cyclohexene-1,2-dicarboxylic acid anhydride, 14.3 g of 4-chloro-m-phenylenediamine and 200 ml of water was reacted under stirring at 100°C for 9 hours. After cooling, the precipitated crystals were collected by filtration to obtain 26.8 g of Compound No. 270 as shown in Table 1.

## EXAMPLE 3:

### Production of N-[2-fluoro-5-[1-(1-methylpropylcarbamoyl)-ethyl]aminophenyl]-3,4,5,6-tetrahydrophthalimide:

7.86 g of 2-(4-fluoro-3-nitrophenylamino)-N-(1-methylpropyl) propionamide was catalytically reduced in 35 ml of acetic acid in the presence of 5% palladium/carbon, and then the catalyst was filtered off. Without isolating the formed 2-(3-amino-4-fluorophenyl)amino-N-(1-methylpropyl) propionamide, 4.21 g of cyclohexene-1,2-dicarboxylic acid anhydride was added to the filtrate, and the mixture was refluxed under heating and stirring for 2 hours. Then, the solvent was distilled off. After adding 50 ml of ethyl acetate, the residue was washed with water and dried over sodium sulfate and the solvent was distilled off. The residue was purified by a silica gel column (developing solvent: ethylacetate: n-hexane = 3:2) to obtain 8.30 g of Compound No. 51 as shown in Table 1.

The starting material 2-(4-fluoro-3-nitrophenyl)amino-N-(1-methylpropyl)propionamide was prepared as follows. Namely, a mixture of 5.20 g of 4-fluoro-3-nitroaniline, 13.7 g of N-(1-methylpropyl)-2-bromo-propionamide and 3.05 g of sodium bicarbonate, was heated at 160°C for

one hour, and the product was purified by a silica gel column (developing solvent: ethyl acetate: n-hexane = 1:1) to obtain 9.19 g of the above mentioned propionamide derivative (m.p. 101-102°C).

EXAMPLE 4:

Production of N-[4-chloro-3-[1-(1-methylpropyl)-carbamoylpropyl]aminophenyl]-3,4,5,6-tetrahydrophthalimide:

A mixture of 2.77 g of N-(3-amino-4-chlorophenyl)-3,4,5,6-tetrahydrophthalimide, 4.44 g of 2-bromo-N-(1-methylpropyl) butanamide and 0.41 g of zinc oxide, was stirred at 160°C for 5 hours, and after cooling it to room temperature, 30 ml of ethyl acetate was added. Insoluble matters were filtered off. The filtrate was concentrated under reduced pressure, and the residue was purified by a silica gel column (developing solvent: ethyl acetate: n-hexane = 1:2) to obtain 2.72 g of Compound No. 151 as shown in Table 1.

EXAMPLE 5:

Production of N-[4-chloro-3-[1-(methylhexyl)carbamoylethyl]aminophenyl]-3,4,5,6-tetrahydrophthalimide, and separation of the formed diastereoisomers:

A mixture of 2.77 g of N-(3-amino-4-chlorophenyl)-3,4,5,6-tetrahydrophthalimide, 6.00 g of 2-bromo-N-(1-methylhexyl)propionamide and 1.0 g of sodium bicarbonate, was stirred at 160°C for 2.5 hours, and after cooling it to room temperature, 30 ml of ethyl acetate was added. The reaction mixture was washed with water and dried over sodium sulfate. The thin layer chromatography of this mixture (carrier: silica gel, developing

solvent: ethyl acetate: n-hexane = 1:2) showed two kinds of products at Rf 0.33 and 0.28. After distilling the solvent off, the residue was separated by a silica gel column (developing solvent: chloroform) to obtain 0.92 g of Compound No. 77 (having a high Rf value), 0.93 g of Compound No. 78 (having a low Rf value) and 0.90 g of a mixture thereof as shown in Table 1.

## EXAMPLE 6:

Production of N-[5-[1-(allylcarbamoyl)propyl]amino-4-chloro-2-fluorophenyl]-3,4,5,6-tetrahydrophthalimide:

A mixture of 2.77 g of N-(5-amino-4-chloro-2-fluorophenyl)-3,4,5,6-tetrahydrophthalimide, 4.12 g of N-allyl-2-bromobutanamide and 1.01 g of sodium bicarbonate, was stirred at 150°C for 2 hours, and after cooling it to room temperature, 40 ml of ethyl acetate was added. The mixture was washed with water and dried over sodium sulfate, and the solvent was distilled off. The residue was purified by a silica gel column (developing solvent: ethyl acetate: n-hexane = 1:2) to obtain 2.30 g of Compound No. 175 as shown in Table 1.

## EXAMPLE 7:

Production of N-[4-chloro-3-[1-(2-methylbutylcarbamoyl)-propyl]aminophenyl]-3,4,5,6-tetrahydrophthalimide:

A mixture of 2.77 g of N-(3-amino-4-chlorophenyl)-3,4,5,6-tetrahydrophthalimide, 4.60 g of N-(2-methylbutyl)-2-bromobutanamide, 1.01 g of sodium bicarbonate, 0.38 g of potassium iodide and 5 ml of sulfolane, was stirred at 160°C for 3 hours, and after cooling it to room temperature, 30 ml of ethyl acetate was added. The mixture was washed

with water and dried over sodium sulfate, and the solvent was distilled off. The residue was purified by a silica gel column (developing solvent: ethyl acetate: n-hexane = 1:3) to obtain 1.90 g of Compound No. 166 as shown in Table 1.

## EXAMPLE 8:

Production of N-[2,4-dibromo-6-fluoro-3-[1-(1-methylpropyl)carbamoylethyl]aminophenyl]-3,4,5,6-tetrahydrophthalimide:

Into a solution composed of 1.93 g of N-[2-fluoro-5-[1-(1-methylpropyl)carbamoylethyl]aminophenyl]-3,4,5,6-tetrahydrophthalimide and 5 ml of acetic acid, 1.60 g of bromine was dropwise added at room temperature while stirring the solution. After 0.5 hour, 20 ml of ethyl acetate was added, and the mixture was washed with water and dried over sodium sulfate. Then, the solvent was distilled off. The residue was purified by a silica gel column (developing solvent: ethyl acetate: n-hexane = 1:1) to obtain 3.10 g of Compound No. 55 as shown in Table 1.

## EXAMPLE 9:

Production of N-[2-chloro-3-(1-ethoxycarbonylpropyl)-amino-6-fluorophenyl]-3,4,5,6-tetrahydrophthalimide:

Into a solution composed of 1.87 g of N-[5-(1-ethoxycarbonylpropyl)amino-2-fluorophenyl]-3,4,5,6-tetrahydrophthalimide and 20 ml of benzene, 0.70 g of sulfuryl chloride was dropwise added at 10°C while stirring the solution. After one hour, the solvent was distilled off, and the residue was purified by a silica gel column (developing solvent: ethyl acetate: n-hexane = 1:2) to obtain 0.95 g of Compound No. 135 as shown in Table 1.

EXAMPLE 10:

Production of N-[3-(1-carboxyethyl)amino-4-methylphenyl]-3,4,5,6-tetrahydrophthalimide:

A mixture of 24.0 g of N-[3-(1-ethoxycarbonylethyl)amino-4-methylphenyl]-3,4,5,6-tetrahydrophthalimide, 8.9 g of sodium hydroxide, 15 ml of water and 80 ml of methanol, was stirred at 80°C for one hour, and then the solvent was distilled off. To the residue, 2.1 g of cyclohexene-1,2-dicarboxylic acid anhydride and 80 ml of acetic acid were added, and the mixture was refluxed under stirring for 3 hours. Then, the solvent was again distilled off, and 120 ml of ethyl acetate was added. The mixture was washed with water and dried over sodium sulfate, and the solvent was then distilled off. The residue was recrystallized from ethyl acetate-n-hexane to obtain 15.7 g of Compound No. 9 as shown in Table 1.

EXAMPLE 11:

Production of N-[4-chloro-3-[1-(N-methoxy-N-methylcarbamoyl)butyl]aminophenyl]-3,4,5,6-tetrahydrophthalimide:

Into a mixture of 1.88 g of N-[4-chloro-3-(1-carboxybutyl)-aminophenyl]-3,4,5,6-tetrahydrophthalimide, 0.99 g of pyridine and 15 ml of dichloromethane, a solution prepared by dissolving 0.65 g of thionyl chloride in 1 ml of dichloromethane was dropwise added at 7°C and the mixture was stirred for 20 minutes. A mixture of 0.39 g of N,O-dimethylhydroxylamine, 0.56 g of triethylamine and 3 ml of dichloromethane was dropwise added thereto, and the mixture was further stirred for 1.5 hours at room temperature. Then, the reaction solution was washed sequentially with 2N hydrochloric acid and water. The organic

phase was dried over sodium sulfate, and then the solvent was distilled off. The residue was purified by a silica gel column (developing solvent: ethyl acetate: n-hexane = 4:7) to obtain 1.85 g of Compound No. 208 as shown in Table 1.

EXAMPLE 12:

Production of N-[4-chloro3-[1-(1-ethoxycarbonylbutyloxycarbonyl)ethyl]aminophenyl]-3,4,5,6-tetrahydrophthalimide:

A mixture of 1.74 g of N-[4-chloro-3-(1-carboxyethyl)aminophenyl]-3,4,5,6-tetrahydrophthalimide, 1.15 g of 2-bromovaleric acid ethyl ester, 0.64 g of potassium fluoride and 10 ml of acetonitrile was refluxed under stirring for 30 hours, and then the solvent was distilled off. 20 ml of ethyl acetate was added thereto, and the mixture was washed with water and dried over sodium sulfate. Then, the solvent was distilled off, and the residue was purified by a silica gel column (developing solvent: ethyl acetate: n-hexane = 4:7) to obtain 1.85 g of Compound No. 25 as shown in Table 1.

EXAMPLE 13:

Production of N-[4-chloro-3-(N-ethoxycarbonyl-N-propyl)-aminophenyl]-3,4,5,6-tetrahydrophthalimide:

A mixture of 1.50 g of N-(4-chloro-3-propylaminophenyl)-3,4,5,6-tetrahydrophthalimide, 0.56 g of ethyl chlorocarbonate, 0.45 g of pyridine and 20 ml of anhydrous tetrahydrofuran, was refluxed under stirring for 2 hours and then the solvent was distilled off. The residue was extracted with ethylacetate, washed with water and dried over sodium sulfate, and then the solvent was distilled off. The residue

was purified by a silica gel column (developing solvent: ethylacetate: n-hexane = 1:3) to obtain 1.25 g of Compound No. 243 as shown in Table 1.

EXAMPLE 14:

Production of N-(3-acetylamino-4-chlorophenyl)-3,4,5,6-tetrahydrophthalimide:

A mixture of 1.20 g of N-(3-amino-4-chlorophenyl)-3,4,5,6-tetrahydrophthalimide and 5 ml of acetic anhydride was heated at 90°C for 5 minutes, and after adding 50 ml of water, the mixture was stirred at room temperature. The precipitated crystals was collected by filtration and recrystallized from ethanol-cyclohexane to obtain 0.90 g of Compound No. 255 as shown in Table 1.

EXAMPLE 15:

Production of N-[4-chloro-3-(methylcarbamoyl)aminophenyl]-3,4,5,6-tetrahydrophthalimide:

A mixture of 1.38 g of N-(3-amino-4-chlorophenyl)-3,4,5,6-tetrahydrophthalimide, 0.3 g of methyl isocyanate and 10 ml of tetrahydrofuran, was left to stand at room temperature for 3 days, and then the solvent was distilled off. The residue was recrystallized from ethylacetate-methanol to obtain 0.50 g of Compound No. 264 as shown in Table 1.

## EXAMPLE 16:

Production of N-[4-chloro-3-(N-methoxy-N-methylcarbamoyl)aminophenyl]-3,4,5,6-tetrahydrophthalimide:

A mixture of 2.77 g of N-(3-amino-4-chlorophenyl)-3,4,5,6-tetrahydrophthalimide, 1.19 g of N-methoxy-N-methylcarbamoyl chloride and 10 ml of pyridine, was left to stand still for 12 hours at room temperature, and then 30 ml of water was added. The precipitated crystals were collected by filtration, washed with water, dried and recrystallized from ethyl acetate-n-hexane to obtain 1.99 g of Compound No. 265 as shown in Table 1.

Other compounds mentioned in Table 1 were prepared by any one of the above described processes. The processes employed are indicated in the column for Example No. in Table 1.

All the compounds listed in Table 1 were identified by IR spectra and/or $^{1}$H-NMR spectra.

Table 1

| Comp. No. | A | X | Y | Z | $U=\text{-}(C R^1 R^2)_n\text{-}COB$: n | $R^1$ | $R^2$ | B | V | Physical property | Exp. No. |
|---|---|---|---|---|---|---|---|---|---|---|---|
| 1 | H | Cl | H | H | 1 | H | H | $OC_2H_5$ | H | mp 97 ~ 98 ℃ | 6 |
| 2 | 〃 | 〃 | 〃 | 〃 | 〃 | 〃 | 〃 | $NHCH_3$ | 〃 | mp 59 ~ 60 ℃ | 6 |
| 3 | 〃 | 〃 | 〃 | 〃 | 〃 | 〃 | 〃 | $NHCH_2CH=CH_2$ | 〃 | mp 58 ~ 59 ℃ | 6 |
| 4 | 〃 | 〃 | 〃 | 〃 | 〃 | 〃 | 〃 | $N(CH_3)_2$ | 〃 | mp 127 ~ 129 ℃ | 6 |
| 5 | 〃 | 〃 | 〃 | 〃 | 〃 | 〃 | 〃 | $N(C_2H_5)(CH_3)$ | 〃 | mp 106 ~ 107 ℃ | 6 |
| 6 | 〃 | 〃 | 〃 | 〃 | 〃 | 〃 | 〃 | $N(C_3H_7\text{-}n)(CH_3)$ | 〃 | mp 55 ~ 57 ℃ | 6 |
| 7 | 〃 | 〃 | 〃 | 〃 | 〃 | $CH_3$ | 〃 | OH | 〃 | mp 213 ~ 214 ℃ | 10 |
| 8 | 〃 | Br | 〃 | 〃 | 〃 | 〃 | 〃 | 〃 | 〃 | mp 204 ~ 206 ℃ | 10 |
| 9 | 〃 | $CH_3$ | 〃 | 〃 | 〃 | 〃 | 〃 | 〃 | 〃 | mp 174 ~ 175.5 ℃ | 10 |
| 10 | 〃 | $OCH_3$ | 〃 | 〃 | 〃 | 〃 | 〃 | 〃 | 〃 | mp 162 ~ 163 ℃ | 10 |

| Comp. No. | A | X | Y | Z | $U=\left(\overset{R^1}{\underset{R^2}{C}}\right)_n COB$ | | | | V | Physical property | Exp. No. |
|---|---|---|---|---|---|---|---|---|---|---|---|
| | | | | | n | $R^1$ | $R^2$ | B | | | |
| 11 | H | Cl | H | H | 1 | $CH_3$ | H | $OCH_3$ | H | mp 126 ~ 128 ℃ | 6 |
| 12 | 〃 | 〃 | 〃 | 〃 | 〃 | 〃 | 〃 | $OC_2H_5$ | 〃 | mp 109 ~ 110 ℃ | 6 |
| 13 | 〃 | Br | 〃 | 〃 | 〃 | 〃 | 〃 | 〃 | 〃 | mp 109 ~ 111 ℃ | 6 |
| 14 | 〃 | $CH_3$ | 〃 | 〃 | 〃 | 〃 | 〃 | 〃 | 〃 | mp 124 ~ 125 ℃ | 6 |
| 15 | 〃 | $OCH_3$ | 〃 | 〃 | 〃 | 〃 | 〃 | $OC_2H_5$ | 〃 | mp 97 ~ 98.5 ℃ | 6 |
| 16 | 〃 | Cl | 〃 | 〃 | 〃 | 〃 | 〃 | $OC_3H_7$-n | 〃 | $n_D^{28}$ 1.5531 | 6 |
| 17 | 〃 | 〃 | 〃 | 〃 | 〃 | 〃 | 〃 | $OC_3H_7$-i | 〃 | $n_D^{28}$ 1.5501 | 6 |
| 18 | 〃 | 〃 | 〃 | 〃 | 〃 | 〃 | 〃 | $OC_6H_{13}$-n | 〃 | $n_D^{28}$ 1.5432 | 6 |
| 19 | 〃 | 〃 | 〃 | 〃 | 〃 | 〃 | 〃 | $OC_{11}H_{23}$-n | 〃 | $n_D^{28}$ 1.5226 | 6 |
| 20 | 〃 | 〃 | 〃 | 〃 | 〃 | 〃 | 〃 | O 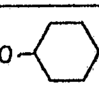 | 〃 | mp 141 ~ 145 ℃ | 6 |

| Comp. № | A | X | Y | Z | $U = (\overset{R^1}{\underset{R^2}{C}})_n COOB$ | | | | V | Physical property | Exp. № |
|---|---|---|---|---|---|---|---|---|---|---|---|
| | | | | | n | $R^1$ | $R^2$ | B | | | |
| 21 | H | Cl | H | H | 1 | $CH_3$ | H | O- 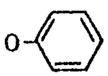 | H | $n_D^{25}$ 1.5733 | 6 |
| 22 | 〃 | 〃 | 〃 | 〃 | 〃 | 〃 | 〃 | $OCH_2$- 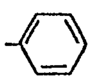 | 〃 | mp 101 ~ 103 ℃ | 6 |
| 23 | 〃 | 〃 | 〃 | 〃 | 〃 | 〃 | 〃 | $CH_3$<br>$OCHCO_2C_2H_5$ | 〃 | $n_D^{25}$ 1.5520 | 12 |
| 24 | 〃 | 〃 | 〃 | 〃 | 〃 | 〃 | 〃 | $C_2H_5$<br>$OCHCO_2C_2H_5$ | 〃 | $n_D^{25}$ 1.5456 | 12 |
| 25 | 〃 | 〃 | 〃 | 〃 | 〃 | 〃 | 〃 | $C_3H_7$-n<br>$OCHCO_2C_2H_5$ | 〃 | $n_D^{25}$ 1.5392 | 12 |
| 26 | 〃 | 〃 | 〃 | 〃 | 〃 | 〃 | 〃 | $CH_3$<br>$OCHCON(CH_3)_2$ | 〃 | Amorphous solid | 12 |
| 27 | 〃 | 〃 | 〃 | 〃 | 〃 | 〃 | 〃 | $CH_3$<br>$OCHCONHC_3H_7$-i | 〃 | Amorphous solid | 12 |
| 28 | 〃 | 〃 | 〃 | 〃 | 〃 | 〃 | 〃 | $C_2H_5$<br>$OCHCONHCH_2CH=CH_2$ | 〃 | mp >130 ℃ | 12 |
| 29 | 〃 | 〃 | 〃 | 〃 | 〃 | 〃 | 〃 | $C_2H_5$<br>$OCHCONHC_4H_9$-i | 〃 | mp 100 ~ 108 ℃ | 12 |

| Comp. No. | A | X | Y | Z | $U=\{C(R^1)(R^2)\}_n COB$ | | | | V | Physical property | Exp. No. |
|---|---|---|---|---|---|---|---|---|---|---|---|
| | | | | | n | $R^1$ | $R^2$ | B | | | |
| 30 | H | Cl | H | H | 1 | $CH_3$ | H | $SC_4H_9$-n | H | $n_D^{25}$ 1.5762 | 6 |
| 31 | 〃 | 〃 | 〃 | 〃 | 〃 | 〃 | 〃 | $NHCH_3$ | 〃 | mp 125 ~ 127 ℃ | 6 |
| 32 | 〃 | 〃 | 〃 | 〃 | 〃 | 〃 | 〃 | $NHC_2H_5$ | 〃 | mp 136 ~ 137 ℃ | 6 |
| 33 | 〃 | 〃 | F | 〃 | 〃 | 〃 | 〃 | 〃 | 〃 | mp 177 ~ 178 ℃ | 6 |
| 34 | 〃 | 〃 | H | 〃 | 〃 | 〃 | 〃 | $NHC_3H_7$-n | 〃 | Amorphous solid | 6 |
| 35 | 〃 | Br | 〃 | 〃 | 〃 | 〃 | 〃 | 〃 | 〃 | mp 98 ~ 102 ℃ | 6 |
| 36 | 〃 | Cl | F | 〃 | 〃 | 〃 | 〃 | 〃 | 〃 | mp 128.5 ~ 129.5 ℃ | 6 |
| 37 | 〃 | Br | 〃 | 〃 | 〃 | 〃 | 〃 | 〃 | 〃 | mp 115 ~ 116.5 ℃ | 6 |
| 38 | 〃 | F | Cl | 〃 | 〃 | 〃 | 〃 | 〃 | 〃 | mp 151.5 ~ 152.5 ℃ | 6 |

| Comp. No. | A | X | Y | Z | $U = \left( C R^1 R^2 \right)_n COB$ n | R¹ | R² | B | V | Physical property | Exp. No. |
|---|---|---|---|---|---|---|---|---|---|---|---|
| | | | | | n | $R^1$ | $R^2$ | B | | | |
| 39 | H | $CH_3$ | H | H | 1 | $CH_3$ | H | $NHC_3H_7$-n | H | mp 145 ~ 146 ℃ | 6 |
| 40 | 〃 | $OCH_3$ | 〃 | 〃 | 〃 | 〃 | 〃 | 〃 | 〃 | mp 145.5 ~ 147 ℃ | 6 |
| 41 | 〃 | Cl | 〃 | 〃 | 〃 | 〃 | 〃 | $NHC_3H_7$-i | 〃 | mp 126 ~ 128 ℃ | 6 |
| 42 | 4-$CH_3$ | 〃 | 〃 | 〃 | 〃 | 〃 | 〃 | 〃 | 〃 | mp 157 ~ 158 ℃ | 6 |
| 43 | H | Cl | F | 〃 | 〃 | 〃 | 〃 | 〃 | 〃 | mp 123 ~ 124.5 ℃ | 6 |
| 44 | 〃 | $CH_3$ | H | 〃 | 〃 | 〃 | 〃 | 〃 | 〃 | Amorphous solid | 6 |
| 45 | 〃 | Cl | 〃 | 〃 | 〃 | 〃 | 〃 | $NHC_4H_9$-n | 〃 | mp 131 ~ 132 ℃ | 6 |
| 46 | 〃 | 〃 | F | 〃 | 〃 | 〃 | 〃 | 〃 | 〃 | Amorphous solid | 6 |
| 47 | 〃 | 〃 | H | 〃 | 〃 | 〃 | 〃 | $NHC_4H_9$-i | 〃 | Amorphous solid | 6 |

| Comp. No. | A | X | Y | Z | $U=\left(\overset{R^1}{\underset{R^2}{C}}\right)_n COB$ | | | | V | Physical property | Exp. No. |
|---|---|---|---|---|---|---|---|---|---|---|---|
| | | | | | n | $R^1$ | $R^2$ | B | | | |
| 48 | H | Cl | F | H | 1 | $CH_3$ | H | $NHC_4H_9$-i | H | Amorphous solid | 6 |
| 49 | 〃 | H | H | 〃 | 〃 | 〃 | 〃 | $NHC_4H_9$-sec | 〃 | mp 83.5 ~ 85.5 ℃ | 6 |
| 50 | 〃 | Cl | 〃 | 〃 | 〃 | 〃 | 〃 | 〃 | 〃 | Amorphous solid | 6 |
| 51 | 〃 | H | F | 〃 | 〃 | 〃 | 〃 | 〃 | 〃 | mp 166 ~ 168.5 ℃ | 3 |
| 52 | 〃 | Cl | 〃 | 〃 | 〃 | 〃 | 〃 | 〃 | 〃 | mp 161.5 ~ 162.5 ℃ | 6 |
| 53 | 〃 | F | 〃 | 〃 | 〃 | 〃 | 〃 | 〃 | 〃 | mp 157 ~ 158 ℃ | 6 |
| 54 | 〃 | Cl | Cl | 〃 | 〃 | 〃 | 〃 | 〃 | 〃 | mp 154 ~ 155.5 ℃ | 6 |
| 55 | 〃 | Br | F | Br | 〃 | 〃 | 〃 | 〃 | 〃 | Amorphous solid | 8 |
| 56 | 〃 | Cl | H | H | 〃 | 〃 | 〃 | $NHC_4H_9$-t | 〃 | mp 201.5 ~ 202.5 ℃ | 6 |

| Comp. No. | A | X | Y | Z | $U = -(\overset{R^1}{\underset{R^2}{C}})_n-COB$ | | | | V | Physical property | Exp. No. |
|---|---|---|---|---|---|---|---|---|---|---|---|
| | | | | | n | $R^1$ | $R^2$ | B | | | |
| 57 | H | Cl | H | H | 1 | $CH_3$ | H | $NHC_5H_{11}$-n | H | Amorphous solid | 6 |
| 58 | 〃 | 〃 | 〃 | 〃 | 〃 | 〃 | 〃 | $NHC_5H_{11}$-i | 〃 | mp 136.5 ~ 138 ℃ | 6 |
| 59 | 〃 | 〃 | F | 〃 | 〃 | 〃 | 〃 | 〃 | 〃 | mp 148.5 ~ 149.5 ℃ | 6 |
| 60 | 〃 | F | 〃 | 〃 | 〃 | 〃 | 〃 | 〃 | 〃 | mp 146 ~ 147 ℃ | 6 |
| 61 | 〃 | Cl | H | 〃 | 〃 | 〃 | 〃 | $NHC_5H_{11}$-neo | 〃 | mp 156.5 ~ 157.5 ℃ | 6 |
| 62 | 〃 | 〃 | F | 〃 | 〃 | 〃 | 〃 | 〃 | 〃 | mp 172 ~ 173 ℃ | 6 |
| 63 | 〃 | 〃 | H | 〃 | 〃 | 〃 | 〃 | $NHC_5H_{11}$-t | 〃 | mp 180 ~ 181 ℃ | 6 |
| 64 | 〃 | 〃 | 〃 | 〃 | 〃 | 〃 | 〃 | $NHCH_2\underset{CH_3}{C}HCO_2H_5$ | 〃 | mp 134.5 ~ 136.5 ℃ | 6 |
| 65 | 〃 | 〃 | F | 〃 | 〃 | 〃 | 〃 | 〃 | 〃 | mp 158.5 ~ 160.5 ℃ | 6 |

| Comp. No. | A | X | Y | Z | $U = (\overset{R^1}{\underset{R^2}{C}})_n COB$ | | | | V | Physical property | Exp. No. |
|---|---|---|---|---|---|---|---|---|---|---|---|
| | | | | | n | $R^1$ | $R^2$ | B | | | |
| 66 | H | F | F | H | 1 | $CH_3$ | H | $NHCH_2CHC_2H_5$<br>$CH_3$ | H | mp 158 ℃ | 6 |
| 67 | 〃 | Cl | Cl | 〃 | 〃 | 〃 | 〃 | 〃 | 〃 | mp 156 ~ 157 ℃ | 6 |
| 68 | 〃 | 〃 | H | 〃 | 〃 | 〃 | 〃 | $NHCH(C_2H_5)_2$ | 〃 | mp 134 ~ 135 ℃ | 6 |
| 69 | 〃 | 〃 | F | 〃 | 〃 | 〃 | 〃 | 〃 | 〃 | mp 157.5 ~ 158.5 ℃ | 6 |
| 70 | 〃 | F | 〃 | 〃 | 〃 | 〃 | 〃 | 〃 | 〃 | mp 155 ~ 155.5 ℃ | 6 |
| 71 | 〃 | Cl | H | 〃 | 〃 | 〃 | 〃 | $NHCHCH(CH_3)_2$<br>$CH_3$ | 〃 | mp 165 ~ 167 ℃ | 6 |
| 72 | 〃 | 〃 | F | 〃 | 〃 | 〃 | 〃 | 〃 | 〃 | mp 164 ~ 166.5 ℃ | 6 |
| 73 | 〃 | F | 〃 | 〃 | 〃 | 〃 | 〃 | 〃 | 〃 | mp 145 ~ 146.5 ℃ | 6 |
| 74 | 〃 | Cl | H | 〃 | 〃 | 〃 | 〃 | $NHC_6H_{13}$-n | 〃 | Amorphous solid | 6 |

| Comp. No. | A | X | Y | Z | $U = -(C R^1 R^2)_n - COB$ | | | | V | Physical property | Exp. No. |
|---|---|---|---|---|---|---|---|---|---|---|---|
| | | | | | n | $R^1$ | $R^2$ | B | | | |
| 75 | H | Cl | H | H | 1 | $CH_3$ | H | $NH(CH_2)_2C(CH_3)_3$ | H | mp 142 ~ 143 ℃ | 6 |
| 76 | 〃 | 〃 | F | 〃 | 〃 | 〃 | 〃 | 〃 | 〃 | mp 189 ~ 190 ℃ | 6 |
| 77 | 〃 | 〃 | H | 〃 | 〃 | 〃 | 〃 | $NH\underset{CH_3}{CH}(CH_2)_4CH_3$ | 〃 | mp 137.5 ~ 138.5 ℃ | 5 |
| 78 | 〃 | 〃 | 〃 | 〃 | 〃 | 〃 | 〃 | 〃 | 〃 | mp 148.5 ~ 150 ℃ | 5 |
| 79 | 〃 | 〃 | F | 〃 | 〃 | 〃 | 〃 | 〃 | 〃 | mp 134.5 ~ 136 ℃ | 5 |
| 80 | 〃 | 〃 | 〃 | 〃 | 〃 | 〃 | 〃 | 〃 | 〃 | mp 123 ~ 124 ℃ | 5 |
| 81 | 〃 | 〃 | H | 〃 | 〃 | 〃 | 〃 | $NHCH_2\underset{C_2H_5}{CH}C_4H_9-n$ | 〃 | Amorphous solid | 6 |
| 82 | 〃 | 〃 | 〃 | 〃 | 〃 | 〃 | 〃 | $NHC_{12}H_{25}-n$ | 〃 | $n_D^{25}$ 1.5439 | 6 |
| 83 | 〃 | 〃 | 〃 | 〃 | 〃 | 〃 | 〃 | $NHCH_2CH=CH_2$ | 〃 | mp 112 ~ 113 ℃ | 6 |

| Comp. No. | A | X | Y | Z | $U = (\overset{R^1}{\underset{R^2}{C}})_n COB$ | | | | V | Physical property | Exp. No. |
|---|---|---|---|---|---|---|---|---|---|---|---|
| | | | | | n | $R^1$ | $R^2$ | B | | | |
| 84 | H | Cl | H | H | 1 | $CH_3$ | H | $NHCH_2CH=CHCH_3$ | H | mp 169 ~ 171 ℃ | 6 |
| 85 | " | " | " | " | " | " | " | $NHCH_2CH_2CH=CH_2$ | " | mp 147.5 ~ 149 ℃ | 6 |
| 86 | " | " | " | " | " | " | " | $NHCH_2C≡CH$ | " | mp 178 ~ 179 ℃ | 6 |
| 87 | " | " | " | " | " | " | " | NH- 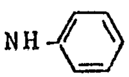 | " | mp 92 ~ 93 ℃ | 6 |
| 88 | " | " | " | " | " | " | " | $NHCH_2$- 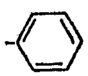 | " | mp 124 ~ 126 ℃ | 6 |
| 89 | " | " | " | " | " | " | " | NH- 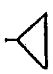 | " | mp 155 ~ 156 ℃ | 6 |
| 90 | " | " | " | " | " | " | " | NH- 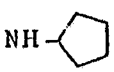 | " | mp 123 ~ 125 ℃ | 6 |
| 91 | " | " | " | " | " | " | " | NH- 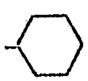 | " | mp 94 ~ 95 ℃ | 6 |
| 92 | " | " | " | " | " | " | " | $NHCH_2CO_2C_2H_5$ | " | Amorphous solid | 11 |

| Comp. No. | A | X | Y | Z | $U = (\overset{R^1}{\underset{R^2}{C}})_n COB$ | | | | V | Physical property | Exp. No. |
|---|---|---|---|---|---|---|---|---|---|---|---|
| | | | | | n | $R^1$ | $R^2$ | B | | | |
| 93 | H | Cl | H | H | 1 | $CH_3$ | H | $NH\overset{CH_3}{C}HCO_2C_2H_5$ | H | mp 149 ~ 151 ℃ | 11 |
| 94 | 〃 | $CH_3$ | 〃 | 〃 | 〃 | 〃 | 〃 | 〃 | 〃 | mp 125 ~ 126 ℃ | 11 |
| 95 | 〃 | Cl | 〃 | 〃 | 〃 | 〃 | 〃 | $NH\overset{CH_3}{C}HCO_2C_4H_9\text{-}i$ | 〃 | mp 145 ~ 147 ℃ | 11 |
| 96 | 〃 | 〃 | 〃 | 〃 | 〃 | 〃 | 〃 | $NH\overset{CH_3}{C}HCONHC_3H_7\text{-}n$ | 〃 | mp 173 ~ 177 ℃ | 11 |
| 97 | 〃 | 〃 | 〃 | 〃 | 〃 | 〃 | 〃 | $NH\overset{CH_3}{C}HCON(CH_3)_2$ | 〃 | mp 173 ~ 175 ℃ | 11 |
| 98 | 〃 | 〃 | 〃 | 〃 | 〃 | $C_2H_5$ | 〃 | $NH\overset{CH_3}{C}HCONHC_3H_7\text{-}i$ | 〃 | mp 165 ~ 167 ℃ | 11 |
| 99 | 〃 | 〃 | 〃 | 〃 | 〃 | $CH_3$ | 〃 | $NH\overset{C_3H_7\text{-}i}{C}HCONHC_3H_7\text{-}i$ | 〃 | mp 114 ~ 116 ℃ | 11 |
| 100 | 〃 | 〃 | 〃 | 〃 | 〃 | 〃 | 〃 | $N(CH_3)_2$ | 〃 | mp 167 ~ 167.5 ℃ | 6 |
| 101 | 〃 | Br | 〃 | 〃 | 〃 | 〃 | 〃 | 〃 | 〃 | mp 196 ~ 198 ℃ | 6 |

| Comp. No. | A | X | Y | Z | $U = \left( \overset{R^1}{\underset{R^2}{C}} \right)_n COB$ | | | | V | Physical property | Exp. No. |
|---|---|---|---|---|---|---|---|---|---|---|---|
| | | | | | n | $R^1$ | $R^2$ | B | | | |
| 102 | H | $Cl$ | F | H | 1 | $CH_3$ | H | $N(CH_3)_2$ | H | mp 190 ~ 191 ℃ | 6 |
| 103 | 〃 | Br | 〃 | 〃 | 〃 | 〃 | 〃 | 〃 | 〃 | mp 162 ~ 163 ℃ | 6 |
| 104 | 〃 | F | $Cl$ | 〃 | 〃 | 〃 | 〃 | 〃 | 〃 | mp 189 ~ 190.5 ℃ | 6 |
| 105 | 〃 | $Cl$ | H | 〃 | 〃 | 〃 | 〃 | $N{<}^{C_2H_5}_{CH_3}$ | 〃 | mp 86 ~ 89 ℃ | 6 |
| 106 | 〃 | 〃 | 〃 | 〃 | 〃 | 〃 | 〃 | $N{<}^{C_3H_7\text{-}n}_{CH_3}$ | 〃 | Amorphous solid | 6 |
| 107 | 〃 | 〃 | 〃 | 〃 | 〃 | 〃 | 〃 | $N{<}^{C_4H_9\text{-}n}_{CH_3}$ | 〃 | Amorphous solid | 6 |
| 108 | 〃 | 〃 | 〃 | 〃 | 〃 | 〃 | 〃 | $N(C_2H_5)_2$ | 〃 | mp 50 ~ 50.5 ℃ | 6 |
| 109 | 〃 | 〃 | 〃 | 〃 | 〃 | 〃 | 〃 | $N{<}^{OCH_3}_{CH_3}$ | 〃 | mp 169.5 ~ 172 ℃ | 6 |
| 110 | 〃 | $CH_3$ | 〃 | 〃 | 〃 | 〃 | 〃 | 〃 | 〃 | mp 163 ~ 164 ℃ | 6 |

| Comp. No. | A | X | Y | Z | $U = (\overset{R^1}{\underset{R^2}{C}})_n COB$ | | | | V | Physical property | Exp. No. |
|---|---|---|---|---|---|---|---|---|---|---|---|
| | | | | | n | $R^1$ | $R^2$ | B | | | |
| 111 | H | Cl | H | H | 1 | $CH_3$ | H | $N<^{OCH_3}_{C_2H_5}$ | H | mp 118 ~ 119.5 ℃ | 6 |
| 112 | " | " | " | " | " | " | " | $N<^{OCH_3}_{C_3H_7-n}$ | " | $n_D^{25.5}$ 1.5665 | 6 |
| 113 | " | " | " | " | " | " | " | $N<^{OC_2H_5}_{CH_3}$ | " | mp 127 ~ 128 ℃ | 6 |
| 114 | " | " | F | " | " | " | " | " | " | mp 150 ~ 151 ℃ | 6 |
| 115 | " | " | H | " | " | " | " | $N<^{OC_2H_5}_{C_2H_5}$ | " | mp 100.5 ~ 102.5 ℃ | 6 |
| 116 | " | " | " | " | " | " | " | $N<^{(CH_2)_2OCH_3}_{CH_3}$ | " | $n_D^{25}$ 1.5712 | 6 |
| 117 | " | " | " | " | " | " | " | $N<^{CH_2CH=CH_2}_{CH_3}$ | " | $n_D^{25}$ 1.5609 | 6 |
| 118 | " | " | " | " | " | " | " | $N<^{CH_2CH=CH_2}_{OCH_3}$ | " | mp 97.5 ~ 98.5 ℃ | 6 |
| 119 | " | " | " | " | " | " | " | $N(CH_2CH=CH_2)_2$ | " | $n_D^{25.5}$ 1.5796 | 6 |

| Comp. No. | A | X | Y | Z | $U = \left( \overset{R^1}{\underset{R^2}{C}} \right)_n COB$ n | R¹ | R² | B | V | Physical property | Exp. No. |
|---|---|---|---|---|---|---|---|---|---|---|---|
| 120 | H | Cl | H | H | 1 | $CH_3$ | H | 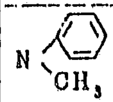 | H | mp 118 ~ 120 ℃ | 6 |
| 121 | 〃 | 〃 | 〃 | 〃 | 〃 | 〃 | 〃 | 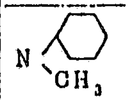 | 〃 | Amorphous solid | 11 |
| 122 | 〃 | 〃 | 〃 | 〃 | 〃 | 〃 | 〃 | 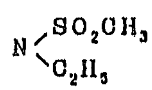 | 〃 | mp 121 ~ 125 ℃ | 6 |
| 123 | 〃 | 〃 | 〃 | 〃 | 〃 | 〃 | 〃 | 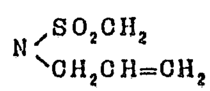 | 〃 | mp 127 ~ 132 ℃ | 6 |
| 124 | 〃 | 〃 | 〃 | 〃 | 〃 | 〃 | 〃 | 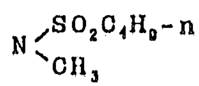 | 〃 | mp 178 ~ 179 ℃ | 6 |
| 125 | 〃 | 〃 | 〃 | 〃 | 〃 | 〃 | 〃 | 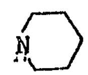 | 〃 | Amorphous solid | 6 |
| 126 | 〃 | 〃 | 〃 | 〃 | 〃 | 〃 | 〃 | 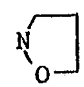 | 〃 | mp 157 ~ 155.5 ℃ | 6 |
| 127 | 〃 | 〃 | 〃 | 〃 | 〃 | 〃 | 〃 | 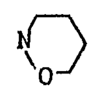 | 〃 | mp 136 ~ 137 ℃ | 6 |
| 128 | 〃 | 〃 | 〃 | 〃 | 〃 | 〃 | 〃 | 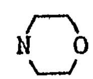 | 〃 | mp 152 ~ 153 ℃ | 6 |

| Comp. No. | A | X | Y | Z | $U = \left( \overset{R^1}{\underset{R^2}{C}} \right)_n COOB$ | | | | V | Physical property | Exp. No. |
|---|---|---|---|---|---|---|---|---|---|---|---|
| | | | | | n | $R^1$ | $R^2$ | B | | | |
| 129 | H | Cl | H | H | 1 | $CH_3$ | $CH_3$ | $OC_2H_5$ | H | mp 92.5 ~ 93.5 ℃ | 6 |
| 130 | 〃 | 〃 | 〃 | 〃 | 〃 | $C_2H_5$ | H | OH | 〃 | mp 164 ~ 166 ℃ | 10 |
| 131 | 〃 | H | 〃 | 〃 | 〃 | 〃 | 〃 | $OC_2H_5$ | 〃 | mp 115 ~ 116 ℃ | 6 |
| 132 | 〃 | Cl | 〃 | 〃 | 〃 | 〃 | 〃 | 〃 | 〃 | mp 105.5 ~ 106.5 ℃ | 6 |
| 133 | 〃 | Br | 〃 | 〃 | 〃 | 〃 | 〃 | 〃 | 〃 | mp 81 ~ 83 ℃ | 6 |
| 134 | 〃 | H | F | 〃 | 〃 | 〃 | 〃 | 〃 | 〃 | Amorphous solid | 6 |
| 135 | 〃 | 〃 | 〃 | Cl | 〃 | 〃 | 〃 | 〃 | 〃 | mp 116 ~ 117.5 ℃ | 9 |
| 136 | 〃 | Cl | 〃 | 〃 | 〃 | 〃 | 〃 | 〃 | 〃 | $n_D^{25}$ 1.5517 | 8 |
| 137 | 〃 | 〃 | H | H | 〃 | 〃 | 〃 | $NHCH_3$ | 〃 | Amorphous solid | 6 |

| Comp. No. | A | X | Y | Z | $U = \left( C(R^1)(R^2) \right)_n COB$ | | | | V | Physical property | Exp. No. |
|---|---|---|---|---|---|---|---|---|---|---|---|
| | | | | | n | $R^1$ | $R^2$ | B | | | |
| 138 | H | Cl | H | H | 1 | $C_2H_5$ | H | $NHC_2H_5$ | H | mp 173 ~ 174 ℃ | 6 |
| 139 | 〃 | 〃 | 〃 | 〃 | 〃 | 〃 | 〃 | $NHC_3H_7$-n | 〃 | mp 121 ~ 124 ℃ | 6 |
| 140 | 〃 | 〃 | F | 〃 | 〃 | 〃 | 〃 | 〃 | 〃 | mp 172 ~ 173 ℃ | 6 |
| 141 | 〃 | Cl | H | 〃 | 〃 | 〃 | 〃 | $NHC_3H_7$-i | 〃 | mp 121 ~ 122 ℃ | 6 |
| 142 | 〃 | 〃 | F | 〃 | 〃 | 〃 | 〃 | 〃 | 〃 | mp 185 ~ 187.5 ℃ | 6 |
| 143 | 〃 | 〃 | H | 〃 | 〃 | 〃 | 〃 | $NHC_4H_9$-n | 〃 | mp 129 ~ 130 ℃ | 6 |
| 144 | 〃 | 〃 | F | 〃 | 〃 | 〃 | 〃 | 〃 | 〃 | mp 141 ~ 142 ℃ | 6 |
| 145 | 〃 | 〃 | H | 〃 | 〃 | 〃 | 〃 | $NHC_4H_9$-i | 〃 | mp 136 ~ 137.5 ℃ | 4 |
| 146 | 3-$CH_3$ | 〃 | 〃 | 〃 | 〃 | 〃 | 〃 | 〃 | 〃 | mp 173 ~ 175 ℃ | 6 |

| Comp. No. | A | X | Y | Z | $U=(\overset{R^1}{\underset{R^2}{C}})_nCOB$ n | $R^1$ | $R^2$ | B | V | Physical property | Exp. No. |
|---|---|---|---|---|---|---|---|---|---|---|---|
| 147 | 4-$CH_3$ | Cl | H | H | 1 | $C_2H_5$ | H | $NHC_4H_9$-i | H | mp 65 ~ 67 ℃ | 6 |
| 148 | H | ″ | F | ″ | ″ | ″ | ″ | ″ | ″ | mp 166.5 ~ 167.5 ℃ | 6 |
| 149 | ″ | F | ″ | ″ | ″ | ″ | ″ | ″ | ″ | mp 110 ~ 111 ℃ | 6 |
| 150 | ″ | Cl | Cl | ″ | ″ | ″ | ″ | ″ | ″ | Amorphous solid | 6 |
| 151 | ″ | ″ | H | ″ | ″ | ″ | ″ | $NHC_4H_9$-sec | ″ | mp 123 ~ 127 ℃ | 4 |
| 152 | 3-$CH_3$ | ″ | ″ | ″ | ″ | ″ | ″ | ″ | ″ | mp 158 ~ 160 ℃ | 6 |
| 153 | 4-$CH_3$ | ″ | ″ | ″ | ″ | ″ | ″ | ″ | ″ | mp 64 ~ 66 ℃ | 6 |
| 154 | H | ″ | F | ″ | ″ | ″ | ″ | ″ | ″ | mp 162 ~ 166 ℃ | 6 |
| 155 | ″ | F | F | ″ | ″ | ″ | ″ | ″ | ″ | mp 142 ~ 143 ℃ | 6 |

| Comp. No. | A | X | Y | Z | $U = (\overset{R^1}{\underset{R^2}{C}})_n COB$ | | | | V | Physical property | Exp. No. |
|---|---|---|---|---|---|---|---|---|---|---|---|
| | | | | | n | $R^1$ | $R^2$ | B | | | |
| 156 | H | Cl | Cl | H | 1 | $C_2H_5$ | H | $NHC_4H_9$-sec | H | mp 157 ~ 158 ℃ | 6 |
| 157 | ″ | ″ | H | ″ | ″ | ″ | ″ | $NHC_4H_9$-t | ″ | mp 150 ~ 151 ℃ | 6 |
| 158 | ″ | ″ | F | ″ | ″ | ″ | ″ | ″ | ″ | mp 185 ~ 186 ℃ | 6 |
| 159 | ″ | ″ | H | ″ | ″ | ″ | ″ | $NHC_5H_{11}$-n | ″ | Amorphous solid | 6 |
| 160 | ″ | ″ | ″ | ″ | ″ | ″ | ″ | $NHC_5H_{11}$-i | ″ | mp 122.5 ~ 123.5 ℃ | 6 |
| 161 | ″ | ″ | F | ″ | ″ | ″ | ″ | ″ | ″ | mp 117.5 ~ 118.5 ℃ | 6 |
| 162 | ″ | ″ | H | ″ | ″ | ″ | ″ | $NHC_5H_{11}$-neo | ″ | mp 154.5 ~ 155.5 ℃ | 6 |
| 163 | ″ | ″ | F | ″ | ″ | ″ | ″ | ″ | ″ | mp 188 ~ 189 ℃ | 6 |
| 164 | ″ | ″ | H | ″ | ″ | ″ | ″ | $NHC_5H_{11}$-t | ″ | mp 128 ~ 129 ℃ | 6 |

| Comp. No. | A | X | Y | Z | $U = -\!(\overset{R^1}{\underset{R^2}{C}})_n\!-COB$ | | | | V | Physical property | Exp. No. |
|---|---|---|---|---|---|---|---|---|---|---|---|
| | | | | | n | $R^1$ | $R^2$ | B | | | |
| 165 | H | Cl | F | H | 1 | $C_2H_5$ | H | $NHC_5H_{11}$-t | H | mp 162.5 ~ 163.5 ℃ | 6 |
| 166 | 〃 | 〃 | H | 〃 | 〃 | 〃 | 〃 | $NHCH_2\underset{CH_3}{C}HC_2H_5$ | 〃 | mp 105 ~ 107 ℃ | 7 |
| 167 | 〃 | 〃 | F | 〃 | 〃 | 〃 | 〃 | 〃 | 〃 | mp 147 ~ 149 ℃ | 6 |
| 168 | 〃 | 〃 | H | 〃 | 〃 | 〃 | 〃 | $NHCH(C_2H_5)_2$ | 〃 | mp 144.5 ~ 146.5 ℃ | 6 |
| 169 | 〃 | 〃 | F | 〃 | 〃 | 〃 | 〃 | $NHCH(C_2H_5)_2$ | 〃 | mp 151.5 ~ 152.5 ℃ | 6 |
| 170 | 〃 | 〃 | H | 〃 | 〃 | 〃 | 〃 | $NH\underset{CH_3}{C}HCH(CH_3)_2$ | 〃 | mp 150 ~ 155 ℃ | 6 |
| 171 | 〃 | 〃 | F | 〃 | 〃 | 〃 | 〃 | 〃 | 〃 | mp 180.5 ~ 182 ℃ | 6 |
| 172 | 〃 | 〃 | H | 〃 | 〃 | 〃 | 〃 | $NH\underset{CH_3}{C}H(CH_2)_4CH_3$ | 〃 | mp 140.5 ~ 142 ℃ | 6 |
| 173 | 〃 | 〃 | F | 〃 | 〃 | 〃 | 〃 | 〃 | 〃 | mp 137.5 ~ 139 ℃ | 6 |

| Comp. No. | A | X | Y | Z | $U = (\overset{R^1}{\underset{R^2}{C}})_n COB$ | | | | V | Physical property | Exp. No. |
|---|---|---|---|---|---|---|---|---|---|---|---|
| | | | | | n | $R^1$ | $R^2$ | B | | | |
| 174 | H | Cl | H | H | 1 | $C_2H_5$ | H | $NHCH_2CH{=}CH_2$ | H | mp 134 ~ 135 ℃ | 5 |
| 175 | ″ | ″ | F | ″ | ″ | ″ | ″ | ″ | ″ | mp 153.5 ~ 154.5 ℃ | 6 |
| 176 | ″ | ″ | H | ″ | ″ | ″ | ″ | $NHCH_2C{\equiv}CH$ | ″ | mp 102 ~ 103 ℃ | 6 |
| 177 | ″ | ″ | ″ | ″ | ″ | ″ | ″ | $NH\overset{CH_3}{\underset{CH_3}{C}}C{\equiv}CH$ | ″ | mp 56 ~ 58 ℃ | 6 |
| 178 | ″ | ″ | ″ | ″ | ″ | ″ | ″ | $N(CH_3)_2$ | ″ | mp 137 ~ 138 ℃ | 6 |
| 179 | ″ | ″ | ″ | ″ | ″ | ″ | ″ | $N\begin{smallmatrix}C_2H_5\\CH_3\end{smallmatrix}$ | ″ | Amorphous solid | 6 |
| 180 | ″ | ″ | F | ″ | ″ | ″ | ″ | ″ | ″ | Amorphous solid | 6 |
| 181 | ″ | ″ | H | ″ | ″ | ″ | ″ | $N\begin{smallmatrix}C_3H_7\text{-}n\\CH_3\end{smallmatrix}$ | ″ | Amorphous solid | 6 |
| 182 | ″ | ″ | ″ | ″ | ″ | ″ | ″ | $N\begin{smallmatrix}OCH_3\\CH_3\end{smallmatrix}$ | ″ | mp 109 ~ 110 ℃ | 6 |

| Comp. No. | A | X | Y | Z | $U = -(C(R^1)(R^2))_n-COB$ | | | | V | Physical property | Exp. No. |
|---|---|---|---|---|---|---|---|---|---|---|---|
| | | | | | n | $R^1$ | $R^2$ | B | | | |
| 183 | H | Cl | H | H | 1 | $C_2H_5$ | H | $N\langle^{OCH_3}_{C_2H_5}$ | H | mp 101 ~ 103 ℃ | 6 |
| 184 | 〃 | 〃 | 〃 | 〃 | 〃 | 〃 | 〃 | $N\langle^{OCH_3}_{C_3H_7-n}$ | 〃 | $n_D^{25.5}$ 1.5611 | 6 |
| 185 | 〃 | 〃 | 〃 | 〃 | 〃 | 〃 | 〃 | $N\langle^{OC_2H_5}_{CH_3}$ | 〃 | mp 104 ~ 105 ℃ | 6 |
| 186 | 〃 | 〃 | 〃 | 〃 | 〃 | 〃 | 〃 | $N\langle^{CH_2CH=CH_2}_{CH_3}$ | 〃 | Amorphous solid | 6 |
| 187 | 〃 | 〃 | 〃 | 〃 | 〃 | 〃 | 〃 | $N\langle^{CH_2CH=CH_2}_{OCH_3}$ | 〃 | $n_D^{25.5}$ 1.5715 | 6 |
| 188 | 〃 | 〃 | 〃 | 〃 | 〃 | 〃 | 〃 | $N(CH_2CH=CH_2)_2$ | 〃 | mp 130 ~ 131 ℃ | 6 |
| 189 | 〃 | 〃 | 〃 | 〃 | 〃 | $n-C_3H_7$ | 〃 | OH | 〃 | mp 164 ~ 166 ℃ | 10 |
| 190 | 〃 | H | 〃 | 〃 | 〃 | 〃 | 〃 | $OC_2H_5$ | 〃 | mp 84 ~ 85 ℃ | 6 |
| 191 | 〃 | Cl | 〃 | 〃 | 〃 | 〃 | 〃 | 〃 | 〃 | mp 81 ~ 82 ℃ | 6 |

| Comp. No. | A | X | Y | Z | $U=\left(\overset{R^1}{\underset{R^2}{C}}\right)_n COB$ | | | | V | Physical property | Exp. No. |
|---|---|---|---|---|---|---|---|---|---|---|---|
| | | | | | n | $R^1$ | $R^2$ | B | | | |
| 192 | H | Br | H | H | 1 | n-$C_3H_7$ | H | $OC_2H_5$ | H | mp 86 ~ 88 ℃ | 6 |
| 193 | 〃 | Cl | 〃 | 〃 | 〃 | 〃 | 〃 | $OC_3H_7$-n | 〃 | mp 69.5 ~ 70.5 ℃ | 6 |
| 194 | 〃 | Br | 〃 | 〃 | 〃 | 〃 | 〃 | 〃 | 〃 | Semi-solid | 6 |
| 195 | 〃 | Cl | 〃 | 〃 | 〃 | 〃 | 〃 | $OC_3H_7$-i | 〃 | mp 88 ~ 88.5 ℃ | 6 |
| 196 | 〃 | 〃 | 〃 | 〃 | 〃 | 〃 | 〃 | $OCH_2CH=CH_2$ | 〃 | $n_D^{25}$ 1.5278 | 6 |
| 197 | 〃 | 〃 | 〃 | 〃 | 〃 | 〃 | 〃 | $OCH_2C\equiv CH$ | 〃 | $n_D^{25}$ 1.4859 | 6 |
| 198 | 〃 | 〃 | 〃 | 〃 | 〃 | 〃 | 〃 | $NHCH_3$ | 〃 | Amorphous solid | 6 |
| 199 | 〃 | 〃 | 〃 | 〃 | 〃 | 〃 | 〃 | $NHC_2H_5$ | 〃 | mp 183 ~ 186 ℃ | 6 |
| 200 | 〃 | 〃 | 〃 | 〃 | 〃 | 〃 | 〃 | $NHC_3H_7$-n | 〃 | mp 167 ~ 169 ℃ | 6 |

| Comp. No. | A | X | Y | Z | $U = \left( \overset{R^1}{\underset{R^2}{C}} \right)_n COB$ | | | | V | Physical property | Exp. No. |
|---|---|---|---|---|---|---|---|---|---|---|---|
| | | | | | n | $R^1$ | $R^2$ | B | | | |
| 201 | H | Cl | H | H | 1 | $n-C_3H_7$ | H | $NHC_3H_7-i$ | H | mp 175 ~ 176 ℃ | 6 |
| 202 | 〃 | 〃 | 〃 | 〃 | 〃 | 〃 | 〃 | $NHCH_2CH=CH_2$ | 〃 | mp 165 ~ 167 ℃ | 6 |
| 203 | 〃 | 〃 | 〃 | 〃 | 〃 | 〃 | 〃 | $NHCH_2C≡CH$ | 〃 | mp 150.5 ~ 152 ℃ | 6 |
| 204 | 〃 | 〃 | 〃 | 〃 | 〃 | 〃 | 〃 | $NHCH_2$- 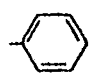 | 〃 | mp 57 ~ 58 ℃ | 6 |
| 205 | 〃 | 〃 | 〃 | 〃 | 〃 | 〃 | 〃 | NH- 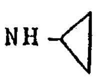 | 〃 | mp 195 ~ 197 ℃ | 6 |
| 206 | 〃 | 〃 | 〃 | 〃 | 〃 | 〃 | 〃 | $N(CH_3)_2$ | 〃 | Amorphous solid | 6 |
| 207 | 〃 | 〃 | 〃 | 〃 | 〃 | 〃 | 〃 | $N(C_2H_5)(CH_3)$ | 〃 | $n_D^{25}$ 1.5405 | 6 |
| 208 | 〃 | 〃 | 〃 | 〃 | 〃 | 〃 | 〃 | $N(OCH_3)(CH_3)$ | 〃 | mp 101 ~ 101.5 ℃ | 11 |
| 209 | 〃 | 〃 | 〃 | 〃 | 〃 | 〃 | 〃 | $N(CH_2CH=CH_2)(CH_3)$ | 〃 | $n_D^{25}$ 1.5595 | 6 |

| Comp. No. | A | X | Y | Z | 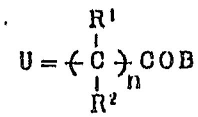 | | | | V | Physical property | Exp. No. |
|---|---|---|---|---|---|---|---|---|---|---|---|
| | | | | | n | $R^1$ | $R^2$ | B | | | |
| 210 | H | Cl | H | H | 1 | n-$C_3H_7$ | H | 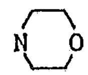 | H | $n_D^{25}$ 1.5469 | 6 |
| 211 | 〃 | 〃 | 〃 | 〃 | 〃 | n-$C_4H_9$ | 〃 | $OC_2H_5$ | 〃 | mp 73 ~ 75 ℃ | 6 |
| 212 | 〃 | Br | 〃 | 〃 | 〃 | 〃 | 〃 | 〃 | 〃 | mp 86 ~ 88 ℃ | 6 |
| 213 | 〃 | Cl | 〃 | 〃 | 〃 | n-$C_6H_{13}$ | 〃 | 〃 | 〃 | $n_D^{27}$ 1.5223 | 6 |
| 214 | 〃 | 〃 | 〃 | 〃 | 〃 | 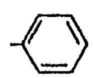 | 〃 | 〃 | 〃 | mp 111.5 ~ 112.5 ℃ | 6 |
| 215 | 〃 | 〃 | 〃 | 〃 | 〃 | $CH_3$ | 〃 | 〃 | $COCH_3$ | mp 115.5 ~ 116.5 ℃ | 13 |
| 216 | 〃 | 〃 | 〃 | 〃 | 〃 | 〃 | 〃 | 〃 | n-$C_3H_7$ | mp 65 ~ 66 ℃ | 6 |
| 217 | 〃 | 〃 | 〃 | 〃 | 〃 | 〃 | 〃 | $NHC_3H_7$-i | $COCH_3$ | mp 228 ~ 230 ℃ | 13 |
| 218 | 〃 | 〃 | 〃 | 〃 | 〃 | 〃 | 〃 | 〃 | $COC_3H_7$-n | mp 151 ~ 152 ℃ | 13 |

| Comp. No. | A | X | Y | Z | $U = \left( C(R^1)(R^2) \right)_n COB$ | | | | V | Physical property | Exp. No. |
|---|---|---|---|---|---|---|---|---|---|---|---|
| | | | | | n | $R^1$ | $R^2$ | B | | | |
| 219 | H | Cl | H | H | 1 | $CH_3$ | H | $NHC_3H_7$-i | $COCH_2Cl$ | mp 174 ~ 175.5 ℃ | 13 |
| 220 | 〃 | 〃 | 〃 | 〃 | 〃 | 〃 | 〃 | 〃 | $COCF_3$ | mp 201 ~ 203 ℃ | 13 |
| 221 | 〃 | H | 〃 | 〃 | 〃 | 〃 | 〃 | $NHC_4H_9$-i | $CO_2C_2H_5$ | Amorphous solid | 13 |
| 222 | 〃 | Cl | 〃 | 〃 | 3 | H | 〃 | $OC_2H_5$ | H | mp 86.5 ~ 87.5 ℃ | 6 |
| 223 | 〃 | 〃 | 〃 | 〃 | 〃 | 〃 | 〃 | 〃 | $COCH_3$ | Amorphous solid | 14 |
| 224 | 〃 | 〃 | 〃 | 〃 | 4 | 〃 | 〃 | 〃 | H | mp 105 ~ 107 ℃ | 6 |

| Comp. No. | A | X | Y | Z | U | V | Physical property | Exp. No. |
|---|---|---|---|---|---|---|---|---|
| 225 | H | Cl | H | H | $CH_3$ | $CH_3$ | mp 57 ~ 58 ℃ | 4 |
| 226 | 〃 | 〃 | 〃 | 〃 | $C_2H_5$ | H | mp 103 ~ 104 ℃ | 4 |
| 227 | 〃 | 〃 | 〃 | 〃 | $n-C_3H_7$ | 〃 | mp 99 ~ 100 ℃ | 4 |
| 228 | 〃 | 〃 | 〃 | 〃 | 〃 | $n-C_3H_7$ | mp 106 ~ 108 ℃ | 4 |
| 229 | 〃 | 〃 | 〃 | 〃 | $i-C_3H_7$ | H | mp 119 ~ 121 ℃ | 4 |
| 230 | 〃 | 〃 | 〃 | 〃 | $CH_2CH=CH_2$ | 〃 | mp 73 ~ 76 ℃ | 4 |
| 231 | 〃 | 〃 | 〃 | 〃 | $CH_2CH=CH_2$ | $-CH_2CH=CH_2$ | mp 83 ~ 84 ℃ | 4 |
| 232 | 〃 | 〃 | 〃 | 〃 | $-CH_2C\equiv CH$ | H | Amorphous solid | 4 |
| 233 | 〃 | 〃 | 〃 | 〃 | $-CH_2Cl$ | 〃 | mp 132 ~ 134 ℃ | 4 |
| 234 | 〃 | 〃 | 〃 | 〃 | $-CH_2CH_2OC_2H_5$ | 〃 | $n_D^{25}$ 1.5743 | 4 |

| Comp. No. | A | X | Y | Z | U | V | Physical property | Exp. No. |
|---|---|---|---|---|---|---|---|---|
| 235 | H | Cl | F | H | 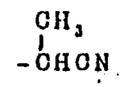 | H | Amorphous solid | 6 |
| 236 | " | " | H | " | 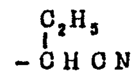 | " | Amorphous solid | 6 |
| 237 | " | " | " | " | 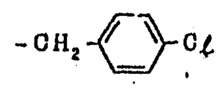 | " | mp 137.5 ~ 138 ℃ | 6 |
| 238 | " | " | " | " | 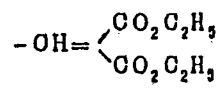 | " | mp 131.5 ~ 132 ℃ | 14 |
| 239 | " | " | " | " | 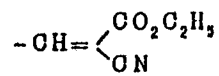 | " | mp 172 ~ 175 ℃ | 14 |
| 240 | " | " | " | " | $-CO_2CH_3$ | " | mp 135 ~ 136 ℃ | 13 |
| 241 | " | " | " | " | " | $n-C_3H_7$ | mp 108 ~ 109 ℃ | 13 |
| 242 | " | " | " | " | $-CO_2C_2H_5$ | H | mp 130 ~ 130.5 ℃ | 13 |
| 243 | " | " | " | " | " | $n-C_3H_7$ | $n_D^{25}$ 1.5539 | 13 |
| 244 | " | " | " | " | $-COSC_2H_5$ | " | $n_D^{25}$ 1.5798 | 13 |

| Comp. No. | A | X | Y | Z | U | V | Physical property | Exp. No. |
|---|---|---|---|---|---|---|---|---|
| 245 | H | Cl | H | H | $-CO_2C_3H_7-n$ | $n-C_3H_7$ | $n_D^{21}$ 1.5508 | 13 |
| 246 | ″ | ″ | ″ | ″ | $-COSC_3H_7-n$ | ″ | $n_D^{25}$ 1.5703 | 13 |
| 247 | ″ | ″ | ″ | ″ | $-CO_2C_5H_{11}-n$ | ″ | $n_D^{25}$ 1.5403 | 13 |
| 248 | ″ | ″ | ″ | ″ | $-CO_2C_8H_{17}-n$ | ″ | $n_D^{25}$ 1.5255 | 13 |
| 249 | ″ | ″ | ″ | ″ | $-CO_2-$ 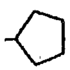 | ″ | $n_D^{25}$ 1.5060 | 13 |
| 250 | ″ | ″ | ″ | ″ | $-CO_2-$ 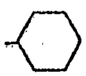 | ″ | $n_D^{25}$ 1.5173 | 13 |
| 251 | ″ | ″ | ″ | ″ | $-CO_2CH_2-$ 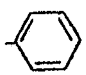 | H | mp 139 ~ 139.5 ℃ | 13 |
| 252 | ″ | ″ | ″ | ″ | $-CO_2-$ 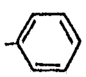 | $n-C_3H_7$ | mp 121.5 ~ 123.5 ℃ | 13 |
| 253 | ″ | ″ | ″ | ″ | $-COS-$ 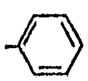 | ″ | $n_D^{20}$ 1.5921 | 13 |
| 254 | ″ | ″ | ″ | ″ | $-CHO$ | H | mp 167 ~ 167.5 ℃ | 13 |

| Comp. № | A | X | Y | Z | U | V | Physical property | Exp. № |
|---|---|---|---|---|---|---|---|---|
| 255 | H | Cl | H | H | $-COCH_3$ | H | mp 156 ~ 157 ℃ | 14 |
| 256 | 〃 | 〃 | 〃 | 〃 | $-COC_2H_5$ | 〃 | mp 130 ~ 134 ℃ | 14 |
| 257 | 〃 | 〃 | 〃 | 〃 | $-COCH=CHCH_3$ | 〃 | mp 170.5 ~ 172 ℃ | 13 |
| 258 | 〃 | 〃 | 〃 | 〃 | $-COCH_2Cl$ | 〃 | mp 118 ~ 118.5 ℃ | 13 |
| 259 | 〃 | 〃 | 〃 | 〃 | 〃 | $n-C_3H_7$ | $n_D^{25}$ 1.5683 | 13 |
| 260 | 〃 | 〃 | 〃 | 〃 | $-COCF_3$ | H | mp 125 ~ 126 ℃ | 14 |
| 261 | 〃 | 〃 | 〃 | 〃 | -CO- 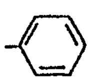 | H | mp 189.5 ~ 190.5 ℃ | 13 |
| 262 | 〃 | 〃 | 〃 | 〃 | 〃 | $n-C_3H_7$ | mp 52 ~ 54 ℃ | 13 |
| 263 | 〃 | 〃 | 〃 | 〃 | -CO- 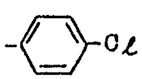 -Cl | H | mp 191 ~ 192 ℃ | 13 |
| 264 | 〃 | 〃 | 〃 | 〃 | $-CONHCH_3$ | 〃 | mp 221 ~ 222 ℃ | 15 |

| Comp. No. | A | X | Y | Z | U | V | Physical property | Exp. No. |
|---|---|---|---|---|---|---|---|---|
| 265 | H | Cl | H | H | $-CON{<}^{CH_3}_{OCH_3}$ | H | mp 162 ~ 165 ℃ | 16 |
| 266 | 〃 | 〃 | 〃 | 〃 | -CONH- 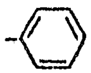 | 〃 | mp 238 ~ 239.5 ℃ | 15 |
| 267 | 〃 | 〃 | 〃 | 〃 | -CONH- 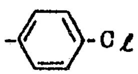 -Cl | 〃 | mp 233 ~ 235 ℃ | 15 |
| 268 | 〃 | 〃 | 〃 | 〃 | $-SO_2CH_3$ | 〃 | mp 174 ~ 177 ℃ | 13 |
| 269 | 〃 | H | 〃 | 〃 | H | 〃 | mp 175.5 ~ 176.5 ℃ | 1 |
| 270 | 〃 | Cl | 〃 | 〃 | 〃 | 〃 | mp 214 ~ 217 ℃ | 2 |
| 271 | $3-CH_3$ | 〃 | 〃 | 〃 | 〃 | 〃 | mp 139 ~ 141 ℃ | 2 |
| 272 | $4-CH_3$ | 〃 | 〃 | 〃 | 〃 | 〃 | mp 184 ~ 185 ℃ | 2 |
| 273 | H | Br | 〃 | 〃 | 〃 | 〃 | mp 226 ~ 228 ℃ | 2 |
| 274 | 〃 | H | F | 〃 | 〃 | 〃 | mp 134.5 ~ 135 ℃ | 1 |

| Comp. No. | A | X | Y | Z | U | V | Physical property | Exp. No. |
|---|---|---|---|---|---|---|---|---|
| 275 | H | F | F | H | H | H | mp 133.5 ~ 135.5 ℃ | 1 |
| 276 | 〃 | Cℓ | Cℓ | 〃 | 〃 | 〃 | mp 185 ~ 187 ℃ | 1 |
| 277 | 〃 | 〃 | F | 〃 | 〃 | 〃 | mp 169 ~ 171 ℃ | 1 |
| 278 | 〃 | Br | 〃 | 〃 | 〃 | 〃 | mp 172 ~ 174 ℃ | 1 |
| 279 | 〃 | F | Cℓ | 〃 | 〃 | 〃 | mp 162.5 ~ 164.5 ℃ | 1 |
| 280 | 〃 | 〃 | Br | 〃 | 〃 | 〃 | mp 176.5 ~ 179 ℃ | 1 |

| Comp. No. | A | X | Y | Z | $U = -(\overset{R^1}{\underset{R^2}{C}})_n-COB$ | | | | V | Physical property | Exp. No. |
|---|---|---|---|---|---|---|---|---|---|---|---|
| | | | | | n | $R^1$ | $R^2$ | B | | | |
| 281 | H | Cl | F | H | 1 | $CH_3$ | H | OH | H | mp 194.5-197°C | 10 |
| 282 | " | " | " | " | " | " | " | $OC_2H_5$ | " | mp 93.5-94°C | 6 |
| 283 | " | " | " | " | " | " | " | $OC_3H_7$-n | " | mp 58-60°C | 6 |
| 284 | " | " | " | " | " | " | " | $OC_4H_9$-n | " | $n_D^{25}$ 1.5438 | 6 |
| 285 | " | " | " | " | " | " | " | $OC_5H_{11}$-n | " | $n_D^{25}$ 1.5425 | 6 |
| 286 | " | " | " | " | " | $C_2H_5$ | " | OH | " | mp 134.5-136.5°C | 10 |
| 287 | " | " | " | " | " | " | " | $OCH_3$ | " | mp 79.5-81°C | 6 |
| 288 | " | " | " | " | " | " | " | $OC_2H_5$ | " | mp 90-91°C | 6 |
| 289 | " | " | " | " | " | " | " | $OC_3H_7$-n | " | $n_D^{26}$ 1.5454 | 6 |
| 290 | " | " | " | " | " | " | " | $OC_3H_7$-i | " | $n_D^{26}$ 1.5440 | 6 |

| Comp. No. | A | X | Y | Z | U = 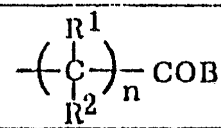 | | | | V | Physical property | Exp. No. |
|---|---|---|---|---|---|---|---|---|---|---|---|
| | | | | | n | $R^1$ | $R^2$ | B | | | |
| 291 | H | Cl | F | H | 1 | $C_2H_5$ | H | $OC_4H_9$-n | H | $n_D^{25}$ 1.5414 | 6 |
| 292 | " | " | " | " | " | " | " | $OC_4H_9$-i | " | $n_D^{25}$ 1.5396 | 6 |
| 293 | " | " | H | " | " | " | " | " | " | mp 107-108°C | 6 |
| 294 | " | " | " | " | " | " | " | 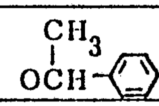 | " | Amorphous solid | 6 |
| 295 | " | " | F | " | " | n-$C_3H_7$ | " | $OCH_3$ | " | mp 116-116.5°C | 6 |
| 296 | " | " | " | " | " | " | " | $OC_2H_5$ | " | mp 104-106°C | 6 |
| 297 | " | " | " | " | " | " | " | $OC_3H_7$-n | " | mp 49-51°C | 6 |
| 298 | " | " | " | " | " | " | " | $OC_3H_7$-i | " | mp 102-103.5°C | 6 |
| 299 | " | " | " | " | " | " | " | $OC_4H_9$-n | " | $n_D^{25}$ 1.5389 | 6 |
| 300 | " | " | " | " | " | " | " | $OC_4H_9$-i | " | $n_D^{25}$ 1.5380 | 6 |

| Comp. No. | A | X | Y | Z | $U = \left(-\overset{R^1}{\underset{R^2}{C}}-\right)_n COB$ | | | | V | Physical property | Exp. No. |
|---|---|---|---|---|---|---|---|---|---|---|---|
| | | | | | n | $R^1$ | $R^2$ | B | | | |
| 301 | H | Cl | F | H | 1 | $n\text{-}C_3H_7$ | H | $OCH_2CH=CHCH_3$ | H | $n_D^{25}$ 1.5470 | 12 |
| 302 | " | " | " | " | " | " | " | $OCH_2CH(CH_3)C_2H_5$ | " | $n_D^{25}$ 1.5350 | 12 |
| 303 | " | " | H | " | " | H | " | $OCH_2CO_2C_2H_5$ | " | mp 134-135°C | 12 |
| 304 | " | " | " | " | " | " | " | $OCH(CH_3)CO_2C_2H_5$ | " | mp 119-121.5°C | 12 |
| 305 | " | " | F | " | " | " | " | " | " | Amorphous solid | 12 |
| 306 | " | " | " | " | " | " | " | $OCH(C_2H_5)CO_2C_2H_5$ | " | $n_D^{25}$ 1.5419 | 12 |
| 307 | " | " | H | " | " | $CH_3$ | " | $OCH(CH_3)CO_2C_3H_7\text{-}n$ | " | $n_D^{25}$ 1.5453 | 12 |
| 308 | " | " | " | " | " | " | " | $OCH(CH_3)CO_2C_4H_9\text{-}t$ | " | Amorphous solid | 12 |
| 309 | " | " | " | " | " | " | " | $OCH(C_2H_5)CO_2C_4H_9\text{-}i$ | " | $n_D^{25}$ 1.5374 | 12 |
| 310 | " | " | F | " | " | " | " | $OCH_2CO_2C_2H_5$ | " | mp 101.5-102.5°C | 12 |

| Comp. No. | A | X | Y | Z | $U = -(\overset{R^1}{\underset{R^2}{C}})_n-COB$ | | | | V | Physical property | Exp. No. |
|---|---|---|---|---|---|---|---|---|---|---|---|
| | | | | | n | $R^1$ | $R^2$ | B | | | |
| 311 | H | Cl | F | H | 1 | $CH_3$ | H | $CH_3$<br>$OCHCO_2C_3H_7$-i | H | Amorphous solid | 12 |
| 312 | " | " | " | " | " | " | " | $CH_3$<br>$OCHCO_2C_4H_9$-i | " | $n_D^{25}$ 1.5306 | 12 |
| 313 | " | " | " | " | " | " | " | $C_2H_5$<br>$OCHCO_2C_2H_5$ | " | $n_D^{25}$ 1.5360 | 12 |
| 314 | " | " | H | " | " | $C_2H_5$ | " | $CH_3$<br>$OCHCO_2C_2H_5$ | " | $n_D^{25}$ 1.5472 | 12 |
| 315 | " | " | " | " | " | " | " | $CH_3$<br>$OCHCO_2C_3H_7$-i | " | $n_D^{25}$ 1.5414 | 12 |
| 316 | " | " | F | " | " | " | " | $OCH_2CO_2C_2H_5$ | " | $n_D^{25}$ 1.5440 | 12 |
| 317 | " | " | " | " | " | " | " | $CH_3$<br>$OCHCO_2C_2H_5$ | " | $n_D^{25}$ 1.5362 | 12 |
| 318 | " | " | " | " | " | " | " | $C_2H_5$<br>$OCHCO_2C_2H_5$ | " | $n_D^{25}$ 1.5350 | 12 |
| 319 | " | " | H | " | " | H | " | $CH_3$<br>$OCHCONHC_3H_7$-i | " | mp 138-140°C | 12 |
| 320 | " | " | " | " | " | " | " | $CH_3$<br>$OCHCON(CH_3)_2$ | " | mp 176.5-177°C | 12 |

| Comp. No. | A | X | Y | Z | $U = -(\overset{R^1}{\underset{R^2}{C}})_n-COB$ | | | | V | Physical property | Exp. No. |
|---|---|---|---|---|---|---|---|---|---|---|---|
| | | | | | n | $R^1$ | $R^2$ | B | | | |
| 321 | H | Cl | F | H | 1 | H | H | $OCH_2CONHC_3H_7$-i | H | mp 163.5-164.5°C | 12 |
| 322 | " | " | " | " | " | " | " | $O\overset{CH_3}{\overset{\vert}{C}}HCON(CH_3)_2$ | " | mp 182-183°C | 12 |
| 323 | " | " | H | " | " | $CH_3$ | " | $OCH_2CONHC_3H_7$-n | " | mp 159.5-160.5°C | 12 |
| 324 | " | " | " | " | " | " | " | $O\overset{CH_3}{\overset{\vert}{C}}HCONHC_4H_9$-i | " | Amorphous solid | 12 |
| 325 | " | " | " | " | " | " | " | $O\overset{CH_3}{\overset{\vert}{C}}HCONHC_4H_9$-t | " | Amorphous solid | 12 |
| 326 | " | " | " | " | " | " | " | $O\overset{C_2H_5}{\overset{\vert}{C}}HCON(CH_3)_2$ | " | Amorphous solid | 12 |
| 327 | " | " | F | " | " | " | " | $O\overset{CH_3}{\overset{\vert}{C}}HCONHC_2H_5$ | " | mp 138-140°C | 12 |
| 328 | " | " | " | " | " | " | " | $O\overset{CH_3}{\overset{\vert}{C}}HCONHCH_2CH=CH_2$ | " | mp 117.5-120°C | 12 |
| 329 | " | " | " | " | " | " | " | $O\overset{C_2H_5}{\overset{\vert}{C}}HCONHC_3H_7$-i | " | mp 121-124°C | 12 |
| 330 | " | " | H | : | : | $C_2H_5$ | " | $OCH_2CONHCH_2CH=CH_2$ | " | mp 119.5-120.5°C | 12 |

| Comp. No. | A | X | Y | Z | $U = -\left(\underset{R^2}{\overset{R^1}{C}}\right)_n-COB$ | | | | V | Physical property | Exp. No. |
|---|---|---|---|---|---|---|---|---|---|---|---|
| | | | | | n | $R^1$ | $R^2$ | B | | | |
| 331 | H | Cl | H | H | 1 | $C_2H_5$ | H | $CH_3$<br>$OCHCONHC_2H_5$ | H | mp 104-107.5°C | 12 |
| 332 | " | " | " | " | " | " | " | $C_2H_5$<br>$OCHCON(CH_3)_2$ | " | Amorphous solid | 12 |
| 333 | " | " | F | " | " | " | " | $CH_3$<br>$OCHCONHC_4H_9$-i | " | Amorphous solid | 12 |
| 334 | " | " | " | " | " | " | " | $CH_3$<br>$OCHCON(CH_3)_2$ | " | Amorphous solid | 12 |
| 335 | " | " | " | " | " | " | " | $C_2H_5$<br>$OCHCONHC_2H_5$ | " | mp 110-112°C | 12 |
| 336 | " | " | " | " | " | $CH_3$ | " | $NHCH_2CO_2C_2H_5$ | " | Amorphous solid | 11 |
| 337 | " | " | " | " | " | " | " | $CH_3$<br>$NHCHCO_2C_2H_5$ | " | mp 145-149°C | 11 |
| 338 | " | " | H | " | " | $C_2H_5$ | " | $NHCH_2CO_2C_2H_5$ | " | mp 105.5-106.5°C | 11 |
| 339 | " | " | " | " | " | " | " | $CH_3$<br>$NHCHCO_2C_2H_5$ | " | mp 108-112°C | 11 |
| 340 | " | " | F | " | " | " | " | $NHCH_2CO_2C_2H_5$ | " | Amorphous solid | 11 |

| Comp. No. | A | X | Y | Z | $U = -\!\left(\overset{R^1}{\underset{R^2}{C}}\right)_{\!n}\!-COB$ | | | | V | Physical property | Exp. No. |
|---|---|---|---|---|---|---|---|---|---|---|---|
| | | | | | n | $R^1$ | $R^2$ | B | | | |
| 341 | H | Cl | F | H | 1 | $C_2H_5$ | H | $NH\overset{CH_3}{C}HCO_2C_2H_5$ | H | mp 125-130°C | 11 |
| 342 | " | " | H | " | " | " | " | $NH\overset{CH_3}{C}HCONHC_2H_5$ | " | mp 210-214°C | 11 |
| 343 | " | " | " | " | " | " | " | $NH\overset{CH_3}{C}HCONHC_3H_7$-n | " | mp 155-160°C | 11 |
| 344 | " | " | " | " | " | " | " | $NH\overset{CH_3}{C}HCONHC_4H_9$-s | " | mp 179-183°C | 11 |
| 345 | " | " | " | " | " | " | " | $NH\overset{CH_3}{C}HCONHC_4H_9$-t | " | mp 141-146°C | 11 |
| 346 | " | " | " | " | " | " | " | $NH\overset{CH_3}{C}HCONHCH_2CH{=}CH_2$ | " | Amorphous solid | 11 |
| 347 | " | " | " | " | " | " | " | $NH\overset{CH_3}{C}HCON(CH_3)_2$ | " | mp 157-162°C | 11 |
| 348 | " | " | F | " | " | " | " | $NH\overset{CH_3}{C}HCONHC_4H_9$-i | " | Amorphous solid | 11 |
| 349 | " | " | " | " | " | " | " | $NH\overset{CH_3}{C}HCON(CH_3)_2$ | " | mp 109-115°C | 11 |
| 350 | " | " | H | " | " | $CH_3$ | " | $N\!<^{SO_2C_2H_5}_{CH_3}$ | " | mp 120-121.5°C | 6 |

| Comp. No. | A | X | Y | Z | $U = -\left(\overset{R^1}{\underset{R^2}{C}}\right)_n-COB$ | | | | V | Physical property | Exp. No. |
|---|---|---|---|---|---|---|---|---|---|---|---|
| | | | | | n | $R^1$ | $R^2$ | B | | | |
| 351 | H | Cl | F | H | 1 | $CH_3$ | H | $N{<}^{SO_2CH_3}_{C_2H_5}$ | H | Amorphous solid | 6 |
| 352 | " | " | " | " | " | " | " | $N{<}^{SO_2C_2H_5}_{C_2H_5}$ | " | mp 114-117°C | 6 |
| 353 | " | " | H | " | " | $C_2H_5$ | " | $N{<}^{SO_2CH_3}_{CH_3}$ | " | Amorphous solid | 6 |
| 354 | " | " | F | " | " | " | " | " | " | Amorphous solid | 6 |
| 355 | " | " | H | " | " | " | " | $N{<}^{SO_2C_2H_5}_{CH_3}$ | " | mp 134.5-135.5°C | 6 |
| 356 | " | " | F | " | " | " | " | " | " | mp 127-129°C | 6 |
| 357 | " | " | H | " | " | " | " | $N{<}^{SO_2CH_3}_{C_2H_5}$ | " | Amorphous solid | 6 |
| 358 | " | " | F | " | " | " | " | " | " | Amorphous solid | 6 |
| 359 | " | " | H | " | 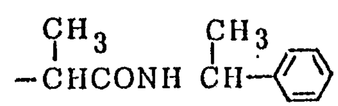 (S) (R) * | | | | " | Amorphous solid | 5 |

| Comp. No. | A | X | Y | Z | U = 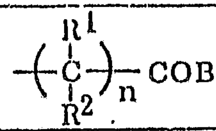 | | | | V | Physical property | Exp. No. |
|---|---|---|---|---|---|---|---|---|---|---|---|
| | | | | | n | $R^1$ | $R^2$ | B | | | |
| 360 | H | Cl | H | H | 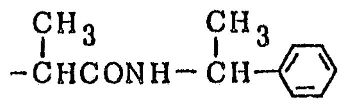 (R) | (R) | | * | H | mp 183-184.5°C | 5 |
| 361 | " | " | " | " | 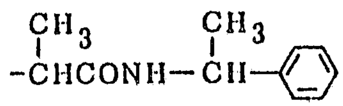 (R) | (S) | | * | " | Amorphous solid | 5 |
| 362 | " | " | " | " | 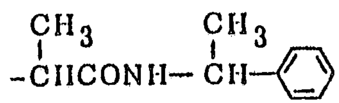 (S) | (S) | | * | " | mp 185-186.5°C | 5 |
| 363 | " | " | " | " | 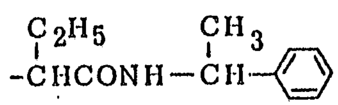 (S) | (R) | | * | " | mp 115-116°C | 5 |
| 364 | " | " | " | " | 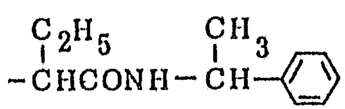 (R) | (R) | | * | " | mp 96-98°C | 5 |

| Comp. No. | A | X | Y | Z | $U = -\left(\overset{R^1}{\underset{R^2}{C}}\right)_n-COB$ | | | | V | Physical property | Exp. No. |
|---|---|---|---|---|---|---|---|---|---|---|---|
| | | | | | n | $R^1$ | $R^2$ | B | | | |
| 365 | H | Cl | H | H | 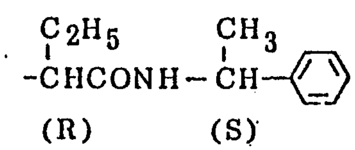 | | | * | H | mp 121-122°C | 5 |
| 366 | " | " | " | " | 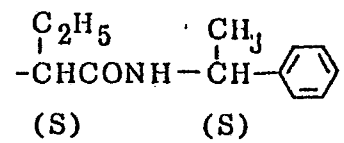 | | | * | " | mp 95-97°C | 5 |

* (R): R-configuration

(S): S-configuration

Although the compounds (I) can be used by themselves as herbicides without any adjuvant, it is common to use them in a form of herbicidal compositions such as an emulsifiable concentrate, a wettable powder, a dust, a granule and a tablet by admixing them with suitable inert liquids or solid carriers and other adjuvants such as suitable surfactants.

Suitable liquid carriers include toluene, xylene, methyl naphthalene, cyclohexane, butanol, glycol, dimethylsulfoxide, dimethylformamide, acetone,methyl isobutyl ketone, animal or vegetable oils, fatty acid, fatty acid esters and water.

Suitable solid carriers include clay, kaolin clay, talc, bentonite, diatomaceous earth, silica, calcium carbonate, and plant powders such as soybean powder, and wheat powder.

It is also possible to incorporate other agricultural chemicals such as agricultural fungicides, insecticides, nematocides, and other herbicides, plant growth regulators, soil improvers and fertilizers.

Further, a suitable adjuvant such as a spreader, an emulsifier, a wet spreader or a sticker may be incorporated to ensure the herbicidal effect.

Suitable amounts of the active ingredient and the adjuvants in the herbicidal compositions of the present invention are as follows.

(% by weight)

| | Active ingredient | Surfactant | Carrier | Other additive |
|---|---|---|---|---|
| Wettable powder | 5 to 80 | 2 to 20 | 10 to 93 | 0 to 5 |
| Flowable | 5 to 60 | 5 to 30 | 10 to 90 | 0 to 20 |
| Granule | 1 to 20 | 2 to 10 | 70 to 97 | 0 to 5 |
| Emulsifiable concentrate | 5 to 80 | 5 to 30 | 10 to 90 | 0 to 5 |

A dose of the compound (I) as the herbicide varies depending upon a kind of the compound, a kind of weed, a season and method of the application and a kind of soil. However it is usually in a range of from 0.1 to 80 g./are, preferably from 0.5 - 20 g./are.

The compounds (I) as the herbicide of the present invention can be applied in flooded soil treatments at preemergence or in treatments at growth period, and they exhibit excellent herbicidal activity against annual weeds and perennial weeds and have low phytotoxicity to transplanted rice seedling which are remarkably preferable as herbicide in paddy field. In the soil treatment at preemergence and the foliage and soil treatment in upland, the compounds of the present invention exhibit excellent herbicidal activity against annual weeds and perennial weeds and good residual effect. The phytotoxicity to crop plants is remarkably low, even when they are applied at a high concentration.
Further, the compounds of the present invention have a special characteristic that they exhibit herbicidal effects not only against ordinary weeds but also against morningglories, jimsonweeds, velvetleaf, and the like which are hardly controlled by the conventional herbicides.

The compounds as a herbicide of the present invention are used for controlling the following weeds.

Dicotyledonous weeds:

| Scientific name | (American name) |
| --- | --- |
| *Ipomoea* spp. | (morningglories) |
| *Galium aparine* | (bedstraw) |
| *Stellaria media* | (common chickweed) |
| *Galinsoga ciliata* | (hairy galinsoga) |
| *Datura stramonium L.* | (jimsonweed) |
| *Chenopodium album* | (lambsquarters) |
| *Abutilon theophrasti* | (velvetleaf) |
| *Brassica kaber* var. *Pinnatifida* | (wild mustard) |
| *Sinapis alba* | (charlock) |
| *Rumex japonicus* | |
| *Polygonum blumei* | (smartweed) |
| *Xanthium* spp. | (cocklebur) |
| *Matricaria moritima* | (chamomile) |
| *Amaranthus lividus* | (livid amaranth) |
| *Ambrosia artemisifolia* | (common ragweed) |
| *Rotala indica* | (toothcup) |
| *Lindernia procumbens* | |
| *Eclipta prostrata* | |
| *Bidens tripartita* | |
| *Dopatrium junceum* | |
| *Elatine triandra* | |
| *Polygonum thunbergii* | |

Monocotyledonous weeds:

| Scientific name | (American name) |
|---|---|
| Echinochloa crus-galli | (barnyard grass) |
| Digitaria sanguinalis | (large crabgrass) |
| Eleusine indica | (goosegrass) |
| Setaria viridis | (green foxtail) |
| Poa annua | (annual bluegrass) |
| Alopecurus aequalis | (water foxtail) |
| Cynodon dectylon | (bermudagrass) |
| Agropyron repens | (quackgrass) |
| Cyperus microiria | |
| Cyperus difformis | |
| Eleocharis kuroguwai | (water chestnut) |
| Eleocharis acicularis | (slender spikerush) |
| Scirpus juncoides | (hardstem bulrush) |
| Cyperus serotinus | |
| Scirpus maritimus | |
| Monochoria vaginalis | |
| Sagitaria pygmaea | |
| Alisma canaliculatum | |
| Sagitaria trifolia | |

The compounds as a harbicide of the present invention can be used as selective herbicides in the cultivation of the following crops.

Dicotyledonous crops:

| | |
|---|---|
| Glycine max | (soybean) |
| Gossypium indicum | (cotton) |
| Beta vulgaris | (sugar beet) |
| Helianthus annuus | (sunflower) |
| Pisum sativum | (pea) |

| | |
|---|---|
| Solanum tuberosum | (potato) |
| Cucumis sativus | (cucumber) |

Monocotyledonous crops:

| | |
|---|---|
| Oryza sativa | (rice) |
| Triticum aestivum | (wheat) |
| Hordoum vulgare | (barley) |
| Avena fatua | (oat) |
| Secale cereale | (rye) |
| Zea mays | (corn) |
| Saccharum officinarum | (sugar cane) |

The compounds as a herbicide of the present invention is applicable not only to the aforementioned plants but also to other plants in the same manner.

The herbicides and herbicidal compositions of the present invention will now be illustrated by certain examples of preparations and herbicidal experimental tests which are provided for purposes of illustration only and are not intended to be limiting the present invention.

In the preparations and the experiments, the term "part" means "part by weight" unless otherwise specified and Compound numbers correspond to Compounds shown in Table 1. As comparative references, the following compounds were also used.

| | |
|---|---|
| Reference A: | 3-(3,4-dichlorophenyl)-1-methoxy-1-methylurea |
| Reference B: | 2,4-dichlorophenyl-4'-nitrophenyl ether |
| Reference C: | N-methoxymethyl-2,6-diethyl-α-chloroacetanilide |
| Reference D: | 3,4-dichloropropionanilide |
| Reference E: | S-(4-chlorobenzyl)-N,N-diethylthiol carbamate |

## PREPARATION EXAMPLE 1:

Wettable powder:

| | |
|---|---|
| Compound shown in Table 1 | 50 parts |
| Carplex #80(Trademark-Shionogi Seiyaku K.K.) | 15 parts |
| N,N-kaolin clay (Trademark-Kaolin K.K.) | 30 parts |
| Sorpol 8070 (Trademark-Toho Kagaku K.K.) (higher alkyl sulfate surfactant) | 5 parts |

The components were uniformly mixed and ground to obtain a wettable powder containing 50% of the active ingredient.

In a similar manner, various wettable powders containing from 5 to 80% of the active ingredient are obtainable by using from 5 to 80 parts of the compounds listed in Table 1, from 2 to 10 parts of Sorpol 8070 as a surfactant and from 10 to 93 parts of a mixture of Carplex #80 and N,N-kaoline clay to bring the total to 100 parts.

## PREPARATION EXAMPLE 2:

Granule:

| | |
|---|---|
| Compound shown in Table 1 | 5 parts |
| Clay (Nippon Talc K.K.) | 38 parts |
| Bentonite (Hojunyoko K.K.) | 55 parts |
| Aerol CT-1 (Trademark-Toho Kagaku K.K.) (succinate type surfactant) | 2 parts |

The components were mixed with water and kneaded and granulated by a granulating machine and dried at 60°C for 2 hours to obtain a granule containing 5% of the active ingredient.

In a similar manner, various granules containing from 1 to 20% of the active ingredient are obtainable by using from 1 to 20 parts of the compounds listed in Table 1, from 2 to 10 parts of Aerol CT-1 as a surfactant and from 70 to 97 parts of a mixture of clay and bentonite to bring the total to 100 parts.

## PREPARATION EXAMPLE 3:

Emulsifiable concentrate:

| | |
|---|---|
| Compound shown in Table 1 | 30 parts |
| Xylene | 30 parts |
| Dimethylformamide | 25 parts |

The compound was dissolved into the solvent mixture of xylene and dimethylformamide and then, 15 parts of polyoxyethylene type surfactant (Sorpol 3005X: Trademark-Toho Kagaku K.K.) was admixed to obtain an emulsifiable concentrate containing 30% of the active ingredient.

In a similar manner, various emulsifiable concentrates containing from 5 to 80% of the active ingredient are obtainable by using

from 5 to 80 parts of the compounds listed in Table 1, from 5 to 30 parts of Sorpol 3005X as a surfactant and from 10 to 90 parts of a solvent mixture of xylene and dimethylformamide to bring the total to 100 parts.

Test 1:

Up-land soil treatment test:

Each plastic pot of 1/2,500 are was filled with black volcano ash soil and manured, and seeds of each of wheat, corn, and barley were sown and were covered with the soil in a depth of 2.5 cm and seeds of each weed shown in Table 2 were mixed in the covered soil layer. Each wettable powder containing each of Compounds of the Table 2 was diluted with water and the solution was sprayed uniformly on the surface of the soil at a dose (3.125, 6.25 or 25 g./are) of the compound by a small size power pressurized spray.

Thirty days after the treatment, herbicidal effects were observed and phytotoxicities to the crop plants were also observed.
The results are shown in Table 2.
Herbicidal effects are rated by the following equation and ratings.

$$\left(1 - \frac{\text{Survival terrestrial weed weight in treated pot}}{\text{Survival terrestrial weed in non-treated pot}}\right) \times 100 = Y(\%)$$

| Herbicidal effect rating | Y(%) |
|---|---|
| 0 | 0 - 5 |
| 1 | 5 -30 |
| 2 | 30- 50 |
| 3 | 50- 70 |
| 4 | 70- 90 |
| 5 | 90 -100 |

Phytotoxicities are rated by the following equation and ratings.

$$\left(1 - \frac{\text{Survival terrestrial crop plant weight in treated pot}}{\text{Survival terrestrial cropplant weight in non-treated pot}}\right) \times 100 = Y(\%)$$

| Phytotoxicity rating | Y(%) |
|---|---|
| 0 | 0 - 5 |
| 1 | 5 -10 |
| 2 | 10 -20 |
| 3 | 20 -40 |
| 4 | 40 -60 |
| 5 | 60-100 |

In Table 2, the following symbols are used to identify the weeds and the crop plants:

A: Large crabgrass (Digitaria sanguinalis)

B: Green foxtail (Setaria viridis)

C: Lambsquarters (Chenopodium album)

D: Smartweed (Polygonum blumei)

E: Pigweed (Amaranthus retroflexus)

F: Morningglories (Ipomoea spp.)

G: Jimsonweed (Datura stramonium L.)

H: Velvetleaf (Abutilon theophrasti)

I: Common chickweed (Stellaria media)

J: Chamomile (Matricaria maritima)

K: Corn (Zea mays)

L: Wheat (Triticum aestivum)

M: Barley (Hordeum vulgare)

Further, in Table 2 and subsequent Table 4, "Dose" means "a dose of the active ingredient"(g/are).

Table 2:

| Compound No. | Dose g/a | Herbicidal effect rating A | B | C | D | E | F | G | H | I | J | Phytotoxicity rating K | L | M |
|---|---|---|---|---|---|---|---|---|---|---|---|---|---|---|
| 3 | 3.125 | 0 | | 3 | 2 | 4 | | | | | | 0 | 0 | 0 |
| | 6.25 | 0 | | 4 | 4 | 4 | | | | | | 0 | 0 | 0 |
| | 12.5 | 0 | | 5 | 4 | 5 | | | | | | 0 | 0 | 0 |
| | 25 | 2 | | 5 | 5 | 5 | | | | | | 0 | 0 | 0 |
| 5 | 3.125 | 1 | | 4 | 3 | 4 | | | | | | 0 | 0 | 0 |
| | 6.25 | 2 | | 4 | 5 | 5 | | | | | | 0 | 0 | 0 |
| | 12.5 | 3 | | 5 | 5 | 5 | | | | | | 0 | 0 | 0 |
| | 25 | 5 | | 5 | 5 | 5 | | | | | | 0 | 0 | 0 |
| 7 | 3.125 | 1 | | 1 | 1 | 3 | | | | | | 0 | 0 | 0 |
| | 6.25 | 1 | | 3 | 1 | 4 | | | | | | 0 | 0 | 0 |
| | 12.5 | 3 | | 5 | 3 | 4 | | | | | | 0 | 0 | 0 |
| | 25 | 5 | | 5 | 5 | 5 | | | | | | 0 | 0 | 0 |
| 13 | 3.125 | 2 | | 1 | 3 | 2 | | | | | | 0 | 0 | 0 |
| | 6.25 | 2 | | 2 | 4 | 3 | | | | | | 0 | 0 | 0 |
| | 12.5 | 4 | | 3 | 5 | 4 | | | | | | 0 | 0 | 0 |
| | 25 | 4 | | 5 | 5 | 5 | | | | | | 0 | 0 | 0 |
| 16 | 3.125 | 2 | | 3 | 3 | 1 | | | | | | 0 | 0 | 0 |
| | 6.25 | 3 | | 3 | 4 | 3 | | | | | | 0 | 0 | 0 |
| | 12.5 | 4 | | 4 | 5 | 4 | | | | | | 0 | 0 | 0 |
| | 25 | 5 | | 5 | 5 | 5 | | | | | | 0 | 0 | 0 |
| 19 | 3.125 | 0 | | 1 | 3 | 2 | | | | | | 0 | 0 | 0 |
| | 6.25 | 1 | | 2 | 3 | 2 | | | | | | 0 | 0 | 0 |
| | 12.5 | 3 | | 3 | 4 | 4 | | | | | | 0 | 0 | 0 |
| | 25 | 5 | | 4 | 5 | 5 | | | | | | 0 | 0 | 0 |
| 22 | 3.125 | 0 | | 1 | 2 | 0 | | | | | | 0 | 0 | 0 |
| | 6.25 | 1 | | 1 | 3 | 2 | | | | | | 0 | 0 | 0 |
| | 12.5 | 2 | | 2 | 4 | 3 | | | | | | 0 | 0 | 0 |
| | 25 | 4 | | 5 | 5 | 4 | | | | | | 0 | 0 | 0 |
| 25 | 3.125 | 1 | | 2 | 3 | 1 | | | | | | 0 | 0 | 0 |
| | 6.25 | 1 | | 3 | 4 | 3 | | | | | | 0 | 0 | 0 |
| | 12.5 | 3 | | 4 | 4 | 4 | | | | | | 0 | 0 | 0 |
| | 25 | 4 | | 5 | 5 | 5 | | | | | | 0 | 0 | 0 |

Table 2:

| Compound No. | Dose g/a | Herbicidal effect rating | | | | | | | | | | Phytotoxicity rating | | |
|---|---|---|---|---|---|---|---|---|---|---|---|---|---|---|
| | | A | B | C | D | E | F | G | H | I | J | K | L | M |
| 26 | 3.125 | 2 | 2 | 5 | 5 | 2 | | | | | | 0 | 0 | 0 |
| | 6.25 | 3 | 4 | 5 | 5 | 4 | | | | | | 0 | 0 | 0 |
| | 12.5 | 4 | 5 | 5 | 5 | 5 | | | | | | 0 | 0 | 0 |
| | 25 | 5 | 5 | 5 | 5 | 5 | | | | | | 1 | 2 | 0 |
| 27 | 3.125 | 2 | 1 | 5 | 4 | 2 | | | | | | 0 | 0 | 0 |
| | 6.25 | 3 | 2 | 5 | 5 | 3 | | | | | | 0 | 0 | 0 |
| | 12.5 | 4 | 4 | 5 | 5 | 4 | | | | | | 1 | 1 | 0 |
| | 25 | 5 | 5 | 5 | 5 | 5 | | | | | | 1 | 1 | 2 |
| 28 | 3.125 | 1 | | 3 | 3 | 2 | | | | | | 0 | 0 | 0 |
| | 6.25 | 2 | | 4 | 5 | 3 | | | | | | 0 | 0 | 0 |
| | 12.5 | 4 | | 4 | 5 | 5 | | | | | | 0 | 0 | 0 |
| | 25 | 4 | | 5 | 5 | 5 | | | | | | 0 | 0 | 0 |
| 29 | 3.125 | 2 | 2 | 5 | 4 | 1 | | | | | | 0 | 0 | 0 |
| | 6.25 | 2 | 2 | 5 | 5 | 3 | | | | | | 0 | 0 | 0 |
| | 12.5 | 3 | 3 | 5 | 5 | 5 | | | | | | 0 | 0 | 0 |
| | 25 | 5 | 5 | 5 | 5 | 5 | | | | | | 0 | 0 | 0 |
| 30 | 3.125 | 1 | | 3 | 0 | 2 | | | | | | 0 | 0 | 0 |
| | 6.25 | 2 | | 3 | 1 | 2 | | | | | | 0 | 0 | 0 |
| | 12.5 | 3 | | 4 | 1 | 5 | | | | | | 0 | 0 | 0 |
| | 25 | 3 | | 5 | 1 | 5 | | | | | | 0 | 0 | 0 |
| 31 | 3.125 | 4 | 1 | 5 | 4 | 5 | | | | | | 0 | 0 | 0 |
| | 6.25 | 5 | 3 | 5 | 5 | 5 | | | | | | 0 | 0 | 0 |
| | 12.5 | 5 | 3 | 5 | 5 | 5 | | | | | | 0 | 0 | 0 |
| | 25 | 5 | 4 | 5 | 5 | 5 | | | | | | 0 | 0 | 0 |
| 32 | 3.125 | 4 | 3 | 4 | 4 | 5 | 2 | 3 | 4 | 1 | 5 | 0 | 0 | 0 |
| | 6.25 | 5 | 3 | 5 | 5 | 5 | 2 | 3 | 4 | 1 | 5 | 0 | 0 | 0 |
| | 12.5 | 5 | 5 | 5 | 5 | 5 | 4 | 4 | 5 | 1 | 5 | 0 | 0 | 0 |
| | 25 | 5 | 5 | 5 | 5 | 5 | 5 | 4 | 5 | 4 | 5 | 0 | 0 | 0 |
| 33 | 3.125 | 5 | 5 | 5 | 5 | 5 | 2 | 5 | 5 | 2 | 5 | 0 | 0 | 0 |
| | 6.25 | 5 | 5 | 5 | 5 | 5 | 2 | 5 | 5 | 2 | 5 | 0 | 0 | 0 |
| | 12.5 | 5 | 5 | 5 | 5 | 5 | 3 | 5 | 5 | 4 | 5 | 0 | 0 | 0 |
| | 25 | 5 | 5 | 5 | 5 | 5 | 4 | 5 | 5 | 4 | 5 | 0 | 0 | 0 |

Table 2:

| Compound No. | Dose g/a | Herbicidal effect rating | | | | | | | | | | Phytotoxicity rating | | |
|---|---|---|---|---|---|---|---|---|---|---|---|---|---|---|
| | | A | B | C | D | E | F | G | H | I | J | K | L | M |
| 34 | 3.125 | 4 | 4 | 5 | 5 | 5 | 2 | 3 | 2 | 1 | 5 | 0 | 0 | 0 |
| | 6.25 | 4 | 5 | 5 | 5 | 5 | 3 | 4 | 5 | 2 | 5 | 0 | 0 | 0 |
| | 12.5 | 5 | 5 | 5 | 5 | 5 | 3 | 5 | 5 | 4 | 5 | 0 | 0 | 0 |
| | 25 | 5 | 5 | 5 | 5 | 5 | 5 | 5 | 5 | 5 | 5 | 0 | 0 | 0 |
| 35 | 3.125 | 4 | 4 | 5 | 4 | | | | | | | 0 | 0 | 0 |
| | 6.25 | 5 | 5 | 5 | 5 | | | | | | | 0 | 0 | 0 |
| | 12.5 | 5 | 5 | 5 | 5 | | | | | | | 0 | 0 | 0 |
| | 25 | 5 | 5 | 5 | 5 | | | | | | | 0 | 0 | 0 |
| 36 | 3.125 | 5 | 5 | 5 | 5 | 5 | 4 | 4 | 4 | 1 | 5 | 0 | 0 | 0 |
| | 6.25 | 5 | 5 | 5 | 5 | 5 | 5 | 5 | 5 | 4 | 5 | 0 | 0 | 0 |
| | 12.5 | 5 | 5 | 5 | 5 | 5 | 5 | 5 | 5 | 4 | 5 | 0 | 0 | 1 |
| | 25 | 5 | 5 | 5 | 5 | 5 | 5 | 5 | 5 | 5 | 5 | 0 | 0 | 1 |
| 37 | 3.125 | 4 | 5 | 5 | 5 | 4 | 3 | 4 | 3 | 3 | 5 | 0 | 0 | 0 |
| | 6.25 | 5 | 5 | 5 | 5 | 4 | 4 | 5 | 5 | 4 | 5 | 0 | 0 | 0 |
| | 12.5 | 5 | 5 | 5 | 5 | 5 | 4 | 5 | 5 | 5 | 5 | 0 | 0 | 0 |
| | 25 | 5 | 5 | 5 | 5 | 5 | 5 | 5 | 5 | 5 | 5 | 0 | 0 | 0 |
| 41 | 3.125 | 3 | 2 | 5 | 5 | 3 | 0 | 4 | 3 | 0 | 5 | 0 | 0 | 0 |
| | 6.25 | 4 | 4 | 5 | 5 | 4 | 3 | 5 | 5 | 3 | 5 | 0 | 0 | 0 |
| | 12.5 | 5 | 5 | 5 | 5 | 5 | 5 | 5 | 5 | 4 | 5 | 0 | 0 | 0 |
| | 25 | 5 | 5 | 5 | 5 | 5 | 5 | 5 | 5 | 5 | 5 | 0 | 0 | 0 |
| 43 | 3.125 | 5 | 5 | 5 | 5 | 3 | 4 | 4 | 5 | 3 | 5 | 0 | 0 | 0 |
| | 6.25 | 5 | 5 | 5 | 5 | 4 | 5 | 5 | 5 | 4 | 5 | 0 | 0 | 0 |
| | 12.5 | 5 | 5 | 5 | 5 | 5 | 5 | 5 | 5 | 4 | 5 | 0 | 0 | 0 |
| | 25 | 5 | 5 | 5 | 5 | 5 | 5 | 5 | 5 | 5 | 5 | 0 | 0 | 0 |
| 45 | 3.125 | 5 | 3 | 3 | 5 | 3 | 1 | 2 | 1 | 3 | 4 | 0 | 0 | 0 |
| | 6.25 | 5 | 4 | 5 | 5 | 5 | 1 | 3 | 3 | 4 | 5 | 0 | 0 | 0 |
| | 12.5 | 5 | 5 | 5 | 5 | 5 | 4 | 5 | 5 | 4 | 5 | 0 | 0 | 0 |
| | 25 | 5 | 5 | 5 | 5 | 5 | 5 | 5 | 5 | 5 | 5 | 0 | 0 | 0 |
| 46 | 3.125 | 5 | 5 | 5 | 5 | 5 | 4 | 4 | 5 | 4 | 5 | 0 | 0 | 0 |
| | 6.25 | 5 | 5 | 5 | 5 | 5 | 5 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 0 |

Table 2:

| Compound No. | Dose g/a | Herbicidal effect rating | | | | | | | | | | Phytotoxicity rating | | |
|---|---|---|---|---|---|---|---|---|---|---|---|---|---|---|
| | | A | B | C | D | E | F | G | H | I | J | K | L | M |
| 47 | 3.125<br>6.25<br>12.5<br>25 | 3<br>4<br>5<br>5 | 2<br>3<br>5<br>5 | 5<br>5<br>5<br>5 | 5<br>5<br>5<br>5 | 3<br>5<br>5<br>5 | 0<br>2<br>3<br>4 | 4<br>4<br>4<br>5 | 2<br>5<br>5<br>5 | 3<br>3<br>5<br>5 | 4<br>5<br>5<br>5 | 0<br>0<br>0<br>0 | 0<br>0<br>0<br>0 | 0<br>0<br>0<br>0 |
| 48 | 3.125<br>6.25<br>12.5<br>25 | 5<br>5<br>5<br>5 | 3<br>3<br>4<br>5 | 5<br>5<br>5<br>5 | 5<br>5<br>5<br>5 | 5<br>5<br>5<br>5 | 4<br>5<br>5<br>5 | 5<br>5<br>5<br>5 | 5<br>5<br>5<br>5 | 4<br>4<br>5<br>5 | 5<br>5<br>5<br>5 | 0<br>0<br>0<br>0 | 0<br>0<br>0<br>0 | 0<br>0<br>0<br>0 |
| 50 | 3.125<br>6.25<br>12.5<br>25 | 5<br>5<br>5<br>5 | 5<br>5<br>5<br>5 | 5<br>5<br>5<br>5 | 5<br>5<br>5<br>5 | 4<br>5<br>5<br>5 | 3<br>5<br>5<br>5 | 5<br>5<br>5<br>5 | 5<br>5<br>5<br>5 | 0<br>2<br>3<br>5 | 5<br>5<br>5<br>5 | 0<br>0<br>0<br>0 | 0<br>0<br>0<br>0 | 0<br>0<br>0<br>0 |
| 52 | 3.125<br>6.25<br>12.5<br>25 | 5<br>5<br>5<br>5 | 5<br>5<br>5<br>5 | 5<br>5<br>5<br>5 | 5<br>5<br>5<br>5 | 5<br>5<br>5<br>5 | 5<br>5<br>5<br>5 | 5<br>5<br>5<br>5 | 5<br>5<br>5<br>5 | 5<br>5<br>5<br>5 | 5<br>5<br>5<br>5 | 0<br>0<br>0<br>0 | 0<br>0<br>0<br>0 | 0<br>0<br>0<br>0 |
| 53 | 3.125<br>6.25<br>12.5<br>25 | 5<br>5<br>5<br>5 | 5<br>5<br>5<br>5 | 5<br>5<br>5<br>5 | 5<br>5<br>5<br>5 | 5<br>5<br>5<br>5 | 4<br>5<br>5<br>5 | 5<br>5<br>5<br>5 | 5<br>5<br>5<br>5 | 4<br>4<br>5<br>5 | 5<br>5<br>5<br>5 | 0<br>0<br>1<br>1 | 0<br>0<br>0<br>1 | 0<br>0<br>1<br>2 |
| 57 | 3.125<br>6.25<br>12.5<br>25 | 3<br>4<br>5<br>5 | 2<br>3<br>4<br>5 | 5<br>5<br>5<br>5 | 2<br>4<br>5<br>5 | 4<br>5<br>5<br>5 | | | | | | 0<br>0<br>0<br>0 | 0<br>0<br>0<br>0 | 0<br>0<br>0<br>0 |
| 59 | 3.125<br>6.25<br>12.5<br>25 | 5<br>5<br>5<br>5 | 4<br>5<br>5<br>5 | 5<br>5<br>5<br>5 | 5<br>5<br>5<br>5 | 5<br>5<br>5<br>5 | 4<br>5<br>5<br>5 | 4<br>5<br>5<br>5 | 5<br>5<br>5<br>5 | 5<br>5<br>5<br>5 | 5<br>5<br>5<br>5 | 0<br>0<br>0<br>0 | 0<br>0<br>0<br>0 | 0<br>0<br>0<br>0 |
| 60 | 3.125<br>6.25<br>12.5<br>25 | 4<br>4<br>5<br>5 | 4<br>4<br>5<br>5 | 5<br>5<br>5<br>5 | 5<br>5<br>5<br>5 | 5<br>5<br>5<br>5 | 5<br>5<br>5<br>5 | 5<br>5<br>5<br>5 | 5<br>5<br>5<br>5 | 3<br>3<br>4<br>5 | 5<br>5<br>5<br>5 | 0<br>0<br>0<br>0 | 0<br>0<br>0<br>1 | 0<br>0<br>0<br>1 |

Table 2:

| Compound No. | Dose g/a | Herbicidal effect rating | | | | | | | | | | Phytotoxicity rating | | |
|---|---|---|---|---|---|---|---|---|---|---|---|---|---|---|
| | | A | B | C | D | E | F | G | H | I | J | K | L | M |
| 62 | 3.125 | 5 | 4 | 5 | 5 | 5 | 3 | 4 | 5 | 5 | 5 | 0 | 0 | 0 |
| | 6.25 | 5 | 5 | 5 | 5 | 5 | 4 | 4 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 1 |
| 65 | 3.125 | 5 | 4 | 5 | 5 | 5 | 5 | 5 | 5 | 5 | 5 | 0 | 0 | 0 |
| | 6.25 | 5 | 5 | 5 | 5 | 5 | 5 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 1 | 2 |
| 66 | 3.125 | 4 | 4 | 5 | 4 | 4 | 4 | 4 | 4 | 3 | 5 | 0 | 0 | 0 |
| | 6.25 | 5 | 5 | 5 | 4 | 5 | 4 | 5 | 5 | 3 | 5 | 0 | 0 | 0 |
| | 12.5 | 5 | 5 | 5 | 5 | 5 | 5 | 5 | 5 | 4 | 5 | 0 | 0 | 0 |
| | 25 | 5 | 5 | 5 | 5 | 5 | 5 | 5 | 5 | 5 | 5 | 0 | 0 | 0 |
| 67 | 3.125 | 4 | 3 | 5 | 5 | 5 | 3 | 4 | 4 | 3 | 5 | 0 | 0 | 0 |
| | 6.25 | 4 | 4 | 5 | 5 | 5 | 3 | 5 | 4 | 4 | 5 | 0 | 0 | 0 |
| | 12.5 | 5 | 5 | 5 | 5 | 5 | 4 | 5 | 5 | 4 | 5 | 0 | 0 | 0 |
| | 25 | 5 | 5 | 5 | 5 | 5 | 5 | 5 | 5 | 5 | 5 | 0 | 0 | 0 |
| 69 | 3.125 | 5 | 4 | 5 | 5 | 5 | 5 | 4 | 5 | 5 | 5 | 0 | 0 | 0 |
| | 6.25 | 5 | 5 | 5 | 5 | 5 | 5 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 1 | 1 |
| 70 | 3.125 | 3 | 3 | 5 | 5 | 5 | 4 | 4 | 5 | 3 | 5 | 0 | 0 | 0 |
| | 6.25 | 5 | 4 | 5 | 5 | 5 | 4 | 5 | 5 | 4 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 1 | 0 | 1 |
| 72 | 3.125 | 5 | 4 | 5 | 5 | 5 | 4 | 5 | 5 | 4 | 5 | 0 | 0 | 0 |
| | 6.25 | 5 | 5 | 5 | 5 | 5 | 5 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 1 | 0 |
| 73 | 3.125 | 4 | 3 | 4 | 4 | 4 | 2 | 3 | 3 | 2 | 5 | 0 | 0 | 0 |
| | 6.25 | 4 | 3 | 5 | 4 | 5 | 3 | 3 | 4 | 3 | 5 | 0 | 0 | 0 |
| | 12.5 | 5 | 5 | 5 | 5 | 5 | 4 | 5 | 5 | 3 | 5 | 0 | 0 | 0 |
| | 25 | 5 | 5 | 5 | 5 | 5 | 5 | 5 | 5 | 5 | 5 | 0 | 0 | 0 |

Table 2:

| Compound No. | Dose g/a | Herbicidal effect rating A | B | C | D | E | F | G | H | I | J | Phytotoxicity rating K | L | M |
|---|---|---|---|---|---|---|---|---|---|---|---|---|---|---|
| 75 | 3.125 | 5 | 4 | 5 | 5 | 5 | 3 | 2 | 3 | 5 | 5 | 0 | 0 | 0 |
| | 6.25 | 5 | 4 | 5 | 5 | 5 | 3 | 3 | 4 | 5 | 5 | 0 | 0 | 0 |
| | 12.5 | 5 | 5 | 5 | 5 | 5 | 4 | 4 | 4 | 5 | 5 | 0 | 0 | 0 |
| | 25 | 5 | 5 | 5 | 5 | 5 | 5 | 5 | 5 | 5 | 5 | 0 | 0 | 0 |
| 76 | 3.125 | 4 | 4 | 5 | 5 | 5 | 5 | 4 | 2 | 4 | 4 | 0 | 0 | 0 |
| | 6.25 | 5 | 5 | 5 | 5 | 5 | 5 | 5 | 3 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 0 |
| 79 | 3.125 | 3 | 3 | 5 | 5 | 5 | 3 | 4 | 3 | 3 | 4 | 0 | 0 | 0 |
| | 6.25 | 4 | 3 | 5 | 5 | 5 | 4 | 4 | 5 | 4 | 5 | 0 | 0 | 0 |
| | 12.5 | 5 | 4 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 0 |
| 83 | 3.125 | 4 | 3 | 5 | 5 | 5 | | | | | | 0 | 0 | 0 |
| | 6.25 | 4 | 3 | 5 | 5 | 5 | | | | | | 0 | 0 | 0 |
| | 12.5 | 5 | 4 | 5 | 5 | 5 | | | | | | 0 | 0 | 0 |
| | 25 | 5 | 5 | 5 | 5 | 5 | | | | | | 0 | 0 | 0 |
| 84 | 3.125 | 5 | 3 | 5 | 2 | 5 | | | | | | 0 | 0 | 0 |
| | 6.25 | 5 | 5 | 5 | 5 | 5 | | | | | | 0 | 0 | 0 |
| | 12.5 | 5 | 5 | 5 | 5 | 5 | | | | | | 1 | 1 | 2 |
| | 25 | 5 | 5 | 5 | 5 | 5 | | | | | | 1 | 1 | 2 |
| 86 | 3.125 | 4 | 1 | 5 | 3 | 5 | | | | | | 0 | 0 | 0 |
| | 6.25 | 4 | 1 | 5 | 4 | 5 | | | | | | 0 | 0 | 0 |
| | 12.5 | 5 | 4 | 5 | 4 | 5 | | | | | | 0 | 0 | 1 |
| | 25 | 5 | 5 | 5 | 5 | 5 | | | | | | 0 | 0 | 1 |
| 89 | 3.125 | 2 | 1 | 5 | 4 | 3 | 0 | 4 | 3 | 0 | 5 | 0 | 0 | 0 |
| | 6.25 | 3 | 3 | 5 | 5 | 5 | 2 | 5 | 5 | 3 | 5 | 0 | 0 | 0 |
| | 12.5 | 5 | 5 | 5 | 5 | 5 | 5 | 5 | 5 | 4 | 5 | 0 | 0 | 0 |
| | 25 | 5 | 5 | 5 | 5 | 5 | 5 | 5 | 5 | 5 | 5 | 0 | 0 | 0 |
| 92 | 3.125 | 2 | | 3 | 4 | 4 | | | | | | 0 | 0 | 0 |
| | 6.25 | 4 | | 4 | 5 | 4 | | | | | | 0 | 0 | 0 |
| | 12.5 | 4 | | 5 | 5 | 5 | | | | | | 0 | 0 | 0 |
| | 25 | 5 | | 5 | 5 | 5 | | | | | | 0 | 0 | 0 |

Table 2:

| Compound No. | Dose g/a | Herbicidal effect rating | | | | | | | | | | Phytotoxicity rating | | |
|---|---|---|---|---|---|---|---|---|---|---|---|---|---|---|
| | | A | B | C | D | E | F | G | H | I | J | K | L | M |
| 100 | 3.125 | 5 | 1 | 5 | 5 | 5 | 2 | 5 | 4 | 5 | 5 | 0 | 0 | 0 |
| | 6.25 | 5 | 2 | 5 | 5 | 5 | 3 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 1 |
| 101 | 3.125 | 5 | 1 | 5 | 5 | 5 | 3 | 3 | 4 | 5 | 5 | 0 | 0 | 0 |
| | 6.25 | 5 | 2 | 5 | 5 | 5 | 5 | 4 | 5 | 5 | 5 | 0 | 0 | 0 |
| | 12.5 | 5 | 2 | 5 | 5 | 5 | 5 | 5 | 5 | 5 | 5 | 0 | 0 | 0 |
| | 25 | 5 | 4 | 5 | 5 | 5 | 5 | 5 | 5 | 5 | 5 | 0 | 0 | 0 |
| 102 | 3.125 | 5 | 5 | 5 | 5 | 5 | 4 | 5 | 5 | 5 | 5 | 0 | 0 | 0 |
| | 6.25 | 5 | 5 | 5 | 5 | 5 | 5 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 1 | 0 | 1 |
| 103 | 3.125 | 5 | 5 | 5 | 5 | 5 | 5 | 5 | 5 | 5 | 5 | 0 | 0 | 0 |
| | 6.25 | 5 | 5 | 5 | 5 | 5 | 5 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 1 |
| 105 | 3.125 | 2 | 0 | 5 | 3 | 5 | | | | | | 0 | 0 | 0 |
| | 6.25 | 4 | 1 | 5 | 4 | 5 | | | | | | 0 | 0 | 0 |
| | 12.5 | 5 | 3 | 5 | 5 | 5 | | | | | | 0 | 0 | 0 |
| | 25 | 5 | 5 | 5 | 5 | 5 | | | | | | 0 | 0 | 0 |
| 113 | 3.125 | 5 | 5 | 5 | 5 | 5 | 2 | 5 | 4 | 3 | 5 | 0 | 0 | 0 |
| | 6.25 | 5 | 5 | 5 | 5 | 5 | 4 | 5 | 5 | 3 | 5 | 0 | 0 | 0 |
| | 12.5 | 5 | 5 | 5 | 5 | 5 | 4 | 5 | 5 | 4 | 5 | 0 | 0 | 0 |
| | 25 | 5 | 5 | 5 | 5 | 5 | 5 | 5 | 5 | 5 | 5 | 0 | 0 | 0 |
| 114 | 3.125 | 5 | 5 | 5 | 5 | 5 | 2 | 5 | 5 | 4 | 5 | 0 | 0 | 0 |
| | 6.25 | 5 | 5 | 5 | 5 | 5 | 2 | 5 | 5 | 5 | 5 | 0 | 0 | 0 |
| | 12.5 | 5 | 5 | 5 | 5 | 5 | 4 | 5 | 5 | 5 | 5 | 0 | 0 | 0 |
| | 25 | 5 | 5 | 5 | 5 | 5 | 5 | 5 | 5 | 5 | 5 | 0 | 0 | 0 |
| 116 | 3.125 | 5 | 4 | 5 | 4 | 5 | 2 | 2 | 3 | 4 | 5 | 0 | 0 | 0 |
| | 6.25 | 5 | 5 | 5 | 5 | 5 | 3 | 3 | 3 | 5 | 5 | 0 | 0 | 0 |
| | 12.5 | 5 | 5 | 5 | 5 | 5 | 5 | 4 | 5 | 5 | 5 | 0 | 0 | 0 |
| | 25 | 5 | 5 | 5 | 5 | 5 | 5 | 5 | 5 | 5 | 5 | 2 | 0 | 1 |

Table 2:

| Compound No. | Dose g/a | Herbicidal effect rating A | B | C | D | E | F | G | H | I | J | Phytotoxicity rating K | L | M |
|---|---|---|---|---|---|---|---|---|---|---|---|---|---|---|
| 121 | 3.125 | 1 | 2 | 4 | 3 | 2 | | | | | | 0 | 0 | 0 |
| | 6.25 | 4 | 4 | 5 | 5 | 5 | | | | | | 0 | 0 | 0 |
| | 12.5 | 5 | 5 | 5 | 5 | 5 | | | | | | 0 | 0 | 0 |
| | 25 | 5 | 5 | 5 | 5 | 5 | | | | | | 0 | 0 | 0 |
| 122 | 3.125 | 5 | | 5 | 4 | 5 | | | | | | 0 | 0 | 0 |
| | 6.25 | 5 | | 5 | 5 | 5 | | | | | | 0 | 0 | 0 |
| | 12.5 | 5 | | 5 | 5 | 5 | | | | | | 0 | 0 | 0 |
| | 25 | 5 | | 5 | 5 | 5 | | | | | | 0 | 0 | 0 |
| 128 | 3.125 | 5 | 4 | 5 | 5 | 5 | | | | | | 0 | 0 | 0 |
| | 6.25 | 5 | 4 | 5 | 5 | 5 | | | | | | 0 | 0 | 0 |
| | 12.5 | 5 | 4 | 5 | 5 | 5 | | | | | | 0 | 0 | 0 |
| | 25 | 5 | 5 | 5 | 5 | 5 | | | | | | 0 | 0 | 0 |
| 137 | 3.125 | 4 | 5 | 5 | 5 | 5 | 4 | 1 | 2 | 3 | 5 | 0 | 0 | 0 |
| | 6.25 | 5 | 5 | 5 | 5 | 5 | 5 | 4 | 5 | 3 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 0 |
| 138 | 3.125 | 4 | 5 | 5 | 5 | 5 | 1 | 4 | 2 | 2 | 5 | 0 | 0 | 0 |
| | 6.25 | 5 | 5 | 5 | 5 | 5 | 2 | 5 | 2 | 3 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 0 |
| 139 | 3.125 | 5 | 4 | 5 | 5 | 3 | 2 | 4 | 4 | 2 | 5 | 0 | 0 | 0 |
| | 6.25 | 5 | 5 | 5 | 5 | 4 | 4 | 5 | 5 | 2 | 5 | 0 | 0 | 0 |
| | 12.5 | 5 | 5 | 5 | 5 | 5 | 4 | 5 | 5 | 4 | 5 | 0 | 0 | 0 |
| | 25 | 5 | 5 | 5 | 5 | 5 | 5 | 5 | 5 | 5 | 5 | 0 | 0 | 0 |
| 140 | 3.125 | 4 | 5 | 5 | 5 | 5 | 5 | 4 | 5 | 4 | 5 | 0 | 0 | 0 |
| | 6.25 | 5 | 5 | 5 | 5 | 5 | 5 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 1 | 0 |
| 141 | 3.125 | 5 | 5 | 5 | 5 | 5 | 4 | 4 | 5 | 4 | 4 | 0 | 0 | 0 |
| | 6.25 | 5 | 5 | 5 | 5 | 5 | 4 | 5 | 5 | 4 | 5 | 0 | 0 | 0 |
| | 12.5 | 5 | 5 | 5 | 5 | 5 | 5 | 5 | 5 | 4 | 5 | 0 | 0 | 0 |
| | 25 | 5 | 5 | 5 | 5 | 5 | 5 | 5 | 5 | 5 | 5 | 0 | 0 | 0 |

Table 2:

| Compound No. | Dose g/a | Herbicidal effect rating | | | | | | | | | | Phytotoxicity rating | | |
|---|---|---|---|---|---|---|---|---|---|---|---|---|---|---|
| | | A | B | C | D | E | F | G | H | I | J | K | L | M |
| 142 | 3.125 | 5 | 5 | 5 | 5 | 5 | 5 | 4 | 5 | 5 | 5 | 0 | 0 | 0 |
| | 6.25 | 5 | 5 | 5 | 5 | 5 | 5 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 0 |
| 143 | 3.125 | 4 | 2 | 5 | 4 | 5 | | | | | | 0 | 0 | 0 |
| | 6.25 | 5 | 4 | 5 | 5 | 5 | | | | | | 0 | 0 | 0 |
| | 12.5 | 5 | 4 | 5 | 5 | 5 | | | | | | 0 | 0 | 0 |
| | 25 | 5 | 5 | 5 | 5 | 5 | | | | | | 0 | 0 | 0 |
| 144 | 3.125 | 5 | 5 | 5 | 5 | 5 | 4 | 5 | 4 | 5 | 5 | 0 | 0 | 0 |
| | 6.25 | 5 | 5 | 5 | 5 | 5 | 4 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 1 |
| 145 | 3.125 | 5 | 4 | 5 | 5 | 5 | 2 | 5 | 5 | 2 | 5 | 0 | 0 | 0 |
| | 6.25 | 5 | 5 | 5 | 5 | 5 | 5 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 1 |
| 148 | 3.125 | 5 | 4 | 5 | 5 | 5 | 5 | 5 | 5 | 5 | 5 | 0 | 0 | 0 |
| | 6.25 | 5 | 5 | 5 | 5 | 5 | 5 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 0 |
| 149 | 3.125 | 5 | 5 | 5 | 5 | 5 | 5 | 4 | 3 | 4 | 5 | 0 | 0 | 0 |
| | 6.25 | 5 | 5 | 5 | 5 | 5 | 5 | 5 | 5 | 4 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 1 | 1 | 1 |
| 150 | 3.125 | 3 | 3 | 5 | 5 | 5 | 2 | 2 | 3 | 3 | 5 | 0 | 0 | 0 |
| | 6.25 | 4 | 3 | 5 | 5 | 5 | 3 | 4 | 3 | 3 | 5 | 0 | 0 | 0 |
| | 12.5 | 5 | 4 | 5 | 5 | 5 | 3 | 5 | 4 | 4 | 5 | 0 | 0 | 0 |
| | 25 | 5 | 5 | 5 | 5 | 5 | 5 | 5 | 5 | 5 | 5 | 0 | 0 | 0 |
| 151 | 3.125 | 5 | 5 | 5 | 5 | 5 | 3 | 5 | 5 | 2 | 5 | 0 | 0 | 0 |
| | 6.25 | 5 | 5 | 5 | 5 | 5 | 5 | 5 | 5 | 2 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 0 |

Table 2:

| Compound No. | Dose g/a | Herbicidal effect rating | | | | | | | | | | Phytotoxicity rating | | |
|---|---|---|---|---|---|---|---|---|---|---|---|---|---|---|
| | | A | B | C | D | E | F | G | H | I | J | K | L | M |
| 154 | 3.125 | 5 | 5 | 5 | 5 | 5 | 5 | 5 | 5 | 5 | 5 | 0 | 0 | 0 |
| | 6.25 | 5 | 5 | 5 | 5 | 5 | 5 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 0 |
| 155 | 3.125 | 5 | 4 | 5 | 5 | 5 | 4 | 4 | 5 | 3 | 5 | 0 | 0 | 0 |
| | 6.25 | 5 | 4 | 5 | 5 | 5 | 5 | 5 | 5 | 4 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 1 | 1 |
| 156 | 3.125 | 3 | 2 | 5 | 4 | 4 | 4 | 5 | 5 | 2 | 4 | 0 | 0 | 0 |
| | 6.25 | 4 | 4 | 5 | 5 | 5 | 4 | 5 | 5 | 3 | 5 | 0 | 0 | 0 |
| | 12.5 | 5 | 4 | 5 | 5 | 5 | 5 | 5 | 5 | 4 | 5 | 0 | 0 | 0 |
| | 25 | 5 | 5 | 5 | 5 | 5 | 5 | 5 | 5 | 5 | 5 | 0 | 0 | 0 |
| 158 | 3.125 | 5 | 5 | 5 | 5 | 5 | 5 | 5 | 5 | 5 | 5 | 0 | 0 | 0 |
| | 6.25 | 5 | 5 | 5 | 5 | 5 | 5 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 1 | 1 | 2 |
| 160 | 3.125 | 3 | 3 | 5 | 5 | 5 | 4 | 5 | 5 | 5 | 5 | 0 | 0 | 0 |
| | 6.25 | 5 | 4 | 5 | 5 | 5 | 4 | 5 | 5 | 5 | 5 | 0 | 0 | 0 |
| | 12.5 | 5 | 4 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 1 | 0 | 1 |
| 161 | 3.125 | 5 | 4 | 5 | 5 | 5 | 4 | 5 | 5 | 5 | 5 | 0 | 0 | 0 |
| | 6.25 | 5 | 5 | 5 | 5 | 5 | 5 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 1 |
| 162 | 3.125 | 5 | 5 | 5 | 5 | 5 | 3 | 5 | 5 | 5 | 5 | 0 | 0 | 0 |
| | 6.25 | 5 | 5 | 5 | 5 | 5 | 4 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 1 | 2 |
| 163 | 3.125 | 5 | 4 | 5 | 5 | 5 | 4 | 5 | 4 | 5 | 4 | 0 | 0 | 0 |
| | 6.25 | 5 | 4 | 5 | 5 | 5 | 5 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 0 |

Table 2:

| Compound No. | Dose g/a | Herbicidal effect rating | | | | | | | | | | Phytotoxicity rating | | |
|---|---|---|---|---|---|---|---|---|---|---|---|---|---|---|
| | | A | B | C | D | E | F | G | H | I | J | K | L | M |
| 164 | 3.125 | 4 | 3 | 5 | 4 | 4 | 4 | 4 | 3 | 4 | 5 | 0 | 0 | 0 |
| | 6.25 | 5 | 4 | 5 | 5 | 5 | 5 | 5 | 5 | 4 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 1 |
| 165 | 3.125 | 5 | 4 | 5 | 5 | 5 | 4 | 5 | 5 | 4 | 4 | 0 | 0 | 0 |
| | 6.25 | 5 | 5 | 5 | 5 | 5 | 5 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 1 | 0 | 2 |
| 166 | 3.125 | 4 | 4 | 5 | 5 | 5 | 3 | 4 | 3 | 5 | 5 | 0 | 0 | 0 |
| | 6.25 | 5 | 4 | 5 | 5 | 5 | 4 | 5 | 4 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 0 |
| 167 | 3.125 | 5 | 5 | 5 | 5 | 5 | 5 | 5 | 5 | 4 | 5 | 0 | 0 | 0 |
| | 6.25 | 5 | 5 | 5 | 5 | 5 | 5 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 1 | 2 |
| 168 | 3.125 | 5 | 4 | 5 | 5 | 5 | 3 | 4 | 5 | 5 | 5 | 0 | 0 | 0 |
| | 6.25 | 5 | 5 | 5 | 5 | 5 | 5 | 4 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 0 |
| 169 | 3.125 | 5 | 5 | 5 | 5 | 5 | 4 | 4 | 4 | 5 | 4 | 0 | 0 | 0 |
| | 6.25 | 5 | 5 | 5 | 5 | 5 | 4 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 0 |
| 170 | 3.125 | 3 | 3 | 5 | 5 | 5 | 5 | 5 | 4 | 5 | 5 | 0 | 0 | 0 |
| | 6.25 | 5 | 4 | 5 | 5 | 5 | 5 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 1 |
| 171 | 3.125 | 5 | 4 | 5 | 5 | 5 | 4 | 3 | 4 | 5 | 5 | 0 | 0 | 0 |
| | 6.25 | 5 | 5 | 5 | 5 | 5 | 4 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 0 |

Table 2:

| Compound No. | Dose g/a | Herbicidal effect rating | | | | | | | | | | Phytotoxicity rating | | |
|---|---|---|---|---|---|---|---|---|---|---|---|---|---|---|
| | | A | B | C | D | E | F | G | H | I | J | K | L | M |
| 175 | 3.125 | 5 | 5 | 5 | 5 | 4 | 2 | 4 | 5 | 3 | 5 | 0 | 0 | 0 |
| | 6.25 | 5 | 5 | 5 | 5 | 5 | 2 | 5 | 5 | 3 | 5 | 0 | 0 | 0 |
| | 12.5 | 5 | 5 | 5 | 5 | 5 | 4 | 5 | 5 | 4 | 5 | 0 | 0 | 0 |
| | 25 | 5 | 5 | 5 | 5 | 5 | 5 | 5 | 5 | 5 | 5 | 0 | 0 | 0 |
| 177 | 3.125 | 3 | 2 | 5 | 5 | 4 | | | | | | 0 | 0 | 0 |
| | 6.25 | 4 | 3 | 5 | 5 | 5 | | | | | | 0 | 0 | 0 |
| | 12.5 | 4 | 4 | 5 | 5 | 5 | | | | | | 0 | 0 | 0 |
| | 25 | 5 | 5 | 5 | 5 | 5 | | | | | | 0 | 0 | 0 |
| 178 | 3.125 | 4 | 4 | 5 | 5 | 5 | 1 | 5 | 5 | 3 | 4 | 0 | 0 | 0 |
| | 6.25 | 5 | 4 | 5 | 5 | 5 | 2 | 5 | 5 | 3 | 5 | 0 | 0 | 0 |
| | 12.5 | 5 | 5 | 5 | 5 | 5 | 4 | 5 | 5 | 4 | 5 | 0 | 0 | 0 |
| | 25 | 5 | 5 | 5 | 5 | 5 | 5 | 5 | 5 | 5 | 5 | 0 | 1 | 0 |
| 179 | 3.125 | 5 | 5 | 5 | 5 | 5 | | | | | | 0 | 0 | 0 |
| | 6.25 | 5 | 5 | 5 | 5 | 5 | | | | | | 0 | 0 | 0 |
| | 12.5 | 5 | 5 | 5 | 5 | 5 | | | | | | 0 | 0 | 0 |
| | 25 | 5 | 5 | 5 | 5 | 5 | | | | | | 0 | 0 | 1 |
| 180 | 3.125 | 5 | 5 | 5 | 5 | 5 | 2 | 5 | 5 | 5 | 5 | 0 | 0 | 0 |
| | 6.25 | 5 | 5 | 5 | 5 | 5 | 3 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 0 |
| 182 | 3.125 | 5 | 5 | 5 | 5 | 4 | 1 | 4 | 1 | 4 | 5 | 0 | 0 | 0 |
| | 6.25 | 5 | 5 | 5 | 5 | 5 | 2 | 5 | 5 | 5 | 5 | 0 | 0 | 0 |
| | 12.5 | 5 | 5 | 5 | 5 | 5 | 4 | 5 | 5 | 5 | 5 | 0 | 0 | 0 |
| | 25 | 5 | 5 | 5 | 5 | 5 | 5 | 5 | 5 | 5 | 5 | 0 | 0 | 0 |
| 199 | 3.125 | 4 | 1 | 5 | 4 | 5 | | | | | | 0 | 0 | 0 |
| | 6.25 | 4 | 2 | 5 | 4 | 5 | | | | | | 0 | 0 | 0 |
| | 12.5 | 5 | 4 | 5 | 4 | 5 | | | | | | 0 | 0 | 0 |
| | 25 | 5 | 5 | 5 | 5 | 5 | | | | | | 0 | 0 | 0 |
| 201 | 3.125 | 3 | | 2 | 3 | 3 | | | | | | 0 | 0 | 0 |
| | 6.25 | 4 | | 4 | 3 | 3 | | | | | | 0 | 0 | 0 |
| | 12.5 | 4 | | 5 | 4 | 4 | | | | | | 0 | 0 | 0 |
| | 25 | 4 | | 5 | 4 | 5 | | | | | | 0 | 0 | 0 |

Table 2:

| Compound No. | Dose g/a | Herbicidal effect rating | | | | | | | | | | Phytotoxicity rating | | |
|---|---|---|---|---|---|---|---|---|---|---|---|---|---|---|
| | | A | B | C | D | E | F | G | H | I | J | K | L | M |
| 203 | 3.125 | 1 | 2 | 3 | 2 | 4 | | | | | | 0 | 0 | 0 |
| | 6.25 | 2 | 2 | 5 | 5 | 5 | | | | | | 0 | 0 | 0 |
| | 12.5 | 4 | 4 | 5 | 5 | 5 | | | | | | 0 | 0 | 0 |
| | 25 | 5 | 5 | 5 | 5 | 5 | | | | | | 0 | 0 | 0 |
| 206 | 3.125 | 2 | 1 | 5 | 4 | 5 | | | | | | 0 | 0 | 0 |
| | 6.25 | 2 | 3 | 5 | 4 | 5 | | | | | | 0 | 0 | 0 |
| | 12.5 | 5 | 4 | 5 | 5 | 5 | | | | | | 0 | 0 | 0 |
| | 25 | 5 | 5 | 5 | 5 | 5 | | | | | | 1 | 0 | 1 |
| 209 | 3.125 | 3 | 4 | 5 | 4 | 3 | 1 | 1 | 5 | 1 | 4 | 0 | 0 | 0 |
| | 6.25 | 5 | 4 | 5 | 5 | 5 | 4 | 4 | 5 | 2 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 0 |
| 216 | 3.125 | 1 | 2 | 4 | 2 | 3 | | | | | | 0 | 0 | 0 |
| | 6.25 | 3 | 3 | 4 | 3 | 3 | | | | | | 0 | 0 | 0 |
| | 12.5 | 5 | 4 | 5 | 4 | 5 | | | | | | 1 | 0 | 0 |
| | 25 | 5 | 5 | 5 | 5 | 5 | | | | | | 1 | 0 | 2 |
| 222 | 3.125 | 1 | 3 | 2 | 4 | 3 | | | | | | 0 | 0 | 0 |
| | 6.25 | 2 | 3 | 3 | 5 | 4 | | | | | | 0 | 0 | 0 |
| | 12.5 | 2 | 4 | 3 | 5 | 5 | | | | | | 0 | 0 | 0 |
| | 25 | 3 | 5 | 4 | 5 | 5 | | | | | | 0 | 0 | 0 |
| 224 | 3.125 | 1 | 2 | 3 | 4 | 5 | | | | | | 0 | 0 | 0 |
| | 6.25 | 2 | 4 | 3 | 5 | 5 | | | | | | 0 | 0 | 0 |
| | 12.5 | 2 | 4 | 3 | 5 | 5 | | | | | | 0 | 0 | 0 |
| | 25 | 3 | 5 | 5 | 5 | 5 | | | | | | 0 | 0 | 0 |
| 226 | 3.125 | 2 | | 3 | 1 | 2 | | | | | | 0 | 0 | 0 |
| | 6.25 | 3 | | 4 | 3 | 3 | | | | | | 0 | 0 | 0 |
| | 12.5 | 4 | | 5 | 3 | 4 | | | | | | 0 | 0 | 0 |
| | 25 | 4 | | 5 | 4 | 5 | | | | | | 0 | 0 | 1 |
| 229 | 3.125 | 3 | | 4 | 3 | 4 | | | | | | 0 | 0 | 0 |
| | 6.25 | 3 | | 5 | 4 | 5 | | | | | | 0 | 0 | 0 |
| | 12.5 | 5 | | 5 | 5 | 5 | | | | | | 0 | 0 | 0 |
| | 25 | 5 | | 5 | 5 | 5 | | | | | | 2 | 1 | 0 |

Table 2:

| Compound No. | Dose g/a | Herbicidal effect rating | | | | | | | | | | Phytotoxicity rating | | |
|---|---|---|---|---|---|---|---|---|---|---|---|---|---|---|
| | | A | B | C | D | E | F | G | H | I | J | K | L | M |
| 232 | 3.125 | 4 | | 5 | 4 | 5 | | | | | | 0 | 0 | 0 |
| | 6.25 | 5 | | 5 | 5 | 5 | | | | | | 0 | 0 | 0 |
| | 12.5 | 5 | | 5 | 5 | 5 | | | | | | 0 | 0 | 0 |
| | 25 | 5 | | 5 | 5 | 5 | | | | | | 1 | 1 | 2 |
| 233 | 3.125 | 3 | | 4 | 3 | 4 | | | | | | 0 | 0 | 0 |
| | 6.25 | 3 | | 5 | 4 | 4 | | | | | | 0 | 0 | 0 |
| | 12.5 | 5 | | 5 | 5 | 5 | | | | | | 0 | 0 | 0 |
| | 25 | 5 | | 5 | 5 | 5 | | | | | | 0 | 0 | 0 |
| 240 | 3.125 | 3 | | 3 | 4 | 4 | | | | | | 0 | 0 | 0 |
| | 6.25 | 3 | | 4 | 4 | 5 | | | | | | 0 | 0 | 0 |
| | 12.5 | 4 | | 4 | 5 | 5 | | | | | | 0 | 0 | 0 |
| | 25 | 5 | | 5 | 5 | 5 | | | | | | 0 | 0 | 0 |
| 241 | 3.125 | 3 | 1 | 5 | 3 | 5 | | | | | | 0 | 0 | 0 |
| | 6.25 | 4 | 3 | 5 | 5 | 5 | | | | | | 0 | 0 | 0 |
| | 12.5 | 5 | 3 | 5 | 5 | 5 | | | | | | 1 | 1 | 0 |
| | 25 | 5 | 5 | 5 | 5 | 5 | | | | | | 1 | 1 | 0 |
| 243 | 3.125 | 3 | | 4 | 3 | 4 | | | | | | 0 | 0 | 0 |
| | 6.25 | 4 | | 5 | 5 | 5 | | | | | | 0 | 0 | 0 |
| | 12.5 | 5 | | 5 | 5 | 5 | | | | | | 0 | 0 | 0 |
| | 25 | 5 | | 5 | 5 | 5 | | | | | | 1 | 0 | 2 |
| 245 | 3.125 | 2 | | 4 | 2 | 5 | | | | | | 0 | 0 | 0 |
| | 6.25 | 3 | | 5 | 3 | 5 | | | | | | 0 | 0 | 0 |
| | 12.5 | 4 | | 5 | 5 | 5 | | | | | | 0 | 0 | 0 |
| | 25 | 5 | | 5 | 5 | 5 | | | | | | 0 | 0 | 0 |
| 254 | 3.125 | 0 | | 4 | 2 | 4 | | | | | | 0 | 0 | 0 |
| | 6.25 | 1 | | 5 | 3 | 4 | | | | | | 0 | 0 | 0 |
| | 12.5 | 1 | | 5 | 3 | 5 | | | | | | 0 | 0 | 0 |
| | 25 | 3 | | 5 | 4 | 5 | | | | | | 0 | 0 | 0 |
| 258 | 3.125 | 1 | | 3 | 4 | 2 | | | | | | 0 | 0 | 0 |
| | 6.25 | 1 | | 3 | 5 | 3 | | | | | | 0 | 0 | 0 |
| | 12.5 | 2 | | 4 | 5 | 4 | | | | | | 0 | 0 | 0 |
| | 25 | 4 | | 4 | 5 | 5 | | | | | | 0 | 0 | 0 |

Table 2:

| Compound No. | Dose g/a | Herbicidal effect rating | | | | | | | | | | Phytotoxicity rating | | |
|---|---|---|---|---|---|---|---|---|---|---|---|---|---|---|
| | | A | B | C | D | E | F | G | H | I | J | K | L | M |
| 259 | 3.125 | 4 | | 4 | 3 | 5 | | | | | | 0 | 0 | 0 |
| | 6.25 | 5 | | 5 | 3 | 5 | | | | | | 0 | 0 | 0 |
| | 12.5 | 5 | | 5 | 4 | 5 | | | | | | 0 | 0 | 0 |
| | 25 | 5 | | 5 | 5 | 5 | | | | | | 0 | 0 | 0 |
| 265 | 3.125 | 2 | | 3 | 3 | 4 | | | | | | 0 | 0 | 0 |
| | 6.25 | 3 | | 4 | 3 | 4 | | | | | | 0 | 0 | 0 |
| | 12.5 | 4 | | 5 | 4 | 5 | | | | | | 0 | 0 | 0 |
| | 25 | 5 | | 5 | 5 | 5 | | | | | | 0 | 0 | 0 |
| 277 | 3.125 | 3 | | 4 | 1 | 4 | | | | | | 0 | 0 | 0 |
| | 6.25 | 4 | | 5 | 3 | 5 | | | | | | 0 | 0 | 0 |
| | 12.5 | 5 | | 5 | 4 | 5 | | | | | | 0 | 0 | 0 |
| | 25 | 5 | | 5 | 5 | 5 | | | | | | 1 | 0 | 0 |
| 278 | 3.125 | 2 | | 3 | 2 | 3 | | | | | | 0 | 0 | 0 |
| | 6.25 | 4 | | 5 | 3 | 5 | | | | | | 0 | 0 | 0 |
| | 12.5 | 5 | | 5 | 4 | 5 | | | | | | 0 | 0 | 0 |
| | 25 | 5 | | 5 | 5 | 5 | | | | | | 1 | 0 | 1 |
| Reference A | 3.125 | 0 | | 0 | 1 | 0 | 0 | 0 | 0 | 0 | 0 | 0 | 0 | 0 |
| | 6.25 | 1 | | 1 | 2 | 1 | 0 | 0 | 1 | 1 | 0 | 0 | 1 | 1 |
| | 12.5 | 3 | | 4 | 3 | 2 | 0 | 1 | 1 | 1 | 2 | 0 | 1 | 2 |
| | 25 | 5 | | 5 | 5 | 5 | 2 | 3 | 2 | 3 | 3 | 2 | 2 | 4 |
| Reference B | 3.125 | 1 | | 3 | 0 | 0 | 0 | 0 | 0 | 0 | 0 | 0 | 0 | 0 |
| | 6.25 | 1 | | 4 | 1 | 1 | 0 | 0 | 0 | 0 | 1 | 1 | 0 | 1 |
| | 12.5 | 2 | | 5 | 3 | 2 | 0 | 0 | 2 | 0 | 2 | 1 | 0 | 1 |
| | 25 | 4 | | 5 | 4 | 4 | 0 | 1 | 2 | 1 | 2 | 2 | 1 | 2 |
| Reference C | 3.125 | 5 | 4 | 0 | 0 | 0 | 0 | 0 | 0 | 0 | 0 | 0 | 0 | 1 |
| | 6.25 | 5 | 5 | 1 | 0 | 1 | 0 | 0 | 0 | 1 | 0 | 0 | 2 | 2 |
| | 12.5 | 5 | 5 | 2 | 1 | 2 | 0 | 0 | 0 | 2 | 1 | 1 | 3 | 2 |
| | 25 | 5 | 5 | 3 | 2 | 2 | 0 | 0 | 0 | 2 | 2 | 1 | 4 | 4 |

Table 2:

| Compound No. | Dose g/a | Herbicidal effect rating | | | | | | | | | | Phytotoxicity rating | | |
|---|---|---|---|---|---|---|---|---|---|---|---|---|---|---|
| | | A | B | C | D | E | F | G | H | I | J | K | L | M |
| 282 | 6.25 | 4.5 | 4.5 | 5 | 5 | 5 | | | 2.5 | 4 | 3.5 | 0 | 0 | 0 |
| | 12.5 | 5 | 5 | 5 | 5 | 5 | | | 3 | 4.5 | 4 | 0 | 0 | 0 |
| | 25 | 5 | 5 | 5 | 5 | 5 | | | 3.5 | 5 | 4.5 | 0 | 0 | 0 |
| | 50 | 5 | 5 | 5 | 5 | 5 | | | 5 | 5 | 5 | 0 | 0 | 0 |
| 283 | 3.125 | 3 | 2.5 | 4.5 | 5 | 4.5 | | | | 3.5 | 3.5 | 0 | 0 | 0 |
| | 6.25 | 4.5 | 4 | 5 | 5 | 5 | | | | 4 | 4 | 0 | 0 | 0 |
| | 12.5 | 5 | 5 | 5 | 5 | 5 | | | | 4.5 | 5 | 0 | 0 | 0 |
| | 25 | 5 | 5 | 5 | 5 | 5 | | | | 5 | 5 | 0 | 0 | 1 |
| 285 | 12.5 | 3 | 3 | 4.5 | 4 | 4.5 | | | | 3.5 | 4 | 0 | 0 | 0 |
| | 25 | 4 | 4 | 5 | 5 | 5 | | | | 4.5 | 5 | 0 | 0 | 0 |
| | 50 | 5 | 5 | 5 | 5 | 5 | | | | 5 | 5 | 0 | 0 | 0 |
| 287 | 3.125 | 4 | 4 | 4.5 | 4.5 | 4.5 | | | | 4.5 | 4 | 0 | 0 | 0 |
| | 6.25 | 5 | 4.5 | 4.5 | 5 | 4.5 | | | | 5 | 4.5 | 0 | 0 | 0 |
| | 12.5 | 5 | 4.5 | 5 | 5 | 5 | | | | 5 | 5 | 0 | 0 | 0 |
| | 25 | 5 | 5 | 5 | 5 | 5 | | | | 5 | 5 | 0 | 1 | 2 |
| 288 | 3.125 | 5 | 4.5 | 5 | 5 | 4.5 | | | | 4 | 4.5 | 0 | 0 | 0 |
| | 6.25 | 5 | 5 | 5 | 5 | 5 | | | | 5 | 5 | 0 | 0 | 0 |
| | 12.5 | 5 | 5 | 5 | 5 | 5 | | | | 5 | 5 | 0 | 0 | 0 |
| | 25 | 5 | 5 | 5 | 5 | 5 | | | | 5 | 5 | 1 | 0 | 1 |
| 289 | 3.125 | 5 | 5 | 5 | 5 | 5 | | | 4.5 | 5 | 5 | 0 | 0 | 0 |
| | 6.25 | 5 | 5 | 5 | 5 | 5 | | | 4.5 | 5 | 5 | 0 | 0 | 0 |
| | 12.5 | 5 | 5 | 5 | 5 | 5 | | | 5 | 5 | 5 | 0 | 0 | 1 |
| | 25 | 5 | 5 | 5 | 5 | 5 | | | 5 | 5 | 5 | 0 | 1 | 1 |
| 290 | 3.125 | 5 | 5 | 5 | 5 | 5 | | | 4.5 | 4 | 4.5 | 0 | 0 | 0 |
| | 6.25 | 5 | 5 | 5 | 5 | 5 | | | 5 | 4.5 | 5 | 0 | 0 | 0 |
| | 12.5 | 5 | 5 | 5 | 5 | 5 | | | 5 | 5 | 5 | 1 | 1 | 0 |
| | 25 | 5 | 5 | 5 | 5 | 5 | | | 5 | 5 | 5 | 1 | 2 | 1 |
| 291 | 3.125 | 4 | 4 | 4 | 4.5 | 4 | | | | 4 | 4.5 | 0 | 0 | 0 |
| | 6.25 | 5 | 4.5 | 4.5 | 4.5 | 5 | | | | 4.5 | 5 | 0 | 0 | 0 |
| | 12.5 | 5 | 5 | 5 | 5 | 5 | | | | 5 | 5 | 0 | 1 | 1 |
| | 25 | 5 | 5 | 5 | 5 | 5 | | | | 5 | 5 | 1 | 2 | 2 |

Table 2:

| Compound No. | Dose g/a | Herbicidal effect rating A | B | C | D | E | F | G | H | I | J | Phytotoxicity rating K | L | M |
|---|---|---|---|---|---|---|---|---|---|---|---|---|---|---|
| 292 | 3.125 | 5 | 5 | 5 | 5 | 5 | | | 4.5 | 4 | 4.5 | 0 | 0 | 0 |
| | 6.25 | 5 | 5 | 5 | 5 | 5 | | | 4.5 | 4 | 5 | 0 | 0 | 0 |
| | 12.5 | 5 | 5 | 5 | 5 | 5 | | | 5 | 5 | 5 | 0 | 2 | 1 |
| | 25 | 5 | 5 | 5 | 5 | 5 | | | 5 | 5 | 5 | 0 | 2 | 2 |
| 296 | 6.25 | 4 | 3.5 | 4.5 | 5 | 5 | | | | 4 | 4.5 | 0 | 0 | 0 |
| | 12.5 | 5 | 4.5 | 5 | 5 | 5 | | | | 5 | 5 | 0 | 0 | 0 |
| | 25 | 5 | 5 | 5 | 5 | 5 | | | | 5 | 5 | 0 | 0 | 0 |
| | 50 | 5 | 5 | 5 | 5 | 5 | | | | 5 | 5 | 0 | 0 | 0 |
| 297 | 6.25 | 4 | 3 | 4.5 | 4.5 | 4.5 | | | | 5 | 5 | 0 | 0 | 0 |
| | 12.5 | 4.5 | 4 | 4.5 | 5 | 5 | | | | 5 | 5 | 0 | 0 | 0 |
| | 25 | 5 | 5 | 5 | 5 | 5 | | | | 5 | 5 | 0 | 0 | 0 |
| | 50 | 5 | 5 | 5 | 5 | 5 | | | | 5 | 5 | 0 | 1 | 2 |
| 298 | 3.125 | 3 | 3 | 4 | 4 | 4 | | | | 3.5 | 4 | 0 | 0 | 0 |
| | 6.25 | 4 | 4.5 | 4.5 | 5 | 4.5 | | | | 4 | 4 | 0 | 0 | 0 |
| | 12.5 | 4.5 | 5 | 5 | 5 | 5 | | | | 5 | 4.5 | 0 | 0 | 0 |
| | 25 | 5 | 5 | 5 | 5 | 5 | | | | 5 | 5 | 0 | 0 | 0 |
| 299 | 12.5 | 4 | 3.5 | 4.5 | 4 | 4.5 | | | | 5 | 5 | 0 | 0 | 0 |
| | 25 | 4.5 | 4 | 5 | 4.5 | 5 | | | | 5 | 5 | 0 | 0 | 0 |
| | 50 | 5 | 5 | 5 | 5 | 5 | | | | 5 | 5 | 0 | 2 | 0 |
| 300 | 12.5 | 4 | 3 | 4 | 4 | 4 | | | | 4 | 4.5 | 0 | 0 | 0 |
| | 25 | 4 | 4 | 4.5 | 4.5 | 5 | | | | 4.5 | 4.5 | 0 | 0 | 0 |
| | 50 | 5 | 5 | 5 | 5 | 5 | | | | 5 | 5 | 1 | 1 | 0 |
| 301 | 6.25 | 4 | 3.5 | 4.5 | 5 | 4.5 | | | | 5 | 4.5 | 0 | 0 | 0 |
| | 12.5 | 4 | 4 | 5 | 5 | 4.5 | | | | 5 | 5 | 0 | 0 | 0 |
| | 25 | 5 | 5 | 5 | 5 | 5 | | | | 5 | 5 | 0 | 0 | 1 |
| | 50 | 5 | 5 | 5 | 5 | 5 | | | | 5 | 5 | 1 | 2 | 2 |
| 308 | 12.5 | 4.5 | 4.5 | 5 | 5 | 5 | | | | 4 | 4.5 | 0 | 0 | 0 |
| | 25 | 5 | 4.5 | 5 | 5 | 5 | | | | 4.5 | 5 | 0 | 0 | 1 |
| | 50 | 5 | 5 | 5 | 5 | 5 | | | | 5 | 5 | 1 | 0 | 2 |

Table 2:

| Compound No. | Dose g/a | Herbicidal effect rating | | | | | | | | | | Phytotoxicity rating | | |
|---|---|---|---|---|---|---|---|---|---|---|---|---|---|---|
| | | A | B | C | D | E | F | G | H | I | J | K | L | M |
| 310 | 6.25<br>12.5<br>25<br>50 | 5<br>5<br>5<br>5 | 4.5<br>5<br>5<br>5 | 5<br>5<br>5<br>5 | 5<br>5<br>5<br>5 | 5<br>5<br>5<br>5 | | | 3<br>4.5<br>5<br>5 | 4<br>4.5<br>5<br>5 | 4.5<br>5<br>5<br>5 | 0<br>0<br>1<br>2 | 0<br>0<br>1<br>2 | 0<br>0<br>1<br>1 |
| 316 | 6.25<br>12.5<br>25<br>50 | 4.5<br>5<br>5<br>5 | 4<br>4.5<br>5<br>5 | 4.5<br>5<br>5<br>5 | 5<br>5<br>5<br>5 | 4.5<br>5<br>5<br>5 | | | 4<br>4<br>5<br>5 | 4<br>4.5<br>5<br>5 | 4.5<br>4.5<br>5<br>5 | 0<br>0<br>0<br>1 | 0<br>0<br>1<br>2 | 0<br>0<br>1<br>1 |
| 318 | 12.5<br>25<br>50 | 5<br>5<br>5 | 5<br>5<br>5 | 5<br>5<br>5 | 5<br>5<br>5 | 5<br>5<br>5 | | | | 4.5<br>4.5<br>5 | 4.5<br>5<br>5 | 0<br>0<br>1 | 0<br>1<br>2 | 0<br>0<br>1 |
| 322 | 12.5<br>25<br>50 | 4.5<br>5<br>5 | 4<br>5<br>5 | 5<br>5<br>5 | 5<br>5<br>5 | 5<br>5<br>5 | | | | 4<br>4<br>5 | 4.5<br>5<br>5 | 0<br>0<br>1 | 0<br>1<br>2 | 0<br>0<br>2 |
| 327 | 6.25<br>12.5<br>25<br>50 | 4<br>4<br>5<br>5 | 3<br>4<br>5<br>5 | 4.5<br>5<br>5<br>5 | 5<br>5<br>5<br>5 | 4.5<br>4.5<br>5<br>5 | | | | 4<br>4<br>4.5<br>5 | 4.5<br>5<br>5<br>5 | 0<br>0<br>0<br>0 | 0<br>0<br>1<br>1 | 0<br>0<br>0<br>1 |
| 328 | 6.25<br>12.5<br>25<br>50 | 4<br>4.5<br>5<br>5 | 4<br>4.5<br>5<br>5 | 5<br>5<br>5<br>5 | 4.5<br>5<br>5<br>5 | 5<br>5<br>5<br>5 | | | | 4<br>4<br>5<br>5 | 4.5<br>5<br>5<br>5 | 0<br>0<br>1<br>2 | 0<br>0<br>0<br>1 | 0<br>0<br>0<br>1 |
| 329 | 6.25<br>12.5<br>25<br>50 | 4<br>4<br>5<br>5 | 4<br>4<br>5<br>5 | 5<br>5<br>5<br>5 | 5<br>5<br>5<br>5 | 5<br>5<br>5<br>5 | | | | 4<br>4<br>5<br>5 | 4.5<br>5<br>5<br>5 | 0<br>0<br>0<br>1 | 0<br>0<br>0<br>0 | 0<br>0<br>0<br>2 |
| 334 | 6.25<br>12.5<br>25<br>50 | 4.5<br>5<br>5<br>5 | 5<br>5<br>5<br>5 | 5<br>5<br>5<br>5 | 5<br>5<br>5<br>5 | 5<br>5<br>5<br>5 | | | | 4<br>4.5<br>5<br>5 | 5<br>5<br>5<br>5 | 0<br>0<br>1<br>2 | 0<br>0<br>0<br>1 | 0<br>0<br>2<br>2 |

Table 2:

| Compound No. | Dose g/a | Herbicidal effect rating | | | | | | | | | | Phytotoxicity rating | | |
|---|---|---|---|---|---|---|---|---|---|---|---|---|---|---|
| | | A | B | C | D | E | F | G | H | I | J | K | L | M |
| 335 | 12.5 | 4 | 4.5 | 5 | 5 | 5 | | | | 4 | 4.5 | 0 | 0 | 0 |
| | 25 | 5 | 4.5 | 5 | 5 | 5 | | | | 5 | 5 | 0 | 0 | 0 |
| | 50 | 5 | 5 | 5 | 5 | 5 | | | | 5 | 5 | 0 | 0 | 0 |
| 342 | 6.25 | 4 | 3.5 | 4.5 | 5 | 4.5 | | | | 4 | 4 | 0 | 0 | 0 |
| | 12.5 | 4 | 4 | 5 | 5 | 5 | | | | 4 | 4.5 | 0 | 0 | 0 |
| | 25 | 5 | 4.5 | 5 | 5 | 5 | | | | 5 | 5 | 0 | 0 | 0 |
| | 50 | 5 | 5 | 5 | 5 | 5 | | | | 5 | 5 | 0 | 0 | 0 |
| 343 | 3.125 | 4.5 | 4 | 5 | 4.5 | 5 | | | | 4.5 | 5 | 0 | 0 | 0 |
| | 6.25 | 5 | 5 | 5 | 5 | 5 | | | | 5 | 5 | 0 | 0 | 0 |
| | 12.5 | 5 | 5 | 5 | 5 | 5 | | | | 5 | 5 | 0 | 0 | 0 |
| | 25 | 5 | 5 | 5 | 5 | 5 | | | | 5 | 5 | 0 | 0 | 0 |
| 344 | 3.125 | 4 | 4 | 5 | 4.5 | 4.5 | | | | 4 | 4.5 | 0 | 0 | 0 |
| | 6.25 | 5 | 5 | 5 | 5 | 5 | | | | 4.5 | 5 | 0 | 0 | 0 |
| | 12.5 | 5 | 5 | 5 | 5 | 5 | | | | 5 | 5 | 0 | 0 | 0 |
| | 25 | 5 | 5 | 5 | 5 | 5 | | | | 5 | 5 | 0 | 0 | 1 |
| 350 | 3.125 | 4.5 | 4 | 5 | 5 | 5 | 3 | 4.5 | 4.5 | 4 | 5 | 0 | 0 | 0 |
| | 6.25 | 5 | 5 | 5 | 5 | 5 | 4 | 4.5 | 5 | 4 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 1 |
| 351 | 3.125 | 5 | 5 | 5 | 5 | 5 | 4.5 | 4.5 | 5 | 4.5 | 5 | 0 | 0 | 0 |
| | 6.25 | 5 | 5 | 5 | 5 | 5 | 4.5 | 5 | 5 | 5 | 5 | 0 | 0 | 0 |
| | 12.5 | 5 | 5 | 5 | 5 | 5 | 5 | 5 | 5 | 5 | 5 | 1 | 0 | 0 |
| | 25 | 5 | 5 | 5 | 5 | 5 | 5 | 5 | 5 | 5 | 5 | 1 | 1 | 0 |
| 352 | 3.125 | 5 | 5 | 5 | 5 | 5 | | | 5 | 4 | 4.5 | 0 | 0 | 0 |
| | 6.25 | 5 | 5 | 5 | 5 | 5 | | | 5 | 5 | 5 | 0 | 0 | 0 |
| | 12.5 | 5 | 5 | 5 | 5 | 5 | | | 5 | 5 | 5 | 0 | 0 | 0 |
| | 25 | 5 | 5 | 5 | 5 | 5 | | | 5 | 5 | 5 | 1 | 1 | 1 |
| 353 | 3.125 | 5 | 5 | 5 | 5 | 5 | 4.5 | 5 | 5 | 4 | 4.5 | 0 | 0 | 0 |
| | 6.25 | 5 | 5 | 5 | 5 | 5 | 5 | 5 | 5 | 5 | 5 | 0 | 0 | 0 |
| | 12.5 | 5 | 5 | 5 | 5 | 5 | 5 | 5 | 5 | 5 | 5 | 1 | 0 | 0 |
| | 25 | 5 | 5 | 5 | 5 | 5 | 5 | 5 | 5 | 5 | 5 | 2 | 0 | 0 |

Table 2:

| Compound No. | Dose g/a | Herbicidal effect rating A | B | C | D | E | F | G | H | I | J | Phytotoxicity rating K | L | M |
|---|---|---|---|---|---|---|---|---|---|---|---|---|---|---|
| 354 | 3.125 | 5 | 5 | 5 | 5 | 5 | 5 | 5 | 5 | 4.5 | 5 | 0 | 0 | 0 |
| | 6.25 | 5 | 5 | 5 | 5 | 5 | 5 | 5 | 5 | 5 | 5 | 0 | 0 | 0 |
| | 12.5 | 5 | 5 | 5 | 5 | 5 | 5 | 5 | 5 | 5 | 5 | 0 | 0 | 0 |
| | 25 | 5 | 5 | 5 | 5 | 5 | 5 | 5 | 5 | 5 | 5 | 0 | 0 | 2 |
| 357 | 3.125 | 5 | 4.5 | 5 | 5 | 5 | | | | 4 | 4.5 | 0 | 0 | 0 |
| | 6.25 | 5 | 5 | 5 | 5 | 5 | | | | 4.5 | 5 | 0 | 0 | 0 |
| | 12.5 | 5 | 5 | 5 | 5 | 5 | | | | 5 | 5 | 1 | 0 | 1 |
| | 25 | 5 | 5 | 5 | 5 | 5 | | | | 5 | 5 | 1 | 0 | 2 |
| 358 | 3.125 | 5 | 5 | 5 | 5 | 5 | 4.5 | 5 | 5 | 4.5 | 5 | 0 | 0 | 0 |
| | 6.25 | 5 | 5 | 5 | 5 | 5 | 5 | 5 | 5 | 5 | 5 | 0 | 0 | 0 |
| | 12.5 | 5 | 5 | 5 | 5 | 5 | 5 | 5 | 5 | 5 | 5 | 0 | 0 | 1 |
| | 25 | 5 | 5 | 5 | 5 | 5 | 5 | 5 | 5 | 5 | 5 | 1 | 0 | 2 |
| 359 | 6.25 | 5 | 5 | 5 | 5 | 5 | | | 5 | 4.5 | 4.5 | 0 | 0 | 0 |
| | 12.5 | 5 | 5 | 5 | 5 | 5 | | | 5 | 4.5 | 5 | 0 | 0 | 0 |
| | 25 | 5 | 5 | 5 | 5 | 5 | | | 5 | 5 | 5 | 0 | 0 | 0 |
| | 50 | 5 | 5 | 5 | 5 | 5 | | | 5 | 5 | 5 | 1 | 0 | 1 |
| 360 | 6.25 | 5 | 5 | 5 | 5 | 5 | | | | 4.5 | 4.5 | 0 | 0 | 0 |
| | 12.5 | 5 | 5 | 5 | 5 | 5 | | | | 4.5 | 5 | 0 | 0 | 0 |
| | 25 | 5 | 5 | 5 | 5 | 5 | | | | 5 | 5 | 0 | 0 | 0 |
| | 50 | 5 | 5 | 5 | 5 | 5 | | | | 5 | 5 | 1 | 0 | 1 |
| 361 | 6.25 | 5 | 4.5 | 4 | 5 | 4.5 | | | | 4 | 4.5 | 0 | 0 | 0 |
| | 12.5 | 5 | 5 | 5 | 5 | 5 | | | | 4.5 | 5 | 0 | 0 | 0 |
| | 25 | 5 | 5 | 5 | 5 | 5 | | | | 5 | 5 | 0 | 0 | 0 |
| | 50 | 5 | 5 | 5 | 5 | 5 | | | | 5 | 5 | 1 | 1 | 1 |
| 362 | 12.5 | 2 | 2 | 4 | 4.5 | 5 | | | | 3 | 4 | 0 | 0 | 0 |
| | 25 | 3 | 3 | 5 | 5 | 5 | | | | 4 | 5 | 0 | 0 | 0 |
| | 50 | 5 | 5 | 5 | 5 | 5 | | | | 5 | 5 | 0 | 0 | 0 |

Test 2:

Foliage treatment test:

Each polyethylene pot was filled with back volcano ash soil and manured and seeds of Sawa millet, Large Crabgrass and Radish, Smartweed, wheat, corn and rice were respectively sown in each pot. The pot was kept in a greenhouse. When Sawa millet was grown to 2 leaf stage, Large crabgrass to 3 leaf stage and Radish to 1 leaf stage, Smartweed to 1.5 leaf stage, wheat to 2 leaf stage, corn to 2.5 leaf stage, rice to 3 leaf stage, each solution prepared by diluting each emulsifiable concentrate containing each of Compounds of the present invention and Reference Compounds, at a concentration of 0.0625, 0.125 or 0.25% was sprayed at a rate of 10 liters per are by a small power pressurized sprayer and the pot in the greenhouse was observed.

Fifteen days after the treatment, the herbicidal effects and phytotoxicity were observed. The results are shown in Table 3.

The herbicidal effects and the phytotoxicities were rated in the same manner as in Test 1.

In Table 3, the following symbols are used to identify the weeds and crop plants:

O: Sawa millet (Echinochloa frumentacea)

A: Large crabgrass (Digitaria sanguinalis)

P: Radish (Raphanus raphanistrum)

D: Smartweed (Polygonum blumei)

K: Corn (Zea mays)

L: Wheat (Triticum aestivum)

N: Rice (Oryza sativa)

Table 3:

| Compound No. | Concentration (%) | Herbicidal effect rating | | | | Phytotoxicity rating | | |
|---|---|---|---|---|---|---|---|---|
| | | O | A | P | D | K | L | N |
| 1 | 0.0625<br>0.125<br>0.25 | 2<br>3<br>4 | 2<br>3<br>4 | 5<br>5<br>5 | 5<br>5<br>5 | 0<br>0<br>2 | 0<br>0<br>1 | 0<br>0<br>1 |
| 2 | 0.0625<br>0.125<br>0.25 | 3<br>3<br>4 | 2<br>3<br>3 | 5<br>5<br>5 | 5<br>5<br>5 | 0<br>0<br>0 | 0<br>0<br>0 | 0<br>0<br>0 |
| 4 | 0.0625<br>0.125<br>0.25 | 2<br>3<br>4 | 3<br>3<br>4 | 5<br>5<br>5 | 5<br>5<br>5 | 0<br>0<br>0 | 0<br>0<br>0 | 0<br>0<br>0 |
| 8 | 0.0625<br>0.125<br>0.25 | 3<br>4<br>4 | 2<br>2<br>3 | 4<br>5<br>5 | 5<br>5<br>5 | 0<br>0<br>1 | 0<br>0<br>2 | 0<br>0<br>1 |
| 10 | 0.0625<br>0.125<br>0.25 | 1<br>2<br>2 | 2<br>3<br>3 | 3<br>4<br>5 | 4<br>4<br>5 | 0<br>0<br>0 | 0<br>0<br>0 | 0<br>0<br>1 |
| 11 | 0.0625<br>0.125<br>0.25 | 4<br>5<br>5 | 5<br>5<br>5 | 5<br>5<br>5 | 5<br>5<br>5 | 0<br>0<br>1 | 0<br>0<br>2 | 0<br>0<br>2 |
| 14 | 0.0625<br>0.125<br>0.25 | 3<br>4<br>4 | 2<br>3<br>3 | 5<br>5<br>5 | 4<br>5<br>5 | 0<br>0<br>0 | 0<br>0<br>0 | 0<br>0<br>0 |
| 17 | 0.0625<br>0.125<br>0.25 | 5<br>5<br>5 | 5<br>5<br>5 | 5<br>5<br>5 | 5<br>5<br>5 | 0<br>1<br>1 | 0<br>0<br>2 | 0<br>0<br>1 |
| 20 | 0.0625<br>0.125<br>0.25 | 3<br>4<br>5 | 4<br>4<br>5 | 5<br>5<br>5 | 5<br>5<br>5 | 0<br>0<br>1 | 0<br>0<br>0 | 0<br>0<br>0 |
| 23 | 0.0625<br>0.125<br>0.25 | 4<br>5<br>5 | 4<br>5<br>5 | 5<br>5<br>5 | 5<br>5<br>5 | 0<br>1<br>1 | 0<br>0<br>2 | 0<br>0<br>1 |

Table 3:

| Compound No. | Concentration (%) | Herbicidal effect rating | | | | Phytotoxicity rating | | |
|---|---|---|---|---|---|---|---|---|
| | | O | A | P | D | K | L | N |
| 26 | 0.0625<br>0.125<br>0.25 | 4<br>4<br>5 | 4<br>4<br>5 | 5<br>5<br>5 | 5<br>5<br>5 | 0<br>0<br>1 | 0<br>0<br>0 | 0<br>0<br>1 |
| 29 | 0.0625<br>0.125<br>0.25 | 5<br>5<br>5 | 5<br>5<br>5 | 5<br>5<br>5 | 5<br>5<br>5 | 0<br>0<br>1 | 0<br>0<br>2 | 0<br>0<br>0 |
| 32 | 0.0625<br>0.125<br>0.25 | 3<br>4<br>5 | 4<br>5<br>5 | 5<br>5<br>5 | 4<br>5<br>5 | 0<br>0<br>0 | 0<br>0<br>0 | 0<br>0<br>0 |
| 36 | 0.0625<br>0.125<br>0.25 | 2<br>5<br>5 | 3<br>5<br>5 | 5<br>5<br>5 | 5<br>5<br>5 | 0<br>0<br>0 | 0<br>0<br>0 | 0<br>0<br>0 |
| 38 | 0.0625<br>0.125<br>0.25 | 2<br>5<br>5 | 2<br>4<br>5 | 4<br>5<br>5 | 3<br>4<br>5 | 0<br>0<br>0 | 0<br>0<br>0 | 0<br>0<br>0 |
| 40 | 0.0625<br>0.125<br>0.25 | 0<br>1<br>1 | 3<br>4<br>5 | 2<br>2<br>3 | 4<br>5<br>5 | 0<br>0<br>0 | 0<br>0<br>0 | 0<br>0<br>0 |
| 44 | 0.0625<br>0.125<br>0.25 | 2<br>3<br>3 | 4<br>5<br>5 | 5<br>5<br>5 | 5<br>5<br>5 | 0<br>0<br>0 | 0<br>0<br>0 | 0<br>0<br>0 |
| 45 | 0.0625<br>0.125<br>0.25 | 2<br>3<br>3 | 4<br>4<br>5 | 5<br>5<br>5 | 4<br>5<br>5 | 0<br>0<br>0 | 0<br>0<br>0 | 0<br>0<br>0 |
| 51 | 0.0625<br>0.125<br>0.25 | 4<br>5<br>5 | 5<br>5<br>5 | 4<br>5<br>5 | 4<br>4<br>5 | 0<br>1<br>1 | 0<br>0<br>2 | 0<br>0<br>1 |
| 54 | 0.0625<br>0.125<br>0.25 | 3<br>4<br>4 | 4<br>5<br>5 | 5<br>5<br>5 | 4<br>5<br>5 | 0<br>0<br>0 | 0<br>0<br>0 | 0<br>0<br>0 |

Table 3:

| Compound No. | Concentration (%) | Herbicidal effect rating | | | | Phytotoxicity rating | | |
|---|---|---|---|---|---|---|---|---|
| | | O | A | P | D | K | L | N |
| 55 | 0.0625 | 4 | 4 | 5 | 4 | 0 | 0 | 0 |
| | 0.125 | 5 | 5 | 5 | 5 | 1 | 0 | 0 |
| | 0.25 | 5 | 5 | 5 | 5 | 1 | 2 | 1 |
| 56 | 0.0625 | 3 | 4 | 5 | 4 | 0 | 0 | 0 |
| | 0.125 | 4 | 4 | 5 | 5 | 0 | 0 | 0 |
| | 0.25 | 4 | 5 | 5 | 5 | 0 | 0 | 0 |
| 58 | 0.0625 | 3 | 4 | 5 | 4 | 0 | 0 | 0 |
| | 0.125 | 4 | 5 | 5 | 5 | 0 | 0 | 0 |
| | 0.25 | 4 | 5 | 5 | 5 | 0 | 0 | 0 |
| 63 | 0.0625 | 3 | 4 | 5 | 4 | 0 | 0 | 0 |
| | 0.125 | 3 | 4 | 5 | 5 | 0 | 0 | 0 |
| | 0.25 | 4 | 5 | 5 | 5 | 0 | 0 | 0 |
| 68 | 0.0625 | 4 | 4 | 5 | 5 | 0 | 0 | 0 |
| | 0.125 | 4 | 5 | 5 | 5 | 0 | 0 | 0 |
| | 0.25 | 4 | 5 | 5 | 5 | 0 | 0 | 0 |
| 74 | 0.0625 | 2 | 2 | 4 | 4 | 0 | 0 | 0 |
| | 0.125 | 3 | 3 | 5 | 5 | 0 | 0 | 0 |
| | 0.25 | 3 | 5 | 5 | 5 | 0 | 0 | 0 |
| 77 | 0.0625 | 1 | 1 | 3 | 2 | 0 | 0 | 0 |
| | 0.125 | 4 | 2 | 4 | 3 | 0 | 0 | 0 |
| | 0.25 | 5 | 4 | 5 | 5 | 0 | 0 | 0 |
| 78 | 0.0625 | 2 | 2 | 4 | 3 | 0 | 0 | 0 |
| | 0.125 | 4 | 3 | 4 | 3 | 0 | 0 | 0 |
| | 0.25 | 5 | 5 | 5 | 5 | 1 | 2 | 1 |
| 85 | 0.0625 | 3 | 2 | 5 | 3 | 0 | 0 | 0 |
| | 0.125 | 4 | 4 | 5 | 4 | 0 | 0 | 0 |
| | 0.25 | 4 | 5 | 5 | 5 | 0 | 0 | 0 |
| 86 | 0.0625 | 2 | 3 | 5 | 4 | 0 | 0 | 0 |
| | 0.125 | 3 | 4 | 5 | 5 | 0 | 0 | 0 |
| | 0.25 | 3 | 5 | 5 | 5 | 0 | 0 | 0 |

Table 3:

| Compound No. | Concentration (%) | Herbicidal effect rating | | | | Phytotoxicity rating | | |
|---|---|---|---|---|---|---|---|---|
| | | O | A | P | D | K | L | N |
| 87 | 0.0625<br>0.125<br>0.25 | 1<br>1<br>1 | 3<br>4<br>5 | 3<br>4<br>5 | 4<br>4<br>5 | 0<br>0<br>0 | 0<br>0<br>0 | 0<br>0<br>0 |
| 89 | 0.0625<br>0.125<br>0.25 | 4<br>5<br>5 | 5<br>5<br>5 | 5<br>5<br>5 | 5<br>5<br>5 | 0<br>0<br>0 | 0<br>0<br>0 | 0<br>0<br>1 |
| 90 | 0.0625<br>0.125<br>0.25 | 2<br>3<br>3 | 3<br>4<br>5 | 5<br>5<br>5 | 4<br>5<br>5 | 0<br>0<br>0 | 0<br>0<br>0 | 0<br>0<br>0 |
| 93 | 0.0625<br>0.125<br>0.25 | 3<br>4<br>5 | 4<br>4<br>5 | 5<br>5<br>5 | 5<br>5<br>5 | 0<br>0<br>1 | 0<br>0<br>0 | 0<br>0<br>0 |
| 96 | 0.0625<br>0.125<br>0.25 | 3<br>4<br>4 | 3<br>4<br>5 | 5<br>5<br>5 | 5<br>5<br>5 | 0<br>0<br>0 | 0<br>0<br>0 | 0<br>0<br>0 |
| 98 | 0.0625<br>0.125<br>0.25 | 3<br>4<br>5 | 4<br>5<br>5 | 5<br>5<br>5 | 5<br>5<br>5 | 0<br>0<br>2 | 0<br>0<br>0 | 0<br>0<br>0 |
| 99 | 0.0625<br>0.125<br>0.25 | 4<br>4<br>5 | 4<br>5<br>5 | 5<br>5<br>5 | 5<br>5<br>5 | 0<br>0<br>1 | 0<br>0<br>2 | 0<br>0<br>2 |
| 104 | 0.0625<br>0.125<br>0.25 | 3<br>4<br>4 | 3<br>3<br>3 | 5<br>5<br>5 | 5<br>5<br>5 | 0<br>0<br>0 | 0<br>0<br>0 | 0<br>0<br>0 |
| 106 | 0.0625<br>0.125<br>0.25 | 5<br>5<br>5 | 5<br>5<br>5 | 5<br>5<br>5 | 5<br>5<br>5 | 0<br>0<br>0 | 0<br>0<br>1 | 0<br>0<br>0 |
| 108 | 0.0625<br>0.125<br>0.25 | 4<br>5<br>5 | 4<br>5<br>5 | 5<br>5<br>5 | 4<br>5<br>5 | 0<br>0<br>1 | 0<br>0<br>0 | 0<br>0<br>1 |

Table 3:

| Compound No. | Concentration (%) | Herbicidal effect rating | | | | Phytotoxicity rating | | |
|---|---|---|---|---|---|---|---|---|
| | | O | A | P | D | K | L | N |
| 109 | 0.0625 | 3 | 5 | 5 | 5 | 0 | 0 | 0 |
| | 0.125 | 4 | 5 | 5 | 5 | 0 | 0 | 0 |
| | 0.25 | 4 | 5 | 5 | 5 | 0 | 0 | 0 |
| 110 | 0.0625 | 2 | 3 | 4 | 4 | 0 | 0 | 0 |
| | 0.125 | 3 | 4 | 5 | 4 | 0 | 0 | 0 |
| | 0.25 | 3 | 4 | 5 | 5 | 0 | 0 | 0 |
| 112 | 0.0625 | 5 | 4 | 5 | 5 | 0 | 0 | 0 |
| | 0.125 | 5 | 5 | 5 | 5 | 0 | 0 | 0 |
| | 0.25 | 5 | 5 | 5 | 5 | 1 | 1 | 0 |
| 115 | 0.0625 | 3 | 4 | 5 | 4 | 0 | 0 | 0 |
| | 0.125 | 4 | 4 | 5 | 5 | 0 | 0 | 0 |
| | 0.25 | 5 | 5 | 5 | 5 | 0 | 0 | 0 |
| 118 | 0.0625 | 5 | 5 | 5 | 5 | 0 | 0 | 0 |
| | 0.125 | 5 | 5 | 5 | 5 | 0 | 0 | 0 |
| | 0.25 | 5 | 5 | 5 | 5 | 0 | 1 | 1 |
| 120 | 0.0625 | 2 | 2 | 5 | 5 | 0 | 0 | 0 |
| | 0.125 | 3 | 2 | 5 | 5 | 0 | 0 | 0 |
| | 0.25 | 3 | 3 | 5 | 5 | 0 | 0 | 0 |
| 122 | 0.0625 | 3 | 4 | 5 | 4 | 0 | 0 | 0 |
| | 0.125 | 4 | 4 | 5 | 5 | 0 | 0 | 0 |
| | 0.25 | 5 | 5 | 5 | 5 | 1 | 2 | 2 |
| 124 | 0.0625 | 5 | 4 | 4 | 5 | 0 | 0 | 0 |
| | 0.125 | 5 | 5 | 5 | 5 | 0 | 0 | 0 |
| | 0.25 | 5 | 5 | 5 | 5 | 1 | 0 | 0 |
| 125 | 0.0625 | 5 | 4 | 5 | 5 | 0 | 0 | 0 |
| | 0.125 | 5 | 5 | 5 | 5 | 0 | 0 | 0 |
| | 0.25 | 5 | 5 | 5 | 5 | 1 | 1 | 0 |
| 126 | 0.0625 | 3 | 2 | 5 | 5 | 0 | 0 | 0 |
| | 0.125 | 5 | 4 | 5 | 5 | 0 | 0 | 0 |
| | 0.25 | 5 | 5 | 5 | 5 | 1 | 1 | 0 |

Table 3:

| Compound No. | Concentration (%) | Herbicidal effect rating | | | | Phytotoxicity rating | | |
|---|---|---|---|---|---|---|---|---|
| | | O | A | P | D | K | L | N |
| 127 | 0.0625 | 2 | 2 | 5 | 5 | 0 | 0 | 0 |
| | 0.125 | 4 | 3 | 5 | 5 | 0 | 0 | 0 |
| | 0.25 | 4 | 4 | 5 | 5 | 0 | 0 | 0 |
| 129 | 0.0625 | 5 | 4 | 5 | 5 | 0 | 0 | 0 |
| | 0.125 | 5 | 5 | 5 | 5 | 0 | 0 | 0 |
| | 0.25 | 5 | 5 | 5 | 5 | 2 | 1 | 0 |
| 130 | 0.0625 | 4 | 5 | 5 | 4 | 0 | 0 | 0 |
| | 0.125 | 5 | 5 | 5 | 5 | 0 | 1 | 0 |
| | 0.25 | 5 | 5 | 5 | 5 | 1 | 1 | 0 |
| 133 | 0.0625 | 4 | 4 | 4 | 4 | 0 | 0 | 0 |
| | 0.125 | 4 | 5 | 5 | 5 | 0 | 0 | 0 |
| | 0.25 | 5 | 5 | 5 | 5 | 1 | 0 | 0 |
| 134 | 0.0625 | 2 | 3 | 3 | 4 | 0 | 0 | 0 |
| | 0.125 | 3 | 4 | 4 | 4 | 0 | 0 | 0 |
| | 0.25 | 3 | 5 | 5 | 5 | 1 | 0 | 1 |
| 136 | 0.0625 | 3 | 4 | 4 | 4 | 0 | 0 | 0 |
| | 0.125 | 4 | 4 | 4 | 5 | 0 | 0 | 0 |
| | 0.25 | 4 | 5 | 5 | 5 | 0 | 0 | 0 |
| 146 | 0.0625 | 3 | 4 | 4 | 4 | 0 | 0 | 0 |
| | 0.125 | 3 | 4 | 5 | 4 | 0 | 0 | 0 |
| | 0.25 | 4 | 5 | 5 | 5 | 0 | 0 | 0 |
| 152 | 0.0625 | 2 | 3 | 4 | 4 | 0 | 0 | 0 |
| | 0.125 | 3 | 3 | 4 | 4 | 0 | 0 | 0 |
| | 0.25 | 5 | 4 | 5 | 5 | 0 | 0 | 0 |
| 157 | 0.0625 | 3 | 2 | 4 | 4 | 0 | 0 | 0 |
| | 0.125 | 4 | 3 | 5 | 5 | 0 | 0 | 0 |
| | 0.25 | 5 | 4 | 5 | 5 | 0 | 0 | 0 |
| 172 | 0.0625 | 3 | 2 | 4 | 4 | 0 | 0 | 0 |
| | 0.125 | 3 | 3 | 5 | 5 | 0 | 0 | 0 |
| | 0.25 | 4 | 5 | 5 | 5 | 1 | 0 | 0 |

Table 3:

| Compound No. | Concentration (%) | Herbicidal effect rating | | | | Phytotoxicity rating | | |
|---|---|---|---|---|---|---|---|---|
| | | O | A | P | D | K | L | N |
| 174 | 0.0625 | 3 | 3 | 5 | 4 | 0 | 0 | 0 |
| | 0.125 | 4 | 4 | 5 | 5 | 0 | 0 | 0 |
| | 0.25 | 4 | 5 | 5 | 5 | 0 | 0 | 0 |
| 176 | 0.0625 | 5 | 4 | 5 | 5 | 0 | 0 | 0 |
| | 0.125 | 5 | 5 | 5 | 5 | 0 | 0 | 0 |
| | 0.25 | 5 | 5 | 5 | 5 | 1 | 1 | 0 |
| 181 | 0.0625 | 4 | 5 | 5 | 5 | 0 | 0 | 0 |
| | 0.125 | 5 | 5 | 5 | 5 | 0 | 0 | 0 |
| | 0.25 | 5 | 5 | 5 | 5 | 1 | 0 | 0 |
| 183 | 0.0625 | 3 | 4 | 5 | 4 | 0 | 0 | 0 |
| | 0.125 | 4 | 4 | 5 | 5 | 0 | 0 | 0 |
| | 0.25 | 4 | 5 | 5 | 5 | 0 | 0 | 1 |
| 185 | 0.0625 | 5 | 4 | 5 | 5 | 0 | 0 | 0 |
| | 0.125 | 5 | 5 | 5 | 5 | 0 | 0 | 0 |
| | 0.25 | 5 | 5 | 5 | 5 | 1 | 1 | 1 |
| 187 | 0.0625 | 3 | 3 | 5 | 5 | 0 | 0 | 0 |
| | 0.125 | 5 | 3 | 5 | 5 | 0 | 0 | 0 |
| | 0.25 | 5 | 4 | 5 | 5 | 1 | 1 | 2 |
| 190 | 0.0625 | 3 | 4 | 5 | 4 | 0 | 0 | 0 |
| | 0.125 | 4 | 4 | 5 | 5 | 0 | 0 | 0 |
| | 0.25 | 5 | 5 | 5 | 5 | 1 | 0 | 0 |
| 191 | 0.0625 | 4 | 4 | 5 | 4 | 0 | 0 | 0 |
| | 0.125 | 5 | 4 | 5 | 5 | 0 | 0 | 0 |
| | 0.25 | 5 | 5 | 5 | 5 | 1 | 1 | 0 |
| 192 | 0.0625 | 3 | 2 | 5 | 4 | 0 | 0 | 0 |
| | 0.125 | 3 | 4 | 5 | 5 | 0 | 0 | 0 |
| | 0.25 | 5 | 5 | 5 | 5 | 0 | 0 | 0 |
| 196 | 0.0625 | 3 | 4 | 4 | 5 | 0 | 0 | 0 |
| | 0.125 | 4 | 4 | 5 | 5 | 0 | 0 | 0 |
| | 0.25 | 4 | 5 | 5 | 5 | 2 | 1 | 0 |

Table 3:

| Compound No. | Concentration (%) | Herbicidal effect rating | | | | Phytotoxicity rating | | |
|---|---|---|---|---|---|---|---|---|
| | | O | A | P | D | K | L | N |
| 198 | 0.0625<br>0.125<br>0.25 | 3<br>4<br>4 | 2<br>4<br>4 | 5<br>5<br>5 | 4<br>4<br>5 | 0<br>0<br>0 | 0<br>0<br>0 | 0<br>0<br>0 |
| 200 | 0.0625<br>0.125<br>0.25 | 1<br>1<br>2 | 2<br>3<br>4 | 3<br>4<br>5 | 4<br>5<br>5 | 0<br>0<br>0 | 0<br>0<br>0 | 0<br>0<br>0 |
| 203 | 0.0625<br>0.125<br>0.25 | 1<br>2<br>2 | 3<br>4<br>4 | 5<br>5<br>5 | 5<br>5<br>5 | 0<br>0<br>0 | 0<br>0<br>0 | 0<br>0<br>0 |
| 204 | 0.0625<br>0.125<br>0.25 | 3<br>4<br>4 | 4<br>4<br>5 | 5<br>5<br>5 | 5<br>5<br>5 | 0<br>0<br>1 | 0<br>0<br>0 | 0<br>0<br>0 |
| 205 | 0.0625<br>0.125<br>0.25 | 2<br>3<br>3 | 4<br>4<br>5 | 5<br>5<br>5 | 5<br>5<br>5 | 0<br>0<br>0 | 0<br>0<br>0 | 0<br>0<br>0 |
| 207 | 0.0625<br>0.125<br>0.25 | 3<br>4<br>4 | 4<br>5<br>5 | 5<br>5<br>5 | 5<br>5<br>5 | 0<br>0<br>0 | 0<br>0<br>0 | 0<br>0<br>0 |
| 211 | 0.0625<br>0.125<br>0.25 | 3<br>4<br>4 | 4<br>5<br>5 | 5<br>5<br>5 | 5<br>5<br>5 | 0<br>0<br>0 | 0<br>0<br>0 | 0<br>0<br>1 |
| 213 | 0.0625<br>0.125<br>0.25 | 3<br>4<br>4 | 4<br>4<br>5 | 5<br>5<br>5 | 4<br>5<br>5 | 0<br>0<br>1 | 0<br>0<br>0 | 0<br>0<br>0 |
| 215 | 0.0625<br>0.125<br>0.25 | 4<br>4<br>5 | 4<br>5<br>5 | 5<br>5<br>5 | 5<br>5<br>5 | 0<br>0<br>0 | 0<br>0<br>1 | 0<br>0<br>1 |
| 216 | 0.0625<br>0.125<br>0.25 | 3<br>4<br>4 | 3<br>4<br>5 | 4<br>5<br>5 | 5<br>5<br>5 | 0<br>0<br>1 | 0<br>0<br>1 | 0<br>0<br>0 |

Table 3:

| Compound No. | Concentration (%) | Herbicidal effect rating | | | | Phytotoxicity rating | | |
|---|---|---|---|---|---|---|---|---|
| | | O | A | P | D | K | L | N |
| 218 | 0.0625<br>0.125<br>0.25 | 3<br>3<br>4 | 4<br>4<br>5 | 5<br>5<br>5 | 5<br>5<br>5 | 0<br>0<br>0 | 0<br>0<br>0 | 0<br>0<br>2 |
| 220 | 0.0625<br>0.125<br>0.25 | 2<br>2<br>3 | 2<br>3<br>5 | 4<br>4<br>5 | 4<br>5<br>5 | 0<br>0<br>0 | 0<br>0<br>0 | 0<br>0<br>0 |
| 222 | 0.0625<br>0.125<br>0.25 | 3<br>4<br>4 | 2<br>2<br>4 | 5<br>5<br>5 | 4<br>5<br>5 | 0<br>0<br>0 | 0<br>0<br>1 | 0<br>0<br>0 |
| 223 | 0.0625<br>0.125<br>0.25 | 3<br>4<br>4 | 2<br>3<br>3 | 4<br>4<br>5 | 4<br>4<br>5 | 0<br>0<br>0 | 0<br>0<br>0 | 0<br>0<br>0 |
| 224 | 0.0625<br>0.125<br>0.25 | 4<br>5<br>5 | 4<br>5<br>5 | 5<br>5<br>5 | 4<br>5<br>5 | 0<br>0<br>0 | 0<br>0<br>0 | 0<br>0<br>0 |
| 225 | 0.0625<br>0.125<br>0.25 | 1<br>3<br>4 | 2<br>3<br>4 | 4<br>5<br>5 | 4<br>5<br>5 | 0<br>0<br>2 | 0<br>0<br>1 | 0<br>0<br>0 |
| 228 | 0.0625<br>0.125<br>0.25 | 1<br>2<br>2 | 2<br>3<br>4 | 4<br>5<br>5 | 3<br>4<br>5 | 0<br>0<br>2 | 0<br>0<br>1 | 0<br>0<br>0 |
| 234 | 0.0625<br>0.125<br>0.25 | 2<br>4<br>4 | 3<br>4<br>4 | 5<br>5<br>5 | 4<br>5<br>5 | 0<br>0<br>0 | 0<br>0<br>2 | 0<br>0<br>0 |
| 235 | 0.0625<br>0.125<br>0.25 | 1<br>2<br>2 | 2<br>2<br>3 | 4<br>5<br>5 | 3<br>4<br>5 | 0<br>0<br>0 | 0<br>0<br>0 | 0<br>0<br>0 |
| 236 | 0.0625<br>0.125<br>0.25 | 1<br>2<br>4 | 2<br>3<br>4 | 4<br>5<br>5 | 4<br>4<br>5 | 0<br>0<br>0 | 0<br>0<br>0 | 0<br>0<br>1 |

Table 3:

| Compound No. | Concentration (%) | Herbicidal effect rating | | | | Phytotoxicity rating | | |
|---|---|---|---|---|---|---|---|---|
| | | O | A | P | D | K | L | N |
| 238 | 0.0625<br>0.125<br>0.25 | 1<br>1<br>1 | 1<br>1<br>2 | 3<br>5<br>5 | 3<br>4<br>5 | 0<br>0<br>0 | 0<br>0<br>0 | 0<br>0<br>0 |
| 244 | 0.0625<br>0.125<br>0.25 | 2<br>4<br>4 | 1<br>2<br>3 | 4<br>5<br>5 | 3<br>5<br>5 | 0<br>0<br>0 | 0<br>0<br>2 | 0<br>0<br>0 |
| 248 | 0.0625<br>0.125<br>0.25 | 1<br>2<br>2 | 1<br>2<br>3 | 2<br>3<br>4 | 2<br>4<br>4 | 0<br>0<br>0 | 0<br>0<br>0 | 0<br>0<br>0 |
| 250 | 0.0625<br>0.125<br>0.25 | 3<br>4<br>4 | 3<br>4<br>5 | 5<br>5<br>5 | 4<br>5<br>5 | 0<br>0<br>1 | 0<br>0<br>1 | 0<br>0<br>0 |
| 253 | 0.0625<br>0.125<br>0.25 | 1<br>1<br>2 | 1<br>2<br>3 | 4<br>5<br>5 | 4<br>4<br>5 | 0<br>0<br>0 | 0<br>0<br>0 | 0<br>0<br>0 |
| 257 | 0.0625<br>0.125<br>0.25 | 2<br>3<br>5 | 3<br>5<br>5 | 5<br>5<br>5 | 4<br>5<br>5 | 0<br>0<br>1 | 0<br>0<br>0 | 0<br>0<br>1 |
| 268 | 0.0625<br>0.125<br>0.25 | 3<br>4<br>4 | 3<br>4<br>5 | 4<br>5<br>5 | 3<br>4<br>5 | 0<br>0<br>0 | 0<br>0<br>0 | 0<br>0<br>0 |
| 269 | 0.0625<br>0.125<br>0.25 | 1<br>2<br>3 | 1<br>2<br>4 | 4<br>5<br>5 | 5<br>5<br>5 | 0<br>0<br>0 | 0<br>0<br>0 | 0<br>0<br>0 |
| 270 | 0.0625<br>0.125<br>0.25 | 1<br>1<br>2 | 1<br>1<br>2 | 5<br>5<br>5 | 5<br>5<br>5 | 0<br>0<br>0 | 0<br>0<br>0 | 0<br>0<br>0 |
| 273 | 0.0625<br>0.125<br>0.25 | 1<br>1<br>1 | 1<br>1<br>2 | 5<br>5<br>5 | 5<br>5<br>5 | 0<br>0<br>0 | 0<br>0<br>0 | 0<br>0<br>0 |

Table 3:

| Compound No. | Concentration (%) | Herbicidal effect rating | | | | Phytotoxicity rating | | |
|---|---|---|---|---|---|---|---|---|
| | | O | A | P | D | K | L | N |
| 274 | 0.0625<br>0.125<br>0.25 | 3<br>4<br>5 | 3<br>3<br>5 | 4<br>5<br>5 | 4<br>5<br>5 | 0<br>0<br>1 | 0<br>0<br>1 | 0<br>0<br>0 |
| 276 | 0.0625<br>0.125<br>0.25 | 3<br>3<br>4 | 2<br>2<br>3 | 5<br>5<br>5 | 5<br>5<br>5 | 0<br>0<br>0 | 0<br>0<br>0 | 0<br>0<br>0 |
| Reference D | 0.0625<br>0.125<br>0.25 | 1<br>2<br>5 | 2<br>3<br>3 | 3<br>3<br>4 | 1<br>2<br>3 | 4<br>5<br>5 | 3<br>4<br>5 | 0<br>0<br>1 |

Table 3:

| Compound No. | Concentration (%) | Herbicidal effect rating | | | | Phytotoxicity rating | | |
|---|---|---|---|---|---|---|---|---|
| | | O | A | P | D | K | L | N |
| 281 | 0.125<br>0.25<br>0.5 | 3<br>4<br>5 | 3.5<br>4.5<br>5 | 5<br>5<br>5 | 5<br>5<br>5 | 0<br>0<br>1 | 0<br>0<br>2 | 0<br>0<br>1 |
| 284 | 0.0625<br>0.125<br>0.25 | 4<br>5<br>5 | 3.5<br>5<br>5 | 5<br>5<br>5 | 5<br>5<br>5 | 0<br>0<br>0 | 0<br>0<br>1 | 0<br>0<br>2 |
| 286 | 0.125<br>0.25<br>0.5 | 3<br>4<br>5 | 3<br>4<br>4.5 | 5<br>5<br>5 | 4<br>5<br>5 | 0<br>1<br>1 | 0<br>0<br>0 | 0<br>1<br>2 |
| 302 | 0.125<br>0.25<br>0.5 | 4.5<br>5<br>5 | 5<br>5<br>5 | 5<br>5<br>5 | 5<br>5<br>5 | 0<br>0<br>0 | 0<br>1<br>1 | 0<br>1<br>2 |
| 303 | 0.125<br>0.25<br>0.5 | 3<br>3<br>5 | 3<br>4<br>5 | 5<br>5<br>5 | 5<br>5<br>5 | 0<br>1<br>2 | 0<br>0<br>1 | 0<br>1<br>2 |
| 304 | 0.125<br>0.25<br>0.5 | 3<br>3<br>5 | 3<br>3.5<br>5 | 4.5<br>5<br>5 | 5<br>5<br>5 | 0<br>1<br>1 | 0<br>0<br>1 | 0<br>0<br>2 |
| 305 | 0.125<br>0.25<br>0.5 | 4.5<br>5<br>5 | 5<br>5<br>5 | 5<br>5<br>5 | 5<br>5<br>5 | 0<br>1<br>2 | 0<br>1<br>2 | 0<br>0<br>1 |
| 306 | 0.125<br>0.25<br>0.5 | 4.5<br>4.5<br>5 | 4.5<br>5<br>5 | 5<br>5<br>5 | 5<br>5<br>5 | 0<br>0<br>1 | 0<br>1<br>2 | 0<br>0<br>1 |
| 309 | 0.125<br>0.25<br>0.5 | 4.5<br>5<br>5 | 5<br>5<br>5 | 5<br>5<br>5 | 5<br>5<br>5 | 0<br>1<br>2 | 0<br>0<br>1 | 0<br>0<br>1 |
| 311 | 0.125<br>0.25<br>0.5 | 5<br>5<br>5 | 5<br>5<br>5 | 5<br>5<br>5 | 5<br>5<br>5 | 0<br>0<br>1 | 0<br>1<br>2 | 0<br>1<br>2 |

Table 3:

| Compound No. | Concentration (%) | Herbicidal effect rating | | | | Phytotoxicity rating | | |
|---|---|---|---|---|---|---|---|---|
| | | O | A | P | D | K | L | N |
| 314 | 0.0625<br>0.125<br>0.25 | 4.5<br>5<br>5 | 5<br>5<br>5 | 5<br>5<br>5 | 5<br>5<br>5 | 0<br>1<br>2 | 0<br>0<br>1 | 0<br>0<br>1 |
| 315 | 0.125<br>0.25<br>0.5 | 3<br>4<br>5 | 4<br>4.5<br>5 | 5<br>5<br>5 | 5<br>5<br>5 | 0<br>0<br>1 | 0<br>1<br>1 | 0<br>0<br>1 |
| 317 | 0.0625<br>0.125<br>0.25 | 5<br>5<br>5 | 5<br>5<br>5 | 5<br>5<br>5 | 5<br>5<br>5 | 0<br>1<br>2 | 0<br>0<br>1 | 0<br>1<br>2 |
| 319 | 0.125<br>0.25<br>0.5 | 4<br>4<br>5 | 3.5<br>4<br>5 | 5<br>5<br>5 | 5<br>5<br>5 | 0<br>0<br>0 | 0<br>0<br>0 | 0<br>0<br>1 |
| 320 | 0.125<br>0.25<br>0.5 | 4<br>4<br>5 | 4<br>4<br>5 | 5<br>5<br>5 | 5<br>5<br>5 | 0<br>0<br>0 | 0<br>0<br>0 | 0<br>0<br>0 |
| 321 | 0.125<br>0.25<br>0.5 | 4<br>4.5<br>5 | 4.5<br>5<br>5 | 5<br>5<br>5 | 5<br>5<br>5 | 0<br>0<br>2 | 0<br>0<br>1 | 0<br>0<br>2 |
| 323 | 0.125<br>0.25<br>0.5 | 3<br>4<br>5 | 4<br>4.5<br>5 | 5<br>5<br>5 | 5<br>5<br>5 | 0<br>0<br>1 | 0<br>0<br>0 | 0<br>0<br>2 |
| 326 | 0.0625<br>0.125<br>0.25 | 5<br>5<br>5 | 5<br>5<br>5 | 5<br>5<br>5 | 5<br>5<br>5 | 0<br>1<br>2 | 0<br>1<br>2 | 0<br>0<br>2 |
| 330 | 0.125<br>0.25<br>0.5 | 3<br>4<br>5 | 3<br>4.5<br>5 | 5<br>5<br>5 | 5<br>5<br>5 | 0<br>0<br>0 | 0<br>0<br>1 | 0<br>0<br>0 |
| 331 | 0.0625<br>0.125<br>0.25 | 4<br>4.5<br>5 | 4.5<br>5<br>5 | 5<br>5<br>5 | 5<br>5<br>5 | 0<br>0<br>1 | 0<br>1<br>2 | 0<br>0<br>1 |

Table 3:

| Compound No. | Concentration (%) | Herbicidal effect rating | | | | Phytotoxicity rating | | |
|---|---|---|---|---|---|---|---|---|
| | | O | A | P | D | K | L | N |
| 332 | 0.0625<br>0.125<br>0.25 | 4.5<br>5<br>5 | 5<br>5<br>5 | 5<br>5<br>5 | 5<br>5<br>5 | 0<br>0<br>1 | 0<br>0<br>0 | 0<br>0<br>2 |
| 336 | 0.125<br>0.25<br>0.5 | 4.5<br>5<br>5 | 5<br>5<br>5 | 5<br>5<br>5 | 5<br>5<br>5 | 0<br>1<br>1 | 0<br>0<br>0 | 0<br>0<br>1 |
| 337 | 0.0625<br>0.125<br>0.25 | 4.5<br>5<br>5 | 5<br>5<br>5 | 5<br>5<br>5 | 5<br>5<br>5 | 0<br>0<br>1 | 0<br>0<br>0 | 0<br>0<br>0 |
| 338 | 0.125<br>0.25<br>0.5 | 5<br>5<br>5 | 5<br>5<br>5 | 5<br>5<br>5 | 5<br>5<br>5 | 0<br>0<br>0 | 0<br>0<br>0 | 0<br>0<br>1 |
| 340 | 0.0625<br>0.125<br>0.25 | 4<br>5<br>5 | 4.5<br>5<br>5 | 5<br>5<br>5 | 5<br>5<br>5 | 0<br>0<br>1 | 0<br>0<br>2 | 0<br>0<br>0 |
| 341 | 0.0625<br>0.125<br>0.25 | 4.5<br>5<br>5 | 5<br>5<br>5 | 5<br>5<br>5 | 5<br>5<br>5 | 0<br>0<br>1 | 0<br>0<br>2 | 0<br>0<br>0 |
| 345 | 0.125<br>0.25<br>0.5 | 4<br>4.5<br>5 | 4.5<br>5<br>5 | 5<br>5<br>5 | 5<br>5<br>5 | 0<br>0<br>2 | 0<br>0<br>0 | 0<br>0<br>0 |
| 346 | 0.0625<br>0.125<br>0.25 | 4.5<br>4.5<br>5 | 4.5<br>5<br>5 | 5<br>5<br>5 | 5<br>5<br>5 | 0<br>0<br>0 | 0<br>0<br>0 | 0<br>0<br>1 |
| 347 | 0.0625<br>0.125<br>0.25 | 5<br>5<br>5 | 5<br>5<br>5 | 5<br>5<br>5 | 5<br>5<br>5 | 0<br>1<br>1 | 0<br>0<br>0 | 0<br>1<br>2 |
| 349 | 0.0625<br>0.125<br>0.25 | 4.5<br>5<br>5 | 5<br>5<br>5 | 5<br>5<br>5 | 5<br>5<br>5 | 0<br>1<br>2 | 0<br>0<br>2 | 0<br>0<br>0 |

Table 3:

| Compound No. | Concentration (%) | Herbicidal effect rating | | | | Phytotoxicity rating | | |
|---|---|---|---|---|---|---|---|---|
| | | O | A | P | D | K | L | N |
| 355 | 0.125<br>0.25<br>0.5 | 4<br>5<br>5 | 4.5<br>5<br>5 | 5<br>5<br>5 | 5<br>5<br>5 | 0<br>0<br>0 | 0<br>0<br>1 | 0<br>0<br>0 |
| 356 | 0.0625<br>0.125<br>0.25 | 4.5<br>5<br>5 | 5<br>5<br>5 | 5<br>5<br>5 | 5<br>5<br>5 | 0<br>0<br>0 | 0<br>0<br>0 | 0<br>0<br>1 |
| 363 | 0.0625<br>0.125<br>0.25 | 3<br>4<br>5 | 3<br>4<br>5 | 5<br>5<br>5 | 5<br>5<br>5 | 0<br>0<br>1 | 0<br>0<br>0 | 0<br>0<br>2 |
| 364 | 0.0625<br>0.125<br>0.25 | 3<br>4<br>5 | 4<br>4<br>5 | 5<br>5<br>5 | 5<br>5<br>5 | 0<br>0<br>1 | 0<br>0<br>0 | 0<br>0<br>2 |
| 365 | 0.0625<br>0.125<br>0.25 | 2.5<br>4<br>5 | 3.5<br>4<br>5 | 5<br>5<br>5 | 5<br>5<br>5 | 0<br>0<br>1 | 0<br>0<br>0 | 0<br>0<br>0 |
| 366 | 0.125<br>0.25<br>0.5 | 4<br>4<br>5 | 4<br>5<br>5 | 4.5<br>5<br>5 | 5<br>5<br>5 | 0<br>0<br>0 | 0<br>0<br>0 | 0<br>0<br>0 |

Test 3:

Flooded paddy field test for preemergence of paddy weeds:

Each pot of 1/2500 are was filled with paddy diluvium soil and manured, and seeds of Barnyardgrass, Tooth cup, Hardstem burlrush or Narrowleaf waterplantain were sown.

On the other hand, two rice plants each consisting two rice seedlings of 3 leaf stage (Sasanishiki, height: 15 cm, quality of the seedlings: good) were transplanted to each pot in a depth of about 2 cm, and the pot was flooded in a depth of 3 cm.

One day after the transplantation, each granule containing each of the Compounds shown in Table 4 and prepared in accordance with Preparation Example 2 was scattered on the flooded surface in an amount to bring the dose of active ingredient to 6.25, 12.5 or 25 g per are.

For three days after the treatment, a leaching loss of water was adjusted at a rate of 3 cm/day, and thereafter, the pot was kept in a greenhouse.

Twenty one days after the treatment, the herbicidal effects and phytotoxicity were investigated. The results thereby obtained are shown in Table 4.

The herbicidal effects and phytotoxicity were rated in the same manner as in Test 1.

In Table 4, the following symbols are used to identify the weeds and the rice:

Q: Barnyardgrass (Echinochloa crus-galli)
R: Tooth cup (Rotala indica)
S: Hardstem bulrush (Scirpus juncoides)
T: Narrowleaf waterplantain (Alisma canaliculatum)
N: Rice (Oryza sativa)

Table 4:

| Compound No. | Dose g/a | Herbicidal effect rating | | | | Phytotoxicity rating |
|---|---|---|---|---|---|---|
| | | Q | R | S | T | N |
| 4 | 6.25<br>12.5<br>25 | 4<br>5<br>5 | 4<br>5<br>5 | 2<br>3<br>3 | 5<br>5<br>5 | 0<br>0<br>0 |
| 6 | 6.25<br>12.5<br>25 | 4<br>5<br>5 | 4<br>5<br>5 | 2<br>4<br>5 | 5<br>5<br>5 | 0<br>0<br>0 |
| 9 | 6.25<br>12.5<br>25 | 3<br>4<br>4 | 4<br>5<br>5 | 3<br>4<br>4 | 5<br>5<br>5 | 0<br>1<br>1 |
| 12 | 6.25<br>12.5<br>25 | 3<br>4<br>5 | 5<br>5<br>5 | 4<br>5<br>5 | 5<br>5<br>5 | 0<br>0<br>1 |
| 15 | 6.25<br>12.5<br>25 | 2<br>2<br>4 | 4<br>5<br>5 | 1<br>2<br>4 | 3<br>4<br>5 | 0<br>0<br>0 |
| 18 | 6.25<br>12.5<br>25 | 3<br>5<br>5 | 4<br>5<br>5 | 1<br>2<br>3 | 4<br>5<br>5 | 0<br>0<br>0 |
| 21 | 6.25<br>12.5<br>25 | 4<br>5<br>5 | 5<br>5<br>5 | 2<br>3<br>3 | 4<br>5<br>5 | 0<br>0<br>1 |
| 24 | 6.25<br>12.5<br>25 | 5<br>5<br>5 | 5<br>5<br>5 | 4<br>5<br>5 | 5<br>5<br>5 | 0<br>0<br>1 |
| 34 | 6.25<br>12.5<br>25 | 3<br>4<br>4 | 4<br>5<br>5 | 4<br>5<br>5 | 5<br>5<br>5 | 0<br>0<br>0 |
| 39 | 6.25<br>12.5<br>25 | 3<br>4<br>5 | 2<br>3<br>5 | 3<br>3<br>4 | 3<br>3<br>5 | 0<br>0<br>0 |

Table 4:

| Compound No. | Dose g/a | Herbicidal effect rating | | | | Phytotoxicity rating |
|---|---|---|---|---|---|---|
| | | Q | R | S | T | N |
| 41 | 6.25 | 5 | 5 | 4 | 5 | 0 |
| | 12.5 | 5 | 5 | 4 | 5 | 0 |
| | 25 | 5 | 5 | 5 | 5 | 1 |
| 42 | 6.25 | 4 | 5 | 4 | 4 | 0 |
| | 12.5 | 4 | 5 | 4 | 5 | 0 |
| | 25 | 5 | 5 | 5 | 5 | 1 |
| 49 | 6.25 | 3 | 4 | 2 | 4 | 0 |
| | 12.5 | 3 | 4 | 3 | 5 | 0 |
| | 25 | 4 | 5 | 3 | 5 | 0 |
| 54 | 6.25 | 5 | 5 | 4 | 5 | 0 |
| | 12.5 | 5 | 5 | 5 | 5 | 0 |
| | 25 | 5 | 5 | 5 | 5 | 1 |
| 61 | 6.25 | 4 | 4 | 3 | 5 | 0 |
| | 12.5 | 5 | 5 | 4 | 5 | 0 |
| | 25 | 5 | 5 | 5 | 5 | 0 |
| 64 | 6.25 | 4 | 5 | 3 | 5 | 0 |
| | 12.5 | 5 | 5 | 3 | 5 | 0 |
| | 25 | 5 | 5 | 4 | 5 | 0 |
| 71 | 6.25 | 5 | 5 | 2 | 4 | 0 |
| | 12.5 | 5 | 5 | 3 | 5 | 0 |
| | 25 | 5 | 5 | 4 | 5 | 1 |
| 80 | 6.25 | 4 | 4 | 3 | 5 | 0 |
| | 12.5 | 4 | 5 | 4 | 5 | 1 |
| | 25 | 5 | 5 | 4 | 5 | 1 |
| 81 | 6.25 | 3 | 5 | 3 | 4 | 0 |
| | 12.5 | 4 | 5 | 3 | 5 | 0 |
| | 25 | 5 | 5 | 4 | 5 | 0 |
| 82 | 6.25 | 4 | 5 | 3 | 5 | 0 |
| | 12.5 | 4 | 5 | 4 | 5 | 0 |
| | 25 | 5 | 5 | 5 | 5 | 1 |

Table 4:

| Compound No. | Dose g/a | Herbicidal effect rating | | | | Phytotoxicity rating |
|---|---|---|---|---|---|---|
| | | Q | R | S | T | N |
| 87 | 6.25<br>12.5<br>25 | 1<br>2<br>3 | 4<br>5<br>5 | 2<br>2<br>3 | 3<br>4<br>5 | 0<br>0<br>0 |
| 88 | 6.25<br>12.5<br>25 | 4<br>5<br>5 | 5<br>5<br>5 | 4<br>5<br>5 | 5<br>5<br>5 | 0<br>0<br>1 |
| 91 | 6.25<br>12.5<br>25 | 5<br>5<br>5 | 5<br>5<br>5 | 4<br>5<br>5 | 5<br>5<br>5 | 0<br>0<br>0 |
| 94 | 6.25<br>12.5<br>25 | 4<br>5<br>5 | 5<br>5<br>5 | 5<br>5<br>5 | 5<br>5<br>5 | 0<br>1<br>1 |
| 95 | 6.25<br>12.5<br>25 | 4<br>5<br>5 | 5<br>5<br>5 | 4<br>4<br>5 | 5<br>5<br>5 | 0<br>1<br>1 |
| 97 | 6.25<br>12.5<br>25 | 5<br>5<br>5 | 5<br>5<br>5 | 4<br>5<br>5 | 5<br>5<br>5 | 0<br>1<br>2 |
| 106 | 6.25<br>12.5<br>25 | 4<br>5<br>5 | 5<br>5<br>5 | 3<br>4<br>4 | 5<br>5<br>5 | 0<br>0<br>0 |
| 107 | 6.25<br>12.5<br>25 | 5<br>5<br>5 | 5<br>5<br>5 | 4<br>5<br>5 | 5<br>5<br>5 | 0<br>0<br>1 |
| 111 | 6.25<br>12.5<br>25 | 4<br>5<br>5 | 5<br>5<br>5 | 3<br>4<br>4 | 5<br>5<br>5 | 0<br>0<br>0 |
| 117 | 6.25<br>12.5<br>25 | 4<br>5<br>5 | 5<br>5<br>5 | 3<br>4<br>5 | 5<br>5<br>5 | 0<br>0<br>0 |

Table 4:

| Compound No. | Dose g/a | Herbicidal effect rating | | | | Phytotoxicity rating |
|---|---|---|---|---|---|---|
| | | Q | R | S | T | N |
| 119 | 6.25<br>12.5<br>25 | 4<br>5<br>5 | 5<br>5<br>5 | 5<br>5<br>5 | 5<br>5<br>5 | 0<br>0<br>1 |
| 123 | 6.25<br>12.5<br>25 | 5<br>5<br>5 | 5<br>5<br>5 | 5<br>5<br>5 | 5<br>5<br>5 | 0<br>1<br>2 |
| 131 | 6.25<br>12.5<br>25 | 4<br>4<br>5 | 5<br>5<br>5 | 3<br>4<br>5 | 5<br>5<br>5 | 0<br>0<br>1 |
| 132 | 6.25<br>12.5<br>25 | 5<br>5<br>5 | 5<br>5<br>5 | 4<br>5<br>5 | 5<br>5<br>5 | 0<br>1<br>1 |
| 135 | 6.25<br>12.5<br>25 | 4<br>5<br>5 | 4<br>5<br>5 | 3<br>3<br>4 | 5<br>5<br>5 | 0<br>0<br>1 |
| 147 | 6.25<br>12.5<br>25 | 3<br>4<br>5 | 4<br>4<br>5 | 2<br>3<br>5 | 4<br>5<br>5 | 0<br>0<br>0 |
| 153 | 6.25<br>12.5<br>25 | 4<br>4<br>5 | 4<br>5<br>5 | 3<br>4<br>5 | 4<br>5<br>5 | 0<br>0<br>1 |
| 159 | 6.25<br>12.5<br>25 | 3<br>5<br>5 | 4<br>5<br>5 | 2<br>4<br>5 | 4<br>5<br>5 | 0<br>0<br>0 |
| 173 | 6.25<br>12.5<br>25 | 4<br>5<br>5 | 5<br>5<br>5 | 4<br>5<br>5 | 5<br>5<br>5 | 0<br>1<br>2 |
| 181 | 6.25<br>12.6<br>25 | 3<br>5<br>5 | 5<br>5<br>5 | 4<br>5<br>5 | 5<br>5<br>5 | 0<br>0<br>1 |

Table 4:

| Compound No. | Dose g/a | Herbicidal effect rating | | | | Phytotoxicity rating |
|---|---|---|---|---|---|---|
| | | Q | R | S | T | N |
| 184 | 6.25 | 4 | 5 | 4 | 5 | 0 |
| | 12.5 | 5 | 5 | 5 | 5 | 0 |
| | 25 | 5 | 5 | 5 | 5 | 1 |
| 186 | 6.25 | 4 | 5 | 2 | 5 | 0 |
| | 12.5 | 5 | 5 | 3 | 5 | 0 |
| | 25 | 5 | 5 | 4 | 5 | 0 |
| 188 | 6.25 | 5 | 5 | 4 | 5 | 0 |
| | 12.5 | 5 | 5 | 5 | 5 | 0 |
| | 25 | 5 | 5 | 5 | 5 | 2 |
| 189 | 6.25 | 2 | 4 | 1 | 4 | 0 |
| | 12.5 | 3 | 4 | 3 | 5 | 0 |
| | 25 | 4 | 5 | 4 | 5 | 0 |
| 191 | 6.25 | 4 | 5 | 5 | 5 | 0 |
| | 12.5 | 5 | 5 | 5 | 5 | 0 |
| | 25 | 5 | 5 | 5 | 5 | 0 |
| 193 | 6.25 | 4 | 5 | 5 | 5 | 0 |
| | 12.5 | 5 | 5 | 5 | 5 | 0 |
| | 25 | 5 | 5 | 5 | 5 | 0 |
| 194 | 6.25 | 3 | 4 | 2 | 3 | 0 |
| | 12.5 | 4 | 4 | 3 | 5 | 0 |
| | 25 | 5 | 5 | 5 | 5 | 0 |
| 195 | 6.25 | 5 | 5 | 4 | 5 | 0 |
| | 12.5 | 5 | 5 | 5 | 5 | 1 |
| | 25 | 5 | 5 | 5 | 5 | 2 |
| 197 | 6.25 | 2 | 5 | 2 | 4 | 0 |
| | 12.5 | 3 | 5 | 2 | 5 | 0 |
| | 25 | 4 | 5 | 3 | 5 | 0 |
| 202 | 6.25 | 4 | 5 | 4 | 5 | 0 |
| | 12.5 | 5 | 5 | 5 | 5 | 0 |
| | 25 | 5 | 5 | 5 | 5 | 0 |

Table 4:

| Compound No. | Dose g/a | Herbicidal effect rating | | | | Phytotoxicity rating |
|---|---|---|---|---|---|---|
| | | Q | R | S | T | N |
| 204 | 6.25<br>12.5<br>25 | 4<br>5<br>5 | 5<br>5<br>5 | 2<br>3<br>5 | 5<br>5<br>5 | 0<br>0<br>0 |
| 208 | 6.25<br>12.5<br>25 | 5<br>5<br>5 | 5<br>5<br>5 | 3<br>4<br>5 | 5<br>5<br>5 | 0<br>0<br>0 |
| 210 | 6.25<br>12.5<br>25 | 3<br>4<br>5 | 5<br>5<br>5 | 4<br>5<br>5 | 5<br>5<br>5 | 0<br>0<br>0 |
| 212 | 6.25<br>12.5<br>25 | 4<br>4<br>5 | 5<br>5<br>5 | 3<br>4<br>5 | 5<br>5<br>5 | 0<br>0<br>0 |
| 213 | 6.25<br>12.5<br>25 | 4<br>5<br>5 | 5<br>5<br>5 | 3<br>5<br>5 | 4<br>5<br>5 | 0<br>0<br>0 |
| 214 | 6.25<br>12.5<br>25 | 2<br>3<br>4 | 4<br>4<br>5 | 2<br>4<br>4 | 3<br>4<br>5 | 0<br>0<br>0 |
| 217 | 6.25<br>12.5<br>25 | 3<br>3<br>5 | 4<br>5<br>5 | 3<br>4<br>4 | 5<br>5<br>5 | 0<br>0<br>0 |
| 219 | 6.25<br>12.5<br>25 | 3<br>4<br>5 | 5<br>5<br>5 | 3<br>4<br>5 | 5<br>5<br>5 | 0<br>0<br>1 |
| 221 | 6.25<br>12.5<br>25 | 3<br>3<br>4 | 5<br>5<br>5 | 4<br>4<br>5 | 5<br>5<br>5 | 0<br>1<br>1 |
| 227 | 6.25<br>12.5<br>25 | 4<br>5<br>5 | 5<br>5<br>5 | 3<br>5<br>5 | 5<br>5<br>5 | 0<br>1<br>1 |

Table 4:

| Compound No. | Dose g/a | Herbicidal effect rating | | | | Phytotoxicity rating |
|---|---|---|---|---|---|---|
| | | Q | R | S | T | N |
| 230 | 6.25 | 3 | 5 | 4 | 5 | 0 |
| | 12.5 | 5 | 5 | 5 | 5 | 0 |
| | 25 | 5 | 5 | 5 | 5 | 0 |
| 231 | 6.25 | 5 | 5 | 3 | 5 | 0 |
| | 12.5 | 5 | 5 | 5 | 5 | 0 |
| | 25 | 5 | 5 | 5 | 5 | 2 |
| 237 | 6.25 | 1 | 1 | 0 | 2 | 0 |
| | 12.5 | 1 | 3 | 1 | 3 | 0 |
| | 25 | 2 | 3 | 3 | 3 | 0 |
| 239 | 6.25 | 2 | 2 | 1 | 3 | 0 |
| | 12.5 | 3 | 3 | 2 | 4 | 0 |
| | 25 | 4 | 3 | 3 | 4 | 0 |
| 242 | 6.25 | 3 | 5 | 2 | 5 | 0 |
| | 12.5 | 5 | 5 | 4 | 5 | 0 |
| | 25 | 5 | 5 | 5 | 5 | 2 |
| 246 | 6.25 | 4 | 5 | 3 | 5 | 0 |
| | 12.5 | 5 | 5 | 4 | 5 | 0 |
| | 25 | 5 | 5 | 4 | 5 | 0 |
| 247 | 6.25 | 4 | 5 | 3 | 5 | 0 |
| | 12.5 | 5 | 5 | 4 | 5 | 0 |
| | 25 | 5 | 5 | 5 | 5 | 0 |
| 249 | 6.25 | 1 | 5 | 4 | 4 | 0 |
| | 12.5 | 3 | 5 | 5 | 5 | 0 |
| | 25 | 4 | 5 | 5 | 5 | 0 |
| 251 | 6.25 | 1 | 5 | 2 | 4 | 0 |
| | 12.5 | 2 | 5 | 4 | 5 | 0 |
| | 25 | 3 | 5 | 5 | 5 | 0 |
| 252 | 6.25 | 2 | 4 | 1 | 4 | 0 |
| | 12.5 | 3 | 5 | 2 | 4 | 0 |
| | 25 | 3 | 5 | 3 | 5 | 0 |

Table 4:

| Compound No. | Dose g/a | Herbicidal effect rating | | | | Phytotoxicity rating |
|---|---|---|---|---|---|---|
| | | Q | R | S | T | N |
| 255 | 6.25<br>12.5<br>25 | 5<br>5<br>5 | 5<br>5<br>5 | 4<br>5<br>5 | 5<br>5<br>5 | 0<br>0<br>2 |
| 256 | 6.25<br>12.5<br>25 | 4<br>5<br>5 | 5<br>5<br>5 | 2<br>4<br>4 | 5<br>5<br>5 | 0<br>0<br>0 |
| 260 | 6.25<br>12.5<br>25 | 1<br>3<br>4 | 5<br>5<br>5 | 3<br>5<br>5 | 4<br>5<br>5 | 0<br>0<br>0 |
| 261 | 6.25<br>12.5<br>25 | 1<br>2<br>3 | 4<br>4<br>5 | 3<br>5<br>5 | 5<br>5<br>5 | 0<br>0<br>0 |
| 262 | 6.25<br>12.5<br>25 | 3<br>5<br>5 | 4<br>5<br>5 | 4<br>5<br>5 | 4<br>5<br>5 | 0<br>0<br>1 |
| 263 | 6.25<br>12.5<br>25 | 0<br>1<br>2 | 1<br>2<br>2 | 2<br>2<br>3 | 1<br>2<br>2 | 0<br>0<br>0 |
| 264 | 6.25<br>12.5<br>25 | 0<br>1<br>1 | 3<br>4<br>5 | 3<br>5<br>5 | 1<br>1<br>2 | 0<br>0<br>0 |
| 271 | 6.25<br>12.5<br>25 | 3<br>4<br>5 | 5<br>5<br>5 | 3<br>4<br>5 | 5<br>5<br>5 | 0<br>0<br>0 |
| 272 | 6.25<br>12.5<br>25 | 2<br>3<br>4 | 4<br>5<br>5 | 3<br>4<br>4 | 5<br>5<br>5 | 0<br>0<br>0 |
| 275 | 6.25<br>12.5<br>25 | 3<br>4<br>5 | 4<br>5<br>5 | 3<br>4<br>4 | 5<br>5<br>5 | 0<br>0<br>0 |

Table 4:

| Compound No. | Dose g/a | Herbicidal effect rating | | | | Phytotoxicity rating |
|---|---|---|---|---|---|---|
| | | Q | R | S | T | N |
| 279 | 6.25<br>12.5<br>25 | 1<br>3<br>4 | 1<br>2<br>4 | 1<br>1<br>3 | 5<br>5<br>5 | 0<br>0<br>0 |
| 280 | 6.25<br>12.5<br>22 | 2<br>3<br>4 | 2<br>3<br>4 | 1<br>2<br>4 | 5<br>5<br>5 | 0<br>0<br>0 |
| Reference E | 6.25<br>12.5<br>25 | 1<br>1<br>4 | 0<br>2<br>4 | 1<br>3<br>4 | 1<br>1<br>2 | 0<br>0<br>2 |

Table 4:

| Compound No. | Dose g/a | Herbicidal effect rating | | | | Phytotoxicity rating |
|---|---|---|---|---|---|---|
| | | Q | R | S | T | N |
| 293 | 6.25<br>12.5<br>25 | 3<br>4<br>5 | 5<br>5<br>5 | 4<br>4<br>5 | 5<br>5<br>5 | 0<br>0<br>1 |
| 294 | 12.5<br>25<br>50 | 4<br>5<br>5 | 5<br>5<br>5 | 4<br>5<br>5 | 5<br>5<br>5 | 0<br>0<br>2 |
| 295 | 6.25<br>12.5<br>25 | 4.5<br>5<br>5 | 5<br>5<br>5 | 4.5<br>4.5<br>5 | 5<br>5<br>5 | 0<br>1<br>1 |
| 296 | 3.125<br>6.25<br>12.5 | 5<br>5<br>5 | 5<br>5<br>5 | 5<br>5<br>5 | 5<br>5<br>5 | 0<br>0<br>1 |
| 297 | 3.125<br>6.25<br>12.5 | 5<br>5<br>5 | 5<br>5<br>5 | 5<br>5<br>5 | 5<br>5<br>5 | 0<br>0<br>0 |
| 299 | 3.125<br>6.25<br>12.5 | 5<br>5<br>5 | 5<br>5<br>5 | 5<br>5<br>5 | 5<br>5<br>5 | 0<br>0<br>0 |
| 301 | 3.125<br>6.25<br>12.5 | 5<br>5<br>5 | 5<br>5<br>5 | 5<br>5<br>5 | 5<br>5<br>5 | 0<br>0<br>1 |
| 307 | 6.25<br>12.5<br>25 | 4.5<br>5<br>5 | 5<br>5<br>5 | 4.5<br>4.5<br>5 | 5<br>5<br>5 | 0<br>1<br>2 |
| 312 | 3.125<br>6.25<br>12.5 | 5<br>5<br>5 | 5<br>5<br>5 | 4.5<br>5<br>5 | 5<br>5<br>5 | 0<br>1<br>2 |
| 323 | 3.125<br>6.25<br>12.5 | 4.5<br>5<br>5 | 5<br>5<br>5 | 4.5<br>5<br>5 | 5<br>5<br>5 | 0<br>0<br>2 |

Table 4:

| Compound No. | Dose g/a | Herbicidal effect rating | | | | Phytotoxicity rating |
|---|---|---|---|---|---|---|
| | | Q | R | S | T | N |
| 324 | 3.125<br>6.25<br>12.5 | 4.5<br>4.5<br>5 | 5<br>5<br>5 | 4.5<br>5<br>5 | 5<br>5<br>5 | 0<br>1<br>2 |
| 325 | 3.125<br>6.25<br>12.5 | 4.5<br>4.5<br>4.5 | 5<br>5<br>5 | 4.5<br>4.5<br>5 | 5<br>5<br>5 | 0<br>0<br>2 |
| 333 | 3.125<br>6.25<br>12.5 | 4.5<br>5<br>5 | 5<br>5<br>5 | 4.5<br>4.5<br>5 | 5<br>5<br>5 | 0<br>0<br>2 |
| 339 | 3.125<br>6.25<br>12.5 | 4<br>4<br>5 | 5<br>5<br>5 | 4.5<br>5<br>5 | 5<br>5<br>5 | 0<br>0<br>1 |
| 348 | 3.125<br>6.25<br>12.5 | 4.5<br>5<br>5 | 5<br>5<br>5 | 5<br>5<br>5 | 5<br>5<br>5 | 1<br>1<br>2 |

CLAIMS :

1) A tetrahydrophthalimide represented by the general formula (I):

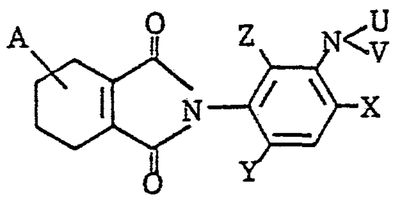

(I)

wherein A is a hydrogen atom or a methyl group,

X is a hydrogen atom, a halogen atom, a methyl group or a methoxy group,

each of Y and Z is a hydrogen atom or a halogen atom,

each of U and V is a hydrogen atom; a $-\!\left(\overset{R^1}{\underset{R^2}{C}}\right)_n\!-COB$ group; an alkyl group; an alkyl group having one or two substituents selected from the group consitsing of a halogen atom, an alkoxy group, a cyano group and an aryl group which may be substituted by a halogen atom; an alkenyl group; an alkenyl group having one or two substituents selected from the group consitsing of an alkoxycarbonyl group and a cyano group; an alkynyl group; a $-\overset{O}{\overset{\|}{C}}-E$ group; or an alkylsulfonyl group,

$R^2$ is a hydrogen atom or a methyl group,

n is an integer of from 1 to 6,

B is $-OR^3$, $-SR^4$ or $-N\!<\!{}^{R^5}_{R^6}$ ,

E is $-R^7$, $-OR^8$, $-SR^9$ , $-N\!<\!{}^{R^{10}}_{R^{11}}$, or a hydrogen atom,

$R^3$ is a hydrogen atom, an alkyl group, an alkenyl group, an alkynyl group, a cycloalkyl group, an aryl group, an aralkyl group or $-\overset{R^{12}}{CH}-COD$ ,

$R^4$ is an alkyl group,

each of $R^5$ and $R^6$ is a hydrogen atom, an alkyl group, an alkoxy group, an alkoxyalkyl group, an alkenyl group, an alkynyl group, an aryl group, an aralkyl group, a cycloalkyl group, an alkylsulfonyl group or $-\overset{R^{12}}{\overset{|}{C}}H-COD$, or $-N<^{R^5}_{R^6}$ is a heterocyclic group which may include an oxygen atom,

$R^7$ is an alkyl group which may be substituted by one or more halogen atoms; an alkenyl group; or an aryl group which may be substituted by a halogen atom,

$R^8$ is an alkyl group, a cycloalkyl group, an aralkyl group or an aryl group,

$R^9$ is an alkyl group or an aryl group,

each of $R^{10}$ and $R^{11}$ is a hydrogen atom, an alkyl group, an alkoxy group or an aryl group which may be substituted by a halogen atom,

$R^{12}$ is a hydrogen atom or an alkyl group, and

D is a hydroxyl group, an alkoxy group or an amino group which may be substituted by one or two alkyl or alkenyl groups.

2) The tetrahydrophthalimide according to Claim 1 which is represented by the general formula (II):

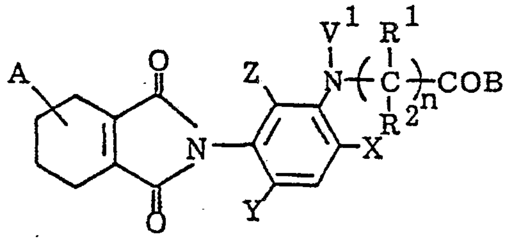

(II)

wherein $V^1$ is a hydrogen atom, an alkyl group, a $-\overset{O}{\overset{\|}{C}}-R^7$, and A, B, n, X, Y, Z, $R^1$, $R^2$ and $R^7$ are as defined with respect to the general formula (I).

3) The tetrahydrophthalimide according to Claim 2 wherein $V^1$ in the general formula (II) is a hydrogen atom.

4) The tetrahydrophthalimide according to Claim 3 which is represented by the general formula (VII):

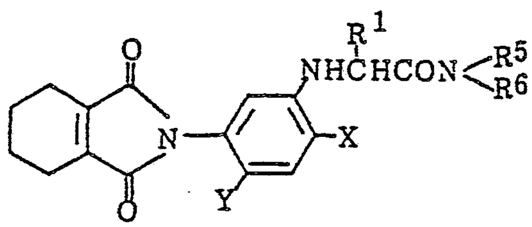

(VII)

wherein X, Y, $R^1$, $R^5$ and $R^6$ are as defined with respect to the general formula (I).

5) The tetrahydrophthalimide according to any one of Claims 1 to 4 wherein in the general formula (I), X is a chlorine atom or a bromine atom, and Y is a hydrogen atom or a fluorine atom.

6) The tetrahydrophthalimide according to Claim 4 wherein in the general formula (VII), X is a chlorine atom or a bromine atom, Y is a hydrogen atom or a fluorine atom, $R^1$ is an alkyl group having from 1 to 3 carbon atoms, $R^5$ is a hydrogen atom and $R^6$ is an alkyl group having from 1 to 5 carbon atoms.

7) The tetrahydrophthalimide according to Claim 4 wherein in the general formula (VII), X is a chlorine atom or a bromine atom, Y is a hydrogen atom or a fluorine atom, $R^1$ is an alkyl group having from 1 to 3 carbon atoms, $R^5$ is an alkyl or alkoxy group having from 1 to 3 carbon atoms and $R^6$ is an alkyl group having from 1 to 3 carbon atoms.

8) The tetrahydrophthalimide according to Claim 1, 2 or 3 which is represented by the general formula (VIII):

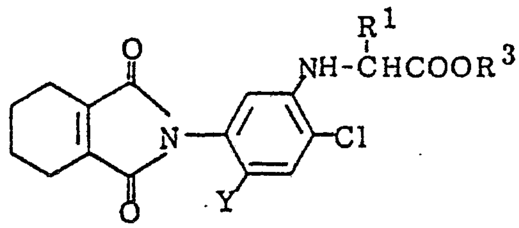

(VIII)

wherein Y, $R^1$ and $R^3$ are as defined with respect to the general formula (I).

9) The tetrahydrophthalimide according to Claim 8 wherein in the general formula (VIII), Y is a hydrogen atom or a fluorine atom, $R^1$ is an alkyl group having from 1 to 4 carbon atoms and $R^3$ is an alkyl group having from 1 to 5 carbon atoms or an alkenyl group having 3 or 4 carbon atoms.

10) The tetrahydrophthalimide according to Claim 1 which is represented by the general formula (IV):

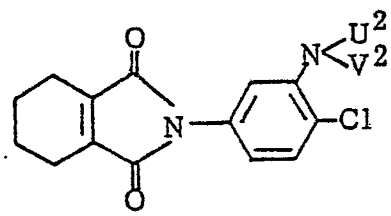

(IV)

wherein each of $U^2$ and $V^2$ is a hydrogen atom; an alkyl group; an alkyl group substituted by a halogen atom, an alkoxy group or a cyano group; an alkenyl group or an alkynyl group, provided one of $U^2$ and $V^2$ is other than a hydrogen atom.

11) The tetrahydrophthalimide according to Claim 1 which is represented by the general formula (V):

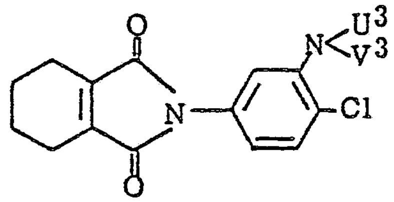

(V)

wherein $U^3$ is a $-\overset{\overset{O}{\|}}{C}-OR^8$ group where $R^8$ is as defined with respect to the general formula (I), and $V^3$ is an alkyl group.

12) A herbicidal composition which comprises the tetrahydrophthalimide of Claim 1 as an active ingredient and an inert carrier.

13) The herbicidal composition according to Claim 12 wherein the active ingredient is the tetrahydrophthalimide of Claim 2.

14) The herbicidal composition according to Claim 12 wherein the active ingredient is the tetrahydrophthalimide of Claim 3.

15) The herbicidal composition according to Claim 12 wherein the active ingredient is the tetrahydrophthalimide of Claim 4.

16) The herbicidal composition according to Claim 12 wherein the active ingredient is the tetrahydrophthalimide of Claim 5.

17) The herbicidal composition according to Claim 12 wherein the active ingredient is the tetrahydrophthalimide of Claim 6.

18) The herbicidal composition according to Claim 12 wherein the active ingredient is the tetrahydrophthalimide of Claim 7.

19) The herbicidal composition according to Claim 12 wherein the active ingredient is the tetrahydrophthalimide of Claim 8.

20) The herbicidal composition according to Claim 12 wherein the active ingredient is the tetrahydrophthalimide of Claim 9.

21) The herbicidal composition according to Claim 12 wherein the active ingredient is the tetrahydrophthalimide of Claim 10.

22) The herbicidal composition according to Claim 12 wherein the active ingredient is the tetrahydrophthalimide of Claim 11.

23) The herbicidal composition according to Claim 12 which comprises from 1 to 80% by weight of the active ingredient, from 2 to 30% by weight of a surfactant, from 10 to 97% of an inert carrier and from 0 to 20% by weight of other additives.

24) The herbicidal composition according to Claim 12 which is formulated in a form of an emulsifiable concentrate, a wettable powder, a dust, granules or tablets.

25) A process for producing a tetrahydrophthalimide represented by the general formula (I):

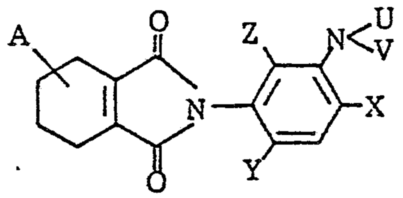

(I)

wherein A, X, Y, Z, U and V are as defined in Claim 1, which comprises:

(a) reacting a tetrahydrophthalic acid anhydride represented by the general formula:

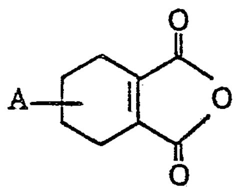

wherein A is as defined with respect to the general formula (I), with an aniline represented by the general formula:

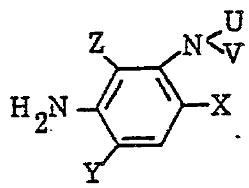

wherein X, Y, Z, U and V are as defined with respect to the general formula (I); or

(b) reacting a compound represented by the general formula:

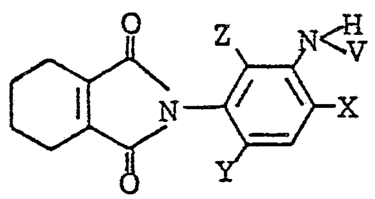

wherein A, X, Y, Z and V are as defined with respect to the general formula (I), with a compound represented by the general formula:

Q - U

wherein U is as defined with respect to the general formula (I) and Q is a halogen atom, mesyl, tosyl or an alkoxy group, or Q forms together with U an acid anhydride or a mixed acid anhydride.

26) A method for controlling undesirable weeds which comprises applying a herbicidally effective amount of a tetrahydrophthalimide of the formula (I) as defined in Claim 1.